# EUROPEAN PATENT SPECIFICATION

(11) **EP 3 953 490 B1**
(45) Date of publication and mention of the grant of the patent: **20.03.2024**
(21) Application number: 20718654.5
(22) Date of filing: 09.04.2020
(51) Int. Cl.: C12Q 1/6806

(54) **METHOD FOR ANALYSING INSERTION SITES**
VERFAHREN ZUR ANALYSE VON INSERTIONSSTELLEN
PROCÉDÉ D'ANALYSE DES SITES D'INSERTION

(30) Priority: 12.04.2019 GB 201905244
(43) Date of publication of application: 16.02.2022
(73) Proprietor: Ospedale San Raffaele S.r.l., 20132 Milano (IT); Fondazione Telethon ETS, 00185 Roma (IT)
(72) Inventor: MONTINI, Eugenio, 20132 Milan (IT); CESANA, Daniela, 20132 Milan (IT)
(74) Representative: D Young & Co LLP
(86) International application number: PCT/EP2020/060285
(87) International publication number: WO 2020/208206

(56) References cited:
- WO-A1-2015/058079
- LAUFS S ET AL: "Insertion of Retroviral Vectors in NOD/SCID Repopulating Human Peripheral Blood Progenitor Cells Occurs Preferentially in the Vicinity of Transcription Start Regions and in Introns", MOLECULAR THERAPY : THE JOURNAL OF THE AMERICAN SOCIETY OF GENE THERAPY, CELL PRESS, US, vol. 10, no. 5, 1 November 2004 (2004-11-01), pages 874-881, XP004660739, ISSN: 1525-0016, DOI: 10.1016/J.YMTHE.2004.08.001
- Andriniaina Andy Nkili-Meyong ET AL: "Genome-wide profiling of human papillomavirus DNA integration in liquid-based cytology specimens from a Gabonese female population using HPV capture technology", Scientific Reports, vol. 9, no. 1, 6 February 2019 (2019-02-06), XP055652032, DOI: 10.1038/s41598-018-37871-2

## Description

### FIELD OF THE INVENTION

The present invention relates to methods for analysing insertion sites in a subject's genome. In particular, the invention relates to the identification and/or quantification of insertion sites of an exogenous nucleotide sequence, such as a viral gene therapy vector, in a subject's genome.

### BACKGROUND TO THE INVENTION

Gene therapy involves the incorporation of genetic material into a cell to treat or prevent disease. The genetic material may supplement defective genes with functional copies of those genes, inactivate improperly functioning genes or introduce genes to instruct new functions to a cell.

Delivery of genetic material to a cell may be achieved through use of vectors which facilitate the transfer of nucleic acids. Viruses may be engineered to deliver a nucleotide of interest (NOI) to a target cell and are commonly employed as vectors in gene therapy. Viruses that have been used in gene therapy to date include retroviruses, lentiviruses, adenoviruses (AdV) and adeno-associated viruses (AAV).

In gene therapy applications using integrating vectors, such as retroviruses (RV), lentiviruses (LV) and transposons, insertion site (IS) analysis is typically performed on cells that are harvested from subjects. For example, in the case of haematopoietic stem and progenitor cell (HSPC)-based gene therapy approaches, analysis is generally carried out on blood or bone marrow (BM) cells.

Insertional profiling studies allow an understanding of several aspects of the safety and efficacy of gene transfer as well as the biology of haematopoiesis. Indeed, IS analysis of the levels of HSPC marking and activity after transplant, and clonal diversity during haematopoietic reconstitution may provide useful efficacy readouts for gene therapy approaches.

Laufs S. et al. Mol Ther. 2004 Nov;10(5):874-81 relates to insertion of retroviral vectors in NOD/SCID repopulating human peripheral blood progenitor cells.

Furthermore, the identification of ISs at serial time points following an intervention (e.g. transplantation) may reveal the emergence of adverse effects such as the in vivo selection and expansion of cell clones harbouring gain-of-function insertional mutants that can give rise to malignancy. Such approaches applied on cell biopsies allowed the detection of expanding myelodysplastic cell clones in two gamma retroviral vector (γRV)-based clinical trials. In both these trials, expanding clones were characterised by the presence of activating insertions within the well-known MECOM proto-oncogene.

However, such IS studies as part of gene therapy procedures provide only a molecular snapshot of the behaviour and dynamics of circulating cells, and do not convey any information on vector-marked cell clones expanding in solid organs unless invasive biopsies are performed.

These limitations were demonstrated in a clinical trial for γRV-based treatment of Severe Combined Immunodeficiencies linked to X chromosome (SCID-X1), in which previous integration studies failed to detect T cell leukaemia events induced by LMO2 activating insertions, which expanded in the thymus, until a full cell transformation occurred and leukaemic clones were released into the circulation.

Accordingly, there remains a significant need for methods that enable characterisation of insertion sites, in particular any negative consequences that they may give rise to, at an early stage and in a manner that is not limited to analysis of a cell sample, which may fail to take into account different insertion sites that may be present elsewhere in an organism. In addition, there is a need for methods that do not require invasive and risky tissue biopsies.

WO2015058079 relates to circulating cancer biomarker and its use.

### SUMMARY OF THE INVENTION

To improve the predictive power of IS studies, for example to characterise viral insertion sites, for example as part of a gene therapy protocol, the inventors have developed a method that allows the identification of ISs from cell-free DNA that may be naturally present in samples from a subject (e.g. blood plasma).

In particular, the inventors have developed a method (referred to as Liquid Biopsy Insertion Site Sequencing (LiBIS-seq)), that enables the identification and quantification of viral and vector insertion sites in genomic DNA (such as retroviral, AAV and transposon insertion sites, and insertion sites of expression cassettes inserted with gene editing technologies) using cell-free DNA. The cell-free DNA may be obtained and/or purified from blood plasma and other body fluids (e.g. cerebrospinal fluid, CSF). Suitable subjects include humans and animal models which are treated using in vivo or ex vivo gene therapy procedures, and/or infected by an integrating and replication competent virus (e.g. HIV or HBV).

The inventors have shown that the method represents a valid procedure for IS studies in in vivo and ex vivo gene therapy applications. Compared to IS analyses using tissue biopsies, the retrieval of IS from cell-free DNA is less risky, non-invasive and can be easily performed multiple times post treatment, thus providing a more comprehensive picture of the clonal repertoire of the transduced organs, as well as longitudinal monitoring of the transduced population.

ISs identified in cell-free DNA isolated from plasma or other body fluids could expand the picture of the clonal diversity of vector marked cells, for example in subjects treated with haematopoietic stem and/or progenitor cell (HSPC) gene therapy, since it may reveal by a simple and non-invasive liquid biopsy the presence of vector marked clones embedded in solid organs, such as liver. Moreover, it may enable the monitoring and study of the clonal repertoire of transduced cells in in vivo gene therapy procedures.

In one aspect the invention provides a method for analysing insertion sites of an exogenous nucleotide sequence in a subject's genome, wherein the method comprises:
(a) using a provided sample from the subject comprising cell-free double-stranded DNA polynucleotides, wherein the sample is a body fluid sample from the subject;
(b) blunting the ends of the polynucleotides;
(c) ligating an oligonucleotide to both ends of the polynucleotides;
(d) amplifying polynucleotides comprising an insertion site; and
(e) sequencing the product of step (d),
wherein the exogenous nucleotide sequence is a viral vector, transposon or expression cassette.

In some embodiments, the analysing comprises identifying the location of one or more insertion sites in the subject's genome. In some embodiments, the analysing comprises quantifying the abundance of one or more insertion sites. In some embodiments, the analysing comprises identifying the location of one or more insertion sites in the subject's genome and quantifying the abundance of each of the one or more insertion sites.

In some embodiments, the analysing comprises determining the risk of insertional mutagenesis.

In preferred embodiments, the analysing comprises identification and quantification of one or more insertion sites, and determining the risk of insertional mutagenesis.

In some embodiments, the subject has been administered gene therapy. In some embodiments, the subject is about to be administered gene therapy.

In some embodiments, the gene therapy is in vivo gene therapy. In some embodiments, the gene therapy is ex vivo gene therapy.

In some embodiments, the gene therapy is haematopoietic stem and/or progenitor cell (HSPC) gene therapy.

In some embodiments, the exogenous nucleotide sequence is a gene therapy vector. In some embodiments, the exogenous nucleotide sequence is a viral genome sequence.

In some embodiments, the subject is infected with a virus, optionally an integrating virus. In some embodiments, the subject is at risk of infection with a virus, optionally an integrating virus. In some embodiments, the integrating virus is a retrovirus, lentivirus or AAV. In some embodiments, the virus is HIV or hepatitis B virus.

In preferred embodiments, the exogenous nucleotide sequence is a viral vector.

In some embodiments, the expression cassette is integrated by gene editing.

In some embodiments, the viral vector is a viral gene therapy vector. In some embodiments, the viral gene therapy vector comprises a nucleotide of interest (NOI), preferably a therapeutic transgene.

In some embodiments, the viral vector is a retroviral, lentiviral or AAV vector.

In some embodiments, the sample is a plasma, serum, urine or cerebrospinal fluid (CSF) sample. In preferred embodiments, the sample is a plasma sample.

In some embodiments, step (a) further comprises a step of purifying the polynucleotides.

In some embodiments, step (b) comprises end-repair. In some embodiments, step (b) comprises digestion of single-stranded overhangs.

In some embodiments, step (b) further comprises 5' phosphorylation and/or 3' adenylation.

In preferred embodiments, the oligonucleotides are partially double-stranded.

In preferred embodiments, the oligonucleotides that bind to both ends of the polynucleotides are the same.

In some embodiments, each oligonucleotide comprises:
(i) a first portion comprising a random nucleotide sequence;
(ii) a second portion comprising a barcode nucleotide sequence; and/or
(iii) a third portion comprising a binding site for a primer for use in the sequencing of step (e), for example Illumina sequencing primer P7 or P5.

In some embodiments, the amplifying of step (d) is by PCR. In some embodiments, the PCR is nested PCR.

In some embodiments, the PCR comprises amplifying the product of step (c) using a primer that binds to a portion of the exogenous nucleotide sequence and a primer that binds to a portion of the oligonucleotide.

In preferred embodiments, the portion of the exogenous nucleotide sequence bound by the primer is a viral LTR sequence.

In some embodiments, the PCR comprises:
(i) amplifying the product of step (c) using a first PCR step using a pair of outer primers to obtain a first PCR product; and
(ii) amplifying the first PCR product using a second PCR step using a pair of inner primers to obtain a second PCR product.

In some embodiments, one primer of the pair of outer primers binds to a first portion of the exogenous nucleotide sequence and one primer of the pair of inner primers binds to a second portion of the exogenous nucleotide sequence.

In some embodiments, one primer of the pair of outer primers binds to a first portion of the oligonucleotide and one primer of the pair of inner primers binds to a second portion of the oligonucleotide.

In some embodiments, the first and second portions of the exogenous nucleotide sequence are the same. In some embodiments, the first and second portions of the exogenous nucleotide sequence are different.

In some embodiments, the first and second portions of the oligonucleotide are the same. In some embodiments, the first and second portions of the oligonucleotide are different.

In some embodiments, the nested PCR comprises:
(i) amplifying the product of step (c) using a first PCR step using a first primer that binds to a first portion of the exogenous nucleotide sequence and a second primer that binds to a first portion of an oligonucleotide to obtain a first PCR product; and
(ii) amplifying the first PCR product using a second PCR step using a third primer that binds to a second portion of the exogenous nucleotide sequence and a fourth primer that binds to a second portion of the oligonucleotide to obtain a second PCR product.

In some embodiments, one primer used for PCR amplification comprises:
(i) a first portion comprising a random nucleotide sequence;
(ii) a second portion comprising a barcode nucleotide sequence; and/or
(iii) a third portion comprising a binding site for a primer for use in the sequencing of step (e), for example Illumina sequencing primer P7 or P5.

The primer bound by the third portion may be different from the primer bound by the third portion of the oligonucleotide ligated to the polynucleotide.

In another aspect the invention provides a method for determining the safety and/or efficacy of a gene therapy, wherein the method comprises:
(a) using a provided sample from a subject comprising cell-free double-stranded DNA polynucleotides, wherein the sample is a body fluid sample from the subject, preferably a plasma, serum, urine or cerebrospinal fluid (CSF) sample;
(b) blunting the ends of the polynucleotides;
(c) ligating an oligonucleotide to both ends of the polynucleotides;
(d) amplifying polynucleotides comprising a gene therapy vector insertion site; and
(e) sequencing the product of step (d) for identifying the location of one or more insertion sites in the subject's genome and/or quantifying the abundance of each of the one or more insertion sites.

In another aspect the invention provides a method for analysing vector insertion sites in a subject's genome, wherein the method comprises:
(a) using a provided sample from the subject comprising cell-free double-stranded DNA polynucleotides, wherein the sample is a body fluid sample from the subject, preferably a plasma, serum, urine or cerebrospinal fluid (CSF) sample;
(b) blunting the ends of the polynucleotides;
(c) ligating an oligonucleotide to both ends of the polynucleotides;
(d) amplifying polynucleotides comprising a vector insertion site; and
(e) sequencing the product of step (d) for identifying the location of one or more insertion sites in the subject's genome and/or quantifying the abundance of each of the one or more insertion sites.

In another aspect the invention provides a method for identifying gene therapy induced cancer cells in a subject, wherein the method comprises:
(a) using a provided sample from a subject comprising cell-free double-stranded DNA polynucleotides, wherein the sample is a body fluid sample from the subject, preferably a plasma, serum, urine or cerebrospinal fluid (CSF) sample;
(b) blunting the ends of the polynucleotides;
(c) ligating an oligonucleotide to both ends of the polynucleotides;
(d) amplifying polynucleotides comprising an insertion site of an exogenous nucleotide sequence; and
(e) sequencing the product of step (d) for identifying the location of one or more insertion sites in the subject's genome and/or quantifying the abundance of each of the one or more insertion sites,
wherein the exogenous nucleotide sequence is a viral vector, transposon or expression cassette.

### DESCRIPTION OF THE DRAWINGS

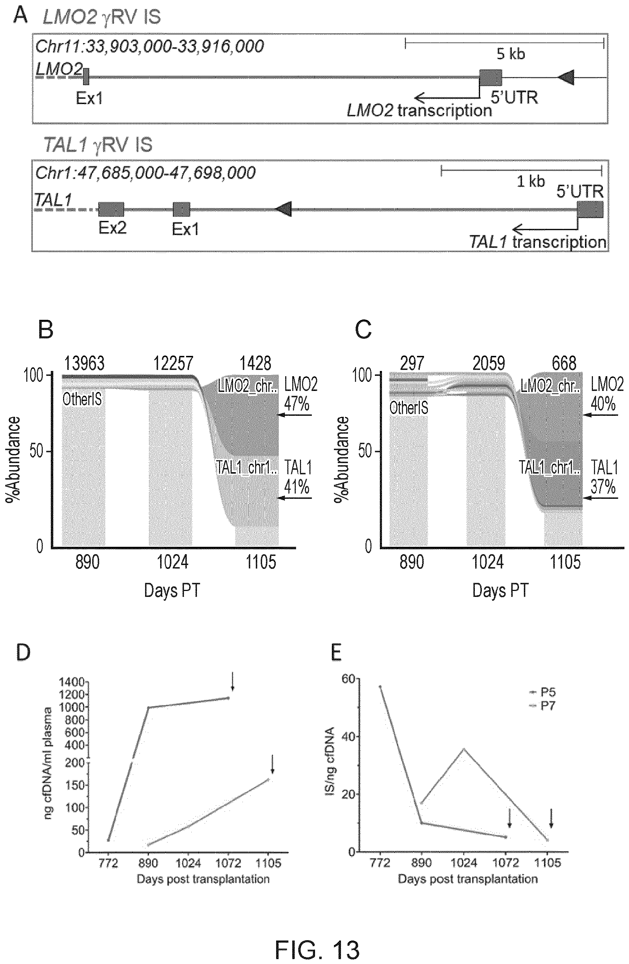

**FIGURE 1**
   **Scheme of Liquid Biopsy Insertion Site Sequencing (LiBIS-seq).** LiBIS-seq is performed on cell-free DNA (circDNA), for example, purified from blood plasma or other body fluids obtained from gene therapy (GT)-treated patients and subjects infected by an integrating vector/virus. Purified circDNA of gene therapy patients may include, for example, fragmented DNA originating from the cellular genome (grey fragments), from the integrated virus/vector (pink fragments) and hybrid fragments containing the viral/genome junction that will be amplified in the PCR procedure. In the example shown, upon purification (STEP 1), the fragmented circDNA is subjected to end repair and 3' adenylation (STEP 2), and then ligated to partially double-stranded linker cassettes (represented in black) containing a sequence barcode used for sample identification and a random sequence (Unique Molecular Identifier, UMI) used for abundance estimation (STEP 3). In the example show, the ligation product is first subjected to 35 cycles of exponential PCR using primers specific for the vector LTR and the linker cassette (STEP 4). Next, the amplified product is re-amplified with an additional 10 PCR cycles using primers specific for the vector LTR and the linker cassette, but located within the already amplified product. Finally, the generated LiBIS-seq PCR products are assembled into libraries and sequenced. Sequencing reads are processed and viral/vector insertion sites are mapped on the reference genome.
**FIGURE 2**
   **Identification of insertion sites in circDNA (circlS) from metachromatic leukodystrophy (MLD) patients treated with lentiviral vector (LV)-based haematopoietic stem cell gene therapy.** (A) Summary table showing the number of total insertion sites (IS) retrieved from each patient between the different sources. (B) Genomic distribution of circlS (red line) and IS determined from cellular DNA (celllS; blue line) in the HLA genomic region of chromosome 6 obtained from patient MLD01. The symbol of some of the targeted genes within the region is indicated. (C) Gene ontology analyses performed using Great software show similar overrepresentation for gene classes in Gene Onthology Cellular Component System for circlS (red line) and celllS (blue line) collected from patient MLD01. (D) IS tracking of the circlS retrieved from blood plasma of patient MLD06 across different cell types and time. Each row represents a unique circlS, the coloured bars indicate when and where the circlS was identified across the different cell lineages and the time point after gene therapy (columns). Lack of a bar indicates that the integration was not retrieved at the indicated time point and source. B: B cells; T: T cells; My: Myeloid cells. (E) Percentage of shared ISs (y-axis) among circlS and BM-derived CD34+ cells and myeloid and lymphoid lineages from peripheral blood and bone marrow. Each column represents the average level of sharing observed among the 7 patients tested for each cell lineage. (F, G) Stacked bar plots showing the abundance of LV IS retrieved over time (months, x-axis) from PBMCs (F) and blood plasma (G) of patient MLD01. LV IS with level of abundance higher than 1% are represented by different coloured regions whose height is proportional to the number of fragments (plasma) or cells (PMBC) retrieved for that IS over the total and for that specific time point (%IS Abundance, y-axis). Ribbons connect LV IS tracked among consecutive time points. LV IS with level of abundance lower than 1% are collapsed into the grey area. The number of unique IS retrieved from each specific time point is indicated above each column.
**FIGURE 3**
   **circIS retrieved from non-human primates (NHP) treated by LV-mediated liver-directed gene therapy.** (A) Table showing the total number of IS retrieved in each treated NHP from plasma and tissue. (B-I) Stacked bar plots showing the abundance of LV IS retrieved from blood plasma (B-G), liver (H) and spleen (I) of NHP treated with control LV or CD47hi-LV. Each LV IS retrieved from blood plasma of the treated NHP is represented by a different colour, with the height in relative proportion to the number of fragments retrieved over the total (%IS Abundance). Ribbons connect LV IS tracked among consecutive time points (Days post injection, x-axis). (H, I) Each LV IS retrieved from liver (H) and spleen (I) of the treated NHP is represented by a different colour, with the height in relative proportion to the number of cells retrieved over the total (%IS Abundance).
**FIGURE 4**
   **circIS retrieved from dogs treated by LV-mediated liver-directed gene therapy.** (A) Table showing the type and dose of LV injected in 3 treated dogs and the number of IS retrieved from the circDNA. (B-D) Stacked bar plots showing the abundance of LV IS retrieved from blood plasma of dogs treated with the different LVs. Each LV IS is represented by a different colour, with the height in relative proportion to the number of fragments retrieved over the total (%IS Abundance). Ribbons connect LV IS tracked among consecutive time points (Days post injection, x-axis).
**FIGURE 5**
   **circIS retrieved from a SCID-X1 patient treated by gamma-retroviral (gRV)-based haematopoietic stem cell gene therapy, and who developed T-cell acute lymphoblastic leukaemia (T-ALL) as a consequence of insertional mutagenesis events.** Stacked bar plots showing the abundance of gRV IS retrieved over time (months, x-axis) from PBMC (A) and blood plasma (B) of patients that underwent haematopoietic stem cell gene therapy for SCID-X1 (A, B). gRV IS with level of abundance higher than 1% are represented by different colours, whose height is proportional to the number of fragments retrieved for that IS over the total and for that specific time point (%IS Abundance, y-axis). The time point at which the LMO2-driven T-ALL was diagnosed in each patient is highlighted in red. Ribbons connect gRV IS tracked between consecutive time points. gRV IS with a level of abundance lower than 1% are collapsed in the grey area. (A) and (B) relate to gRV IS collected from PBMC (A) and blood plasma (B) of patient P9 from the SCID-X1 haematopoietic stem cell gene therapy clinical trial.
**FIGURE 6**
   **Retrieval of IS from cfDNA purified from plasma of MLD patients treated by LV-based HSC-GT.** A) cfDNA recovered over time per ml of blood plasma (months, x-axis) of the 7 MLD patients; grey area refers to cfDNA concentration levels in healthy subjects; B) Stacked bar plots showing the abundance of LV IS retrieved over time (months, x-axis) from cfDNA of 7 MLD patients. In each column, each LV IS is represented by different colors, whose height is proportional with the number of fragments (plasma) retrieved for that IS over the total and for each specific time point (%IS Abundance, y-axis). Ribbons connect LV IS tracked among consecutive time points. The number of unique IS retrieved from each specific time points is indicated in red above each column. C) IS retrieved over time from cfDNA and normalized for the ng used for all MLD patients (months, x-axis);. D) VCN measured over time (months, x-axis) from PBMC of the 7 MLD patients; E) Classification map showing all LV IS (left part of the graph) retrieved from cfDNA over time from blood plasma of MLD06 patient. Each row represents a unique LV IS, the coloured bar indicate when and where the IS was identified across the different cell lineage and time point after gene therapy (columns). Lack of colour indicates that the integration was not retrieved at the indicated time point and source. F,G) Frequency of cfDNA-derived ISs (y axis) shared with the different hematopoietic lineages from BM (F) and peripheral blood (G). H,I) Relative frequency of the different hematopoietic lineages (y axis) in BM (H) and peripheral blood (I). Wh.: whole blood or whole bone marrow cells; MNC: Mononuclear Cells;; PBMC: Peripheral Blood Mononuclear Cells; CD34: HSPCs; CD13, CD14, CD15: (Myeloid cells); CD19: B cells; CD3:T cells. * Unpaired t-test of transformed lod-odd values.
**FIGURE 7**
   **Retrieval of IS from cfDNA purified from blood plasma of MLD patients treated by LV-based HSC-GT.** A) cfDNA per ml of blood plasma of the 6 MLD patients and collected pre-gene therapy (GT), within the first 3 months post-GT (Early 1-3 mo) and within 72 (Late 6-72 mo) months post GT; p values have been determined using unpaired, two tailed Mann-Whitney t-test, *** stands for p<0.001; MLD04 was excluded from the analyses; grey area refers to cfDNA concentration levels in healthy subjects; . B) Representative examples of the size profile of the cfDNA fragments purified from blood plasma of the indicated MLD patients; C,D) Distribution of the fragment sizes after PCR amplification and sequencing starting from sonicated genomic DNA purified from PBMC (C) and cfDNA (D). The distribution of the fragment sizes after PCR amplification and sequencing from cfDNA, except for a peak around 167 bp, was similar to the size distribution of PCR products generated from the sonicated cell-derived DNA.
**FIGURE 8**
   **Retrieval of IS from cfDNA purified from blood plasma of MLD patients treated by LV-based HSC-GT.** A) cfDNA recovered per ml of blood plasma of the 7 MLD patients; B) Number of LV IS retrieved from cfDNA purified from blood plasma of 7 MLD patients. C) Frequency distribution along chromosomes (chr numbers and span shown on top) of human refSeq genes (black track) and LV integrations retrieved from cell-derived genomic DNA (blue track) and cfDNA (red track). D) Frequency distributions of LV IS collected from the 7 MLD patients and retrieved from the cfDNA (red line) and cell-derived genomic DNA (blue line) at 4 selected chromosomal regions targeted at targeted at high frequency. Distribution of % IS (y-axix) across the selected chromosomal coordinates (x-axis). The symbols of some highly targeted genes within the region are indicated; E, F) Frequency distribution of LV integrations around gene transcriptional start sites in cfDNA (E) and cell-derived genomic DNA (F) of all 7 MLD patients. Analyses were performed by Great software (http://great.stanford.edu/); G) Gene Ontology (GO) analysis of ISs retrieved from cfDNA of 7 MLD patients and performed using GREAT software. Significant overrepresented Gene Ontology classes of the Cellular Component, Biological Process and Molecular Function System are shown if having a Fold enrichment score higher than 2. Red lines indicate the threshold level of significance.
**FIGURE 9**
   **Distribution of the abundance level of IS retrieved from cfDNA of HSC-GT treated MLD patients.** A) Frequency of IS (y-axis) represented by the indicated number of genomes (x-axis, Genome bin) in IS derived from cfDNA of the 7 MLD patients treated by HSC-GT; (B) Stacked bar plots showing the frequency of LV IS represented by the indicated number of fragments.
**FIGURE 10**
   **Retrieval of IS from genomic DNA purified from blood cells of MLD patients treated by LV-based HSC-GT.** Stacked bar plots showing the abundance of LV IS retrieved over time (months, x-axis) from genomic DNA purified from PBMC (A) and whole blood cells (B) of 7 MLD patients. In each column, each LV IS is represented by different colors, whose height is proportional with the number of fragments (plasma) retrieved for that IS over the total and for each specific time point (%IS Abundance, y-axis). Ribbons connect LV IS tracked among consecutive time points. The number of unique IS retrieved from each specific time points is indicated in red above each column. PBMC: Peripheral Blood Mononuclear Cells.
**FIGURE 11**
   **Tracking of LV IS retrieved from cfDNA among cell-derived IS.** A-F) Classification map showing all LV IS (left part of the graph) retrieved over time from cfDNA purified from blood plasma of the indicated patients. Each row represents a unique LV IS, and for each IS the coloured bars indicate when and where the IS was identified across the different cell lineage and time point after gene therapy (columns). Lack of colour indicates that the integration was not retrieved at the indicated time point and source. Whole: whole blood or bone marrow cell population; MNC: Mononuclear Cells; PBMC: Peripheral Blood Mononuclear cells; CD34: HSPCs; CD13, CD14, CD15: Myeloid cells; CD19: B cells; CD3:T cells.
**FIGURE 12**
   **LV IS retrieved from cfDNA purified from serum of dogs treated by in vivo liver-directed GT.** A) cfDNA for ml (ng/ml) of serum purified at different time points in each dog; B) total number of IS retrieved at each time point; C-E) Stacked bar plots showing the abundance and tracking over time of LV IS retrieved from cfDNA purified from sureum of treated dogs. In each column, each LV IS is represented by a different color, with the height in relative proportion with the total number of fragments retrieved (%IS Abundance). Ribbons connect LV IS tracked among consecutive time points (x-axis: days post injection,). The number of unique IS retrieved for each time points is indicated above each column. F) Stacked bar plots showing the abundance of LV IS retrieved from genomic DNA isolated from liver necropsies of treated dogs. The number of unique IS retrieved in each animal is indicated above the specific column. G) Number of IS retrieved for each ng of cfDNA used; H) Frequency of IS (y-axis) represented by the indicated number of genomes (x-axis, Genome bin) in IS derived from cfDNA purified from HSPC-GT MLD patients (blue line) or dogs subjected to in vivo liver directed GT; IS derived from MLD patients were represented by a significantly higher number of genomes compared to dog IS, as determined by unpaired t-test.and the Holm-Sidak method for multiple comparison correction. I, L) Heatmap graph showing LV IS that were identified in cfDNA and liver genomic DNA of the indicated dog (I, refers to M57; L refers to 059). Each row represents a unique LV IS, the coloured bar indicate when and where the IS was identified across the different time point or DNA sources (columns). Lack of colour indicates that the integration was not retrieved at the indicated time point and source; M) Estimate of the population size of marked cell clones contributing to the IS shared over time for dog M57 and 059. ****refer to p-value < 0.0001.
**FIGURE 13**
   **IS retrieved from cfDNA allows identification of vector-marked tumors expanding in solid organs.** A) Genomic distribution of gRV IS targeting LMO2 and TAL1 in WAS P7. In the schema, genomic coordinates and scale are indicated. Blue boxes and bars indicate exons, black arrows indicate the orientation of gene transcription; red triangles indicate position and orientation of the integration gRV IS site. The gRV IS is indicated in red. B, C) Stacked bar plots showing abundance and tracking over time (months, x-axis) of gRV IS retrieved from PBMC-derived genomic DNA (B) and from cfDNA obtained from matched blood plasma collection (C) of WAS P7. gRV IS with level of abundance higher than 1% are represented by different colors, whose height is proportional with the number of fragments retrieved for that IS over the total (% IS Abundance, y-axis). IS with level of abundance lower than 1% are collapsed in the grey area. Ribbons connect gRV IS tracked between consecutive time points. On the x-axis is indicated in red the time point where the leukemia disorders was diagnosed. Number of unique IS retrieved at each time point is indicated above the column. D) cfDNA recovered over time per ml of blood plasma (months, x-axis) for P5 and P7 WAS patients; E) IS retrieved over time and normalized for the ng of cfDNA used for P5 and P7 WAS patients (months, x-axis);. F) Experimental strategy of T-cell lymphoblastic lymphoma mouse model. Briefly, non-conditioned X-SCID mice are transplanted with SIN.LV transduced WT CD45.1 BM-derived lin- cells, and blood is collected on a monthly bases. Tissues are harvested at euthanasia; G) Survival curve of transplanted X-SCID mice; H) Heat-map showing tracking over time of IS shared among DNA sources in mouse A3. Age at euthanasia is indicated. Each row represents a unique LV IS whose coloured is proportional with the relative level of abundance (red: high; blue: low level of abundance). Lack of colour indicates that the IS was not retrieved at the indicated time point and source. Number of unique IS retrieved at each time point is indicated above the column; I) Relative level of abundance over time and among the different DNA sources of the top 3 most abundant LV IS retrieved in the thymus of mouse A3. These LV IS are mapped near the RefSeq gene Cdk19, Asf1a and Man2a1 respectively.
**FIGURE 14**
   **IS retrieved from cfDNA allows identification of vector-marked tumors expanding in solid organs.** A) Genomic distribution of gRV ISs targeting LMO2 and retrieved from genomic DNA purified from total PBMC and cfDNA of P9 X-SCID. In the schema, genomic coordinates and scale are indicated. Blue boxes and bars indicate exons, black arrow indicates the orientation of gene transcription; red triangle indicates position and orientation of the gRV IS integration site. B, C) Stacked bar plots showing the abundance and tracking over time (months, x-axis) of gRV IS retrieved from P9-SCIDX1. IS were collected from PBMC-derived genomic DNA (B) and cfDNA obtained from matched blood plasma collection (C). Legend as Figure 13B, C. A) cfDNA recovered over time per ml of blood plasma (months, x-axis) for P9 SCID-X1 patient; B) IS retrieved over time and normalized for the ng of cfDNA used for P9 SCID-X1 patient (months, x-axis).
**FIGURE 15**
   Shannon diversity index (y-axis) by bins of ng of cfDNA (x-axis and distinct color). Each dot represents a sample, and the shape of each dot refers to a different study. Statistical results of tests are reported with p-values above each pair of bins connected by whisker, or annotated on top considering all bins.
**FIGURE 16**
   Scatter plots of fragment abundance by number of distinct fragments (x-axis) and number of estimated fragments with "SonicLength" (y-axis). Each box refers to a different study. Each dot represents an integration site identified for each available time point. The shape of each dot indicates if that integration belongs or not to malignant clones.
**FIGURE 17**
   **AAV-IS retrieval in immunodeficient mice treated with a ZAP-70 expressing AAV.** A) Map of ZAP-70 expressing AAV and oligonucleotides used for SLiM-PCR (purple arrows). The AAV construct is surrounded by the left and right inverted terminal repeats (L- and R-ITR, green arrows). The murine phosphoglycerate kinase (mPGK, blu arrow) promoter drives the expression of the ZAP70 transgene (yellow arrow), followed by the polyadenylation signal (PolyA, red box); B) Number of AAV-IS (N°IS) retrieved from lymph nodes (LN), Liver and plasma samples; tissue.
**FIGURE 18**
   **Distribution and abundance of AAV IS retrieved in tissues and cfDNA.** A) Frequency of IS retrieved by different PCR primer sets within each indicated dataset. B) Stacked bar plots showing the frequency of AAV IS represented by the indicated number of fragments (when cfDNA is used) or genomes (when sonicated genomic DNA is adopted); C) Stacked bar plots showing the abundance of AAV IS retrieved from cfDNA, LN and LIVER tissue. In each column, each IS is represented by different colors, whose height is proportional with the number of fragments (plasma) or genomes (for LN and Liver) retrieved for that IS over the total (%IS Abundance, y-axis). The number of unique IS retrieved from each specific time points is indicated in red above each column. D) Distribution of AAV-IS at mouse Tcr-α. Each AAV insertion is indicated by a black vertical line. E) Frequency of AAV IS targeting Tcr-a, Tcr-b and Tcr-g gene for the different AAV dataset. F) Distribution of the number of fragments or genomes associated to each AAV integration targeting the Tcr-a gene within the indicated dataset.

### DETAILED DESCRIPTION OF THE INVENTION

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

### Exogenous nucleotide sequence

The term "exogenous nucleotide sequence" as used herein may refer to a nucleotide sequence that is not part of a subject's endogenous genetic material. For example, the exogenous nucleotide sequence may be derived from an exogenous source, such as a gene therapy vector, transposon or expression cassette (e.g. which has been inserted using gene editing methods).

In some embodiments, the exogenous nucleotide sequence is a gene therapy vector. In some embodiments, the exogenous nucleotide sequence is a viral genome sequence. In some embodiments, the exogenous nucleotide sequence is a natural viral sequence, optionally an integrating virus. In some embodiments, the integrating virus is a retrovirus, lentivirus or AAV. In some embodiments, the virus is HIV or hepatitis B virus.

### Vector

A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid, or facilitating the expression of the protein encoded by a segment of nucleic acid.

Vectors may be non-viral or viral. Examples of vectors used in recombinant nucleic acid techniques include, but are not limited to, plasmids and viruses. The vector may be single stranded or double stranded. It may be linear and optionally the vector comprises one or more homology arms. The vector may also be, for example, a naked nucleic acid (e.g. DNA). In its simplest form, the vector may itself be a nucleotide of interest.

The vectors used in the invention may be, for example, plasmid or virus vectors and may include a promoter for the expression of a polynucleotide and optionally a regulator of the promoter.

Vectors may be introduced into cells using a variety of techniques known in the art, such as transformation, transfection and transduction. Several techniques are known in the art, for example transduction with recombinant viral vectors, such as retroviral, lentiviral, adenoviral, adeno-associated viral, baculoviral and herpes simplex viral vectors, Sleeping Beauty vectors; direct injection of nucleic acids and biolistic transformation.

Non-viral delivery systems include but are not limited to DNA transfection methods. Here, transfection includes a process using a non-viral vector to deliver a gene to a target cell. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nature Biotechnology (1996) 14: 556) and combinations thereof.

The term "vector" includes an expression vector, i.e. a construct capable of in vivo or in vitro/ex vivo expression. Expression may be controlled by a vector sequence, or, for example in the case of insertion at a target site, expression may be controlled by a target sequence.

The exogenous nucleotide sequences analysed in the present invention, which may be a vector, are integrated into a subject's genome. In preferred embodiments, the vector is an integrating vector.

In some embodiments, the vector is a retroviral, lentiviral or AAV vector.

### Retroviral and lentiviral vectors

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV). A detailed list of retroviruses may be found in Coffin, J.M. et al. (1997) Retroviruses, Cold Spring Harbour Laboratory Press, 758-63.

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses. A review of these retroviruses is presented in Coffin, J.M. et al. (1997) Retroviruses, Cold Spring Harbour Laboratory Press, 758-63.

The basic structure of retrovirus and lentivirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective.

Portions of the viral genome may also be replaced by a library encoding candidate modulating moieties operably linked to a regulatory control region and a reporter moiety in the vector genome in order to generate a vector comprising candidate modulating moieties which is capable of transducing a target host cell and/or integrating its genome into a host genome.

Lentivirus vectors are part of the larger group of retroviral vectors. A detailed list of lentiviruses may be found in Coffin, J.M. et al. (1997) Retroviruses, Cold Spring Harbour Laboratory Press, 758-63. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells (Lewis, P et al. (1992) EMBO J. 11: 3053-8; Lewis, P.F. et al. (1994) J. Virol. 68: 510-6). In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans). Examples of non-primate lentiviruses may be any member of the family of lentiviridae which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

The viral vector may have a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

### Adeno-associated viral (AAV) vectors

Adeno-associated virus (AAV) has a high frequency of integration and it can infect non-dividing cells. This makes it useful for delivery of genes into mammalian cells in tissue culture.

AAV has a broad host range for infectivity. Details concerning the generation and use of AAV vectors are described in US Patent No. 5139941 and US Patent No. 4797368.

Recombinant AAV vectors have been used successfully for in vitro and in vivo transduction of marker genes and genes involved in human diseases.

### Nucleotide of interest

The exogenous nucleotide sequence of the invention preferably comprises a nucleotide of interest (NOI).

Preferably the nucleotide of interest gives rise to a therapeutic effect.

Suitable NOIs include, but are not limited to, sequences encoding enzymes, cytokines, chemokines, hormones, antibodies, anti-oxidant molecules, engineered immunoglobulin-like molecules, single chain antibodies, fusion proteins, immune co-stimulatory molecules, immunomodulatory molecules, anti-sense RNA, microRNA, shRNA, siRNA, ribozymes, miRNA target sequences, a transdomain negative mutant of a target protein, toxins, conditional toxins, antigens, tumour suppressor proteins, growth factors, transcription factors, membrane proteins, surface receptors, anti-cancer molecules, vasoactive proteins and peptides, anti-viral proteins and ribozymes, and derivatives thereof (such as derivatives with an associated reporter group). The NOIs may also encode pro-drug activating enzymes.

An example of a NOI is the beta-globin chain which may be used for gene therapy of thalassemia/sickle cell disease.

NOIs also include those useful for the treatment of other diseases requiring non-urgent/elective gene correction in the myeloid lineage such as: chronic granulomatous disease (CGD, e.g. the gp91phox transgene), leukocyte adhesion defects, other phagocyte disorders in patients without ongoing severe infections and inherited bone marrow failure syndromes (e.g. Fanconi anaemia), as well as primary immunodeficiencies (SCIDs).

NOIs also include those useful in the treatment of lysosomal storage disorders and immunodeficiencies.

### Expression cassettes and transposons

The term "expression cassette" as used herein may refer to a nucleotide sequence that comprises an open reading frame operably linked to one or more regulatory sequences, such as a promoter and/or a 3' untranslated region (3'-UTR).

A transposon (transposable element) is a DNA sequence that can change position within a genome, which may have the effect of creating or reversing mutations, altering a cell's genetic identity and genome size. Transposition may result in duplication of genetic material.

Transposable elements are non-viral gene delivery vehicles found ubiquitously in nature. Transposon-based vectors have the capacity of stable genomic integration and long-lasting expression of transgene constructs in cells.

DNA transposons translocate via a non-replicative "cut-and-paste" mechanism. This requires recognition of the two terminal inverted repeats (TIRs) by a DNA transposase that cleaves its target and consequently releases the DNA transposon from its donor template. Upon excision, the DNA transposons subsequently integrate into the acceptor DNA that is cleaved by the same transposase. Typically, this results in target-site duplications (TSDs) at the insertion sites. There are evolutionary remnants of transposon DNA in the human genome but they have become silent during evolution and in principle are unable to undergo transposition.

For gene transfer applications with DNA transposons, a binary system has been developed based on two distinct plasmids whereby the transposase-encoding sequence is physically separated from the transposon DNA containing the gene of interest flanked by the TIRs. Co-delivery of the transposon and transposase plasmids into target cells enables transposition via a conventional cut-and-paste mechanism. Ideally, the transposase should be expressed for a short time only and sustained expression is preferably avoided as this may lead to continuous transposon mobilisation and integration. It may be beneficial to minimise the number of vector copies per cell as this increases the risk of insertional oncogenesis. Typically, the expression plasmid encoding the transposase should gradually disappear from the transfected cells due to DNA degradation and/or dilution upon cell division. As a safer alternative, it is also possible to deliver the transposase as an mRNA that results in its transient expression sufficient to enable transposition while minimising the risk of insertional oncogenesis.

Different types of transposons are used in gene therapy applications, including: (i) Sleeping Beauty (SB); (ii) piggyBac (PB); and (iii) Tol2 transposons. These differ for the species of origin, classification, molecular structure, cargo capacity and DNA integration profile.

### Gene editing

The term "gene editing" as used herein refers to a type of genetic engineering in which a nucleic acid is inserted, deleted or replaced in a cell, typically in a targeted manner. Gene editing may be achieved using engineered nucleases, which may be targeted to a desired site in a polynucleotide (e.g. a genome). Such nucleases may create site-specific double-strand breaks at desired locations, which may then be repaired through non-homologous end-joining (NHEJ) or homologous recombination (HR), resulting in targeted mutations.

Such nucleases may be delivered to a target cell using vectors, such as viral vectors.

Examples of suitable nucleases known in the art include zinc finger nucleases (ZFNs), transcription activator like effector nucleases (TALENs), and the clustered regularly interspaced short palindromic repeats (CRISPR)/Cas system (Gaj, T. et al. (2013) Trends Biotechnol. 31: 397-405; Sander, J.D. et al. (2014) Nat. Biotechnol. 32: 347-55).

Meganucleases (Silve, G. et al. (2011) Cur. Gene Ther. 11: 11-27) may also be employed as suitable nucleases for gene editing.

The CRISPR/Cas system is an RNA-guided DNA binding system (van der Oost et al. (2014) Nat. Rev. Microbiol. 12: 479-92), wherein the guide RNA (gRNA) may be selected to enable a Cas9 domain to be targeted to a specific sequence. Methods for the design of gRNAs are known in the art. Furthermore, fully orthogonal Cas9 proteins, as well as Cas9/gRNA ribonucleoprotein complexes and modifications of the gRNA structure/composition to bind different proteins, have been recently developed to simultaneously and directionally target different effector domains to desired genomic sites of the cells (Esvelt et al. (2013) Nat. Methods 10: 1116-21; Zetsche, B. et al. (2015) Cell pii: S0092-8674(15)01200-3; Dahlman, J.E. et al. (2015) Nat. Biotechnol. 2015 Oct 5. doi: 10.1038/nbt.3390. [Epub ahead of print]; Zalatan, J.G. et al. (2015) Cell 160: 339-50; Paix, A. et al. (2015) Genetics 201: 47-54).

### Insertion site

The term "insertion site" as used herein may refer to the location in a genome at which an exogenous nucleotide sequence is inserted. For example, in the context of an integrating viral gene therapy vector the insertion site is the location in a genome at which genetic material derived from the virus is integrated.

An exogenous nucleotide sequence that is integrated into a genome at an insertion site may be derived, for example, from a viral genome or vector which is integrated using gene editing methods. Certain types of vectors may exhibit preferences for insertion at particular genomic sites, such as gene coding regions, CpG islands or transcriptional start sites.

Insertion of an exogenous nucleotide sequence creates junctions, which may be referred to as insertion junctions, between the exogenous nucleotide sequence and the host genome at the ends of the exogenous nucleotide sequence.

A potential risk that is associated with integration of an exogenous nucleotide sequence in a genome is insertional mutagenesis, i.e. mutations that arise as a consequence of the introduction of a nucleotide sequence.

Such insertional mutagenesis bears risks including disrupting genetic information within a cell and accidental activation of oncogenes, which may cause malignant transformation of the cell.

Insertion site locations in a host genome constitute molecular markers that may be used in monitoring the fate of affected cells.

### Methods

In one aspect the invention provides a method for analysing insertion sites of an exogenous nucleotide sequence in a subject's genome, wherein the method comprises:
(a) using a provided sample from the subject comprising cell-free double-stranded DNA polynucleotides, wherein the sample is a body fluid sample from the subject;
(b) blunting the ends of the polynucleotides;
(c) ligating an oligonucleotide to both ends of the polynucleotides;
(d) amplifying polynucleotides comprising an insertion site; and
(e) sequencing the product of step (d),
wherein the exogenous nucleotide sequence is a viral vector, transposon or expression cassette.

The methods of analysis may be used to determine the safety and/or efficacy of a gene therapy.

For example, the methods of analysis may comprise identifying the location of one or more insertion sites in the subject's genome and/or quantifying the abundance of each of the one or more insertion sites.

The analysis may inform the safety of a gene therapy by determining whether an insertion site may have potentially deleterious consequences, for example deactivation or mutation of a beneficial genetic trait or activation of an oncogene.

In some embodiments, the analysis comprises determining the risk of insertional mutagenesis.

In addition or in the alternative, the analysis may inform the efficacy of a gene therapy by characterising insertion sites and also enabling longitudinal monitoring of the transduced population.

In the context of infection of a subject by a virus, the analysis may inform the health status of the subject. For example, the analysis by enable determination of whether a viral integration may give rise to insertional mutagenesis and/or activation of an oncogene.

### Sample

The capability of using fluid samples in the methods of the invention is advantageous because fluid samples may be taken from a subject in a less invasive manner and with reduced risks compared to the taking of solid tissue biopsies.

In some embodiments, the sample is a plasma, serum, urine or cerebrospinal fluid (CSF) sample.

Such samples may be readily obtained from a subject by the skilled person using methods that are known in the art.

In preferred embodiments, the sample is a plasma sample.

### Cell-free DNA

The term "cell-free DNA" as used herein refers to doubled-stranded DNA that is present in body fluids of a subject, such as plasma. Cell-free DNA fragments may be released into body fluids by apoptotic and necrotic cells in the body.

The cell-free DNA may be from a body fluid such as plasma, serum, urine or cerebrospinal fluid (CSF). In preferred embodiments, the cell-free DNA is from plasma. The cell-free DNA may be circulating cell-free DNA.

Cell-free DNA is detectable in plasma of healthy individuals, however, increased levels are observed in pathologic conditions like cancer, inflammation and autoimmune diseases.

### Polynucleotide and oligonucleotide

The term "oligonucleotide" as used herein may refer to short nucleic acid polymers, for example polymers of DNA nucleotides. Although the exact length of an oligonucleotide is not particularly limited, an oligonucleotide may be, for example, about 4-200 nucleotides in length.

The term "polynucleotide" as used herein may refer to longer nucleic acid polymers, for example polymers of DNA nucleotides.

The term "hybridisation" as used herein refers to the hydrogen bonding of opposing nucleic acid strands, preferably Watson-Crick hydrogen bonding between complementary nucleoside or nucleotide bases.

Nucleotides each comprise a nucleobase. The term "nucleobase" as used herein refers to nitrogenous bases, including purines and pyrimidines, such as the DNA nucleobases A, T, G and C, the RNA nucleobases A, U, C and G, as well as non-DNA/RNA nucleobases, such as 5-methylcytosine (^{Me}C), isocytosine, pseudoisocytosine, 5-bromouracil, 5-propynyluracil, 5-propyny-6-fluorouracil, 5-methylthiazoleuracil, 6-aminopurine, 2-aminopurine, inosine, 2,6-diaminopurine, 7-propyne-7-deazaadenine, 7-propyne-7-deazaguanine and 2-chloro-6-aminopurine.

Nucleic acids may be, for example, single- or double-stranded.

The "sense" strand ("positive" strand) has the same sequence as the messenger RNA into which the double-stranded polynucleotide is transcribed (with the exception of any typical nucleobases differences, e.g. between DNA and RNA, T is replaced by U). The opposite, "anti-sense" strand ("negative" strand) is used as the template for messenger RNA during transcription. The anti-sense strand is thus responsible for the RNA that may be, for example, translated to protein, while the sense strand possesses a nearly identical makeup to that of the messenger RNA.

Complementarity is the principle affecting the binding of two single-stranded nucleic acids to form a double-stranded nucleic acid. It is a property shared between two nucleic acid sequences, such that when they are aligned antiparallel to each other, the nucleotides opposing each other in the two sequences will all be complementary for optimal binding. At the molecular level, complementarity is determined by optimal hydrogen bonding between specific base pairs. For example, in DNA, adenine is complementary to thymine, and guanine is complementary to cytosine; and in RNA, adenine is complementary to uracil, and guanine is complementary to cytosine. Complementary pairing of bases allows information to be copied from one molecule to another, and, in nature, from one generation of cells to another.

A double-stranded nucleic acid may be comprised of two strands of the same length, in which case both ends of the double-stranded nucleic acid may be blunt ended.

Alternatively, one or both ends of a double-stranded nucleic acid may exhibit an overhang of single-stranded nucleic acid (i.e. the nucleic acid is partially double-stranded), for example if one strand is longer than the other or if the two strands are offset from one another. Such overhangs may enable a single-stranded nucleic acid to bind to two or more complementary nucleic acids, and thus, by the same token, the double-stranded nucleic acid may bind to one or more further single-stranded nucleic acids by virtue of base pairing with the overhang, thus creating regions of overlap between opposing single-stranded nucleic acids.

### Linker cassette

The oligonucleotide that is ligated to the ends of the polynucleotide may also be referred to herein as a "linker cassette" or "linker oligonucleotide".

In some embodiments, the oligonucleotide comprises a sequence to which primers used in the amplification step bind.

In preferred embodiments, the oligonucleotides are partially double-stranded.

In preferred embodiments, the oligonucleotides that bind to both ends of the polynucleotides are the same.

In some embodiments, each oligonucleotide comprises:
(i) a first portion comprising a random nucleotide sequence, preferably capable of distinguishing the polynucleotide to which it is ligated;
(ii) a second portion comprising a barcode nucleotide sequence; and/or
(iii) a third portion comprising a binding site for a primer for use in the sequencing of step (e).

The random nucleotide sequence may be unique to each individual oligonucleotide and may enable individual polynucleotides within a sample to be distinguished from one another.

Barcoding may be used to enable multiplex sequencing to be carried out. Multiplex sequencing enables large numbers of libraries to be pooled and sequenced simultaneously during a single sequencing run. Using the oligonucleotides disclosed herein, barcode nucleotide sequences may be added to each DNA polynucleotide so that each sequencing read can be identified and sorted before final data analysis. Pooling samples in such a manner exponentially increases the number of samples that can be analysed in a single run.

### Purifying

The method of the invention may comprise a step of purifying the cell-free double-stranded DNA polynucleotides.

Purification of DNA may isolate or enrich the DNA from a sample that comprises other components, such as proteins and cell debris. The purified DNA may be free of or substantially free of such other components.

Purification of DNA may be readily carried out by the skilled person using methods that are known in the art. Suitable methods are disclosed herein in the Examples.

For example, commercial DNA purification kits such as QIAamp Circulating Nucleic Acid Kit (55114, QIAGEN) and Maxell RSC ccfDNA Plasma kit (AS1480, Promega) may be used.

### Blunting

Cell-free double-stranded DNA isolated from a subject may be partially double-stranded, for example having a portion of overhanging single-stranded DNA at one or both ends.

Blunting of such partially double-stranded DNA may be achieved by digestion of the single-stranded DNA, for example using a nuclease (e.g. an exonuclease), and/or filling in the missing nucleotide sequence by the addition of nucleotides on the complementary strand using the overhang as a template for polymerisation, for example using a polymerase. This process may be referred to as end-repair.

In some embodiments, blunting comprises digestion of single-stranded DNA. In some embodiments, blunting comprises filling in missing nucleotide sequence. In some embodiments, blunting comprises digestion of single-stranded DNA and filling in missing nucleotide sequence, for example a 5'-protruded DNA ends may be filed in (e.g. using a 5'→3' polymerase) and a 3' overhangs may be removed (e.g. using a 3'→5' exonuclease).

Blunting of DNA may be readily carried out by the skilled person using methods that are known in the art. Suitable methods are disclosed herein in the Examples.

For example, DNA polymerases, such as the Klenow fragment of DNA Polymerase I and T4 DNA Polymerase may be used to fill in (5'→3') and digest (3'→5'). Removal of a 5' overhang may be achieved, for example, with a nuclease, such as Mung Bean Nuclease.

In preferred embodiments, the DNA polynucleotides are 5'-phosphorylated, for example after the step of blunting. 5'-phosphorylation adds 5'-phosphates to ends of unphosphorylated DNA.

5'-phosphorylation may be readily carried out by the skilled person using methods that are known in the art. Suitable methods are disclosed herein in the Examples.

For example, T4 polynucleotide kinase (PNK) may be used to 5'-phosphorylate DNA.

### Ligating

Ligation joins DNA strands together with the formation of phosphodiester bonds. Thus, ligation of the oligonucleotide and polynucleotide as disclosed herein joins the oligonucleotide and polynucleotide by phosphodiester bonds.

Ligation of DNA may be readily carried out by the skilled person using methods that are known in the art. Suitable methods are disclosed herein in the Examples.

For example, T4 DNA Ligase may be used to ligate DNA. For example, commercial DNA ligation kits such as the DNA Technologies Ligation Kit may be used.

### Amplifying

Amplification of DNA may be readily carried out by the skilled person using methods that are known in the art, in particular using the polymerase chain reaction (PCR).

The methods of the invention comprise the amplification of polynucleotides comprising an insertion site. A polynucleotide comprising an insertion site may comprise one or more insertion junctions, typically one insertion junction. Preferably, the polynucleotides comprising an insertion site are selectively amplified (i.e. polynucleotides comprising an insertion site that are comprised with a population of polynucleotides, some of which do and some of which do not comprise an insertion site, will be amplified while the polynucleotides that lack an insertion site will not be amplified).

Amplification of polynucleotides comprising an insertion site may be achieved using PCR using two primers that bind to sequences in the polynucleotide that are on opposing sides of an insertion site.

In some embodiments, the PCR comprises amplifying the product of step (c) using a primer that binds to a portion of the exogenous nucleotide sequence and a primer that binds to a portion of the oligonucleotide.

The portion of the exogenous nucleotide sequence may be, for example, a portion of a nucleotide of interest or a portion of a viral sequence, such as a viral LTR sequence.

In preferred embodiments, the portion of the exogenous nucleotide sequence bound by the primer is a viral LTR sequence.

In some embodiments, the PCR is nested PCR.

Nested PCR may be readily carried out by the skilled person using methods that are known in the art. Suitable methods are disclosed herein in the Examples.

Nested PCR may achieve greater specificity in amplifying polynucleotides comprising an insertion site compared to standard PCR.

In some embodiments, the PCR comprises:
(i) amplifying the product of step (c) using a first PCR step using a pair of outer primers to obtain a first PCR product; and
(ii) amplifying the first PCR product using a second PCR step using a pair of inner primers to obtain a second PCR product.

In some embodiments, one primer of the pair of outer primers binds to a first portion of the exogenous nucleotide sequence and one primer of the pair of inner primers binds to a second portion of the exogenous nucleotide sequence. In some embodiments, one primer of the pair of outer primers binds to a first portion of the oligonucleotide and one primer of the pair of inner primers binds to a second portion of the oligonucleotide.

In some embodiments, the first and second portions of the exogenous nucleotide sequence are the same. In some embodiments, the first and second portions of the exogenous nucleotide sequence are different. In some embodiments, the first and second portions of the oligonucleotide are the same. In some embodiments, the first and second portions of the oligonucleotide are different.

In some embodiments, the nested PCR comprises:
(i) amplifying the product of step (c) using a first PCR step using a first primer that binds to a first portion of the exogenous nucleotide sequence and a second primer that binds to a first portion of an oligonucleotide to obtain a first PCR product; and
(ii) amplifying the first PCR product using a second PCR step using a third primer that binds to a second portion of the exogenous nucleotide sequence and a fourth primer that binds to a second portion of the oligonucleotide to obtain a second PCR product.

### Sequencing

Sequencing, for example next-generation sequencing, may be readily carried out by the skilled person using methods that are known in the art. Suitable methods are disclosed herein in the Examples.

In preferred embodiments, the sequencing is next-generation sequencing.

Next-generation sequencing (NGS; also referred to as high-throughput sequencing) refers to sequencing technologies that have been developed since the earlier Sanger sequencing, such as Illumina (Solexa) sequencing, Roche 454 sequencing, Ion torrent: Proton / PGM sequencing and SOLiD sequencing.

For example, sequencing may be carried out using the Illumina Myseq/HiSeq platform (Illumina, San Diego, CA.).

Sequencing data may be processed using by the skilled person using bioinformatics methods that are known in the art, for example as disclosed in Spinozzi, G. et al. (2017) BMC Bioinformatics 18: 520.

For example, paired sequence reads may be filtered for quality standards. Barcodes (e.g. comprised within the oligonucleotide) may then be identified for sample demultiplexing of the sequence reads.

The location of insertion sites may be determined, for example, by mapping onto a reference genome (e.g. a human genome sequence). Locations may be characterised, for example by mapping each insertion site to the nearest RefSeq gene.

Insertion site quantification may be carried out, for example, by a method in which the number of genomes with the same insertion site is calculated by counting the different number of fragments identified within the cell-free DNA; then, the relative abundance of each insertion site may be calculated as the percentage of cell-free DNA with a specific insertion site divided by the total number of fragments harbouring different insertion sites identified (Firouzi, S. et al. (2014) Genome Medicine 6: 46; Gillet, N.A. et al. (2011) Blood 117: 3113-3122).

### Further methods

The method of the invention may be used in combination with a further method for analysing insertion sites of an exogenous nucleotide sequence using cellular DNA.

In some embodiments, the further method comprises:
(a) providing a sample of cells from the subject;
(b) isolating DNA from the cells, optionally comprising disrupting the cells and/or purifying the DNA;
(c) fragmenting the DNA, optionally by sonication; and
(d) (i) blunting the ends of the polynucleotides; (ii) ligating an oligonucleotide to both ends of the polynucleotides; (iii) amplifying polynucleotides comprising an insertion site; and (iv) sequencing the product of step (iii).

Further methods for analysing insertion sites of an exogenous nucleotide sequence are envisaged.

In another aspect, the invention provides a method for analysing insertion sites of an exogenous nucleotide sequence in a subject's genome, wherein the method comprises:
(a) using a provided sample from the subject comprising cell-free double-stranded DNA polynucleotides, wherein the sample is a body fluid sample from the subject;
(b) blunting the ends of the polynucleotides;
(c) ligating an oligonucleotide to both ends of the polynucleotides;
(d) purifying polynucleotides comprising an insertion site; and
(e) sequencing the product of step (d)
wherein the exogenous nucleotide sequence is a viral vector, transposon or expression cassette.

The purification of polynucleotides comprising an insertion site may be achieved, for example, using a probe that binds to a portion of the exogenous nucleotide sequence, for example a single-stranded nucleotide sequence that is complementary to a portion of the exogenous nucleotide sequence. The probe may be linked to an agent that enables purification of the polynucleotide: probe complex, for example a bead.

### Subject

The term "subject" as used herein refers to either a human or non-human animal.

Examples of non-human animals include vertebrates, for example mammals, such as non-human primates (particularly higher primates), dogs, rodents (e.g. mice, rats or guinea pigs), pigs and cats. The non-human animal may be a companion animal.

In preferred embodiments, the subject is human.

The skilled person will understand that they can combine all features of the invention disclosed herein without departing from the scope of the invention as disclosed.

Preferred features and embodiments of the invention will now be described by way of nonlimiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

### EXAMPLES

### EXAMPLE 1

The inventors have developed a method, referred to as Liquid Biopsy Insertion Site Sequencing (LiBIS-seq), that enables the identification and quantification of viral and vector insertion sites (such as retroviral, AAV and transposon insertion sites, and insertion sites of expression cassettes inserted with gene editing technologies) in genomic DNA using circulating cell-free DNA (circDNA). The circulating cell-free DNA may be obtained from blood plasma and other body fluids (e.g. cerebrospinal fluid, CSF). Suitable subjects include humans and animal models, which are treated using in vivo or ex vivo gene therapy procedures, and/or infected by an integrating and replication competent virus (e.g. HIV or HBV).

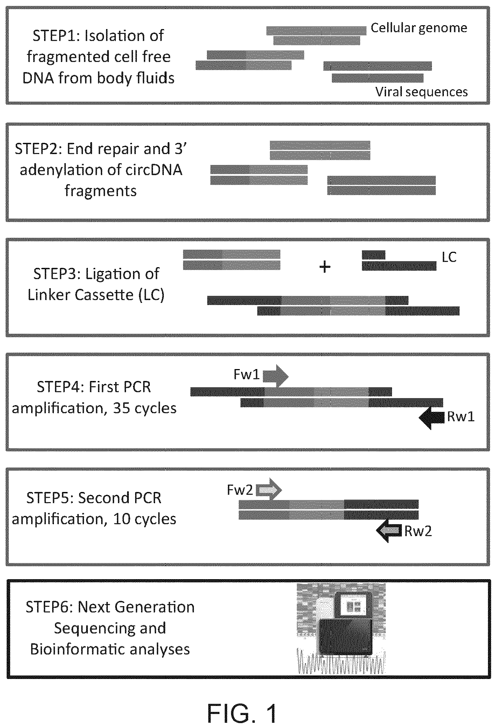

An overview of an example method is shown in Figure 1, which includes the following steps:
1. Purification of circDNA from blood plasma or other suitable body fluid;
2. End repair of the total and fragmented double-stranded DNA, phosphorylation of 5' prime ends and A-tailing of 3' ends;
3. Ligation of DNA linker cassettes (LC) containing sequence barcodes and Unique Molecular Identifiers (UMI);
4. A first PCR amplification using primers complementary to the LC and vector genome end;
5. A second PCR amplification using primers binding to the DNA fragment previously amplified. This step may increase the specificity of the amplified fragment containing the junction of the integrated vector and the cellular genome; and
6. Sequencing. Sequence results may be subsequently de-multiplexed, and vector/genome junctions identified and mapped on a reference genome using bioinformatics approaches (Biffi, A. et al. (2013) Science 341: 1233158; Spinozzi, G. et al. (2017) BMC Bioinformatics 18: 520).

### RESULTS

### Retrieval of insertion sites (IS) from the blood plasma circDNA of metachromatic leukodystrophy patients treated with lentiviral vector-based haematopoietic stem cell gene therapy

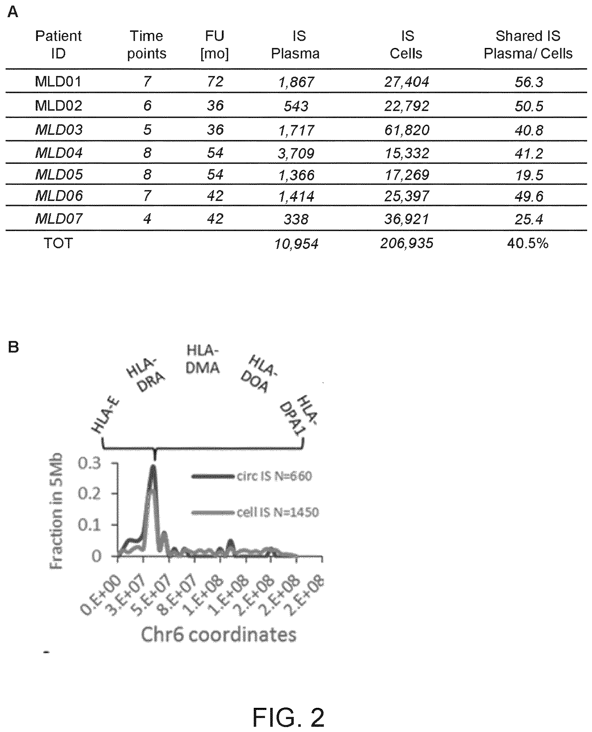

The LiBIS-seq approach was applied on circDNA purified from the blood plasma of 7 metachromatic leukodystrophy (MLD) patients undergoing lentiviral vector (LV)-based haematopoietic stem cell gene therapy. For each patient, 1-2 mL of blood plasma was collected at 7-8 different time points post transplantation (range 1-72 months, Figure 2A). circDNA was the isolated and the LiBIS-seq approach performed.

PCR products were sequenced using different Illumina platforms and mapped with a dedicated bioinformatics pipeline (Biffi, A. et al. (2013) Science 341: 1233158; Spinozzi, G. et al. (2017) BMC Bioinformatics 18: 520). Overall, we retrieved >10900 unique IS from circDNA (circIS, range 338 to 3709 per patient).

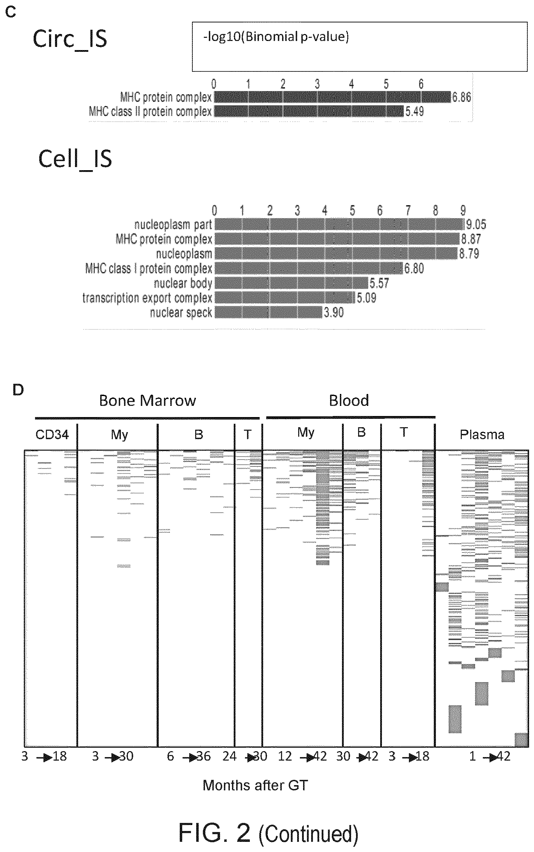

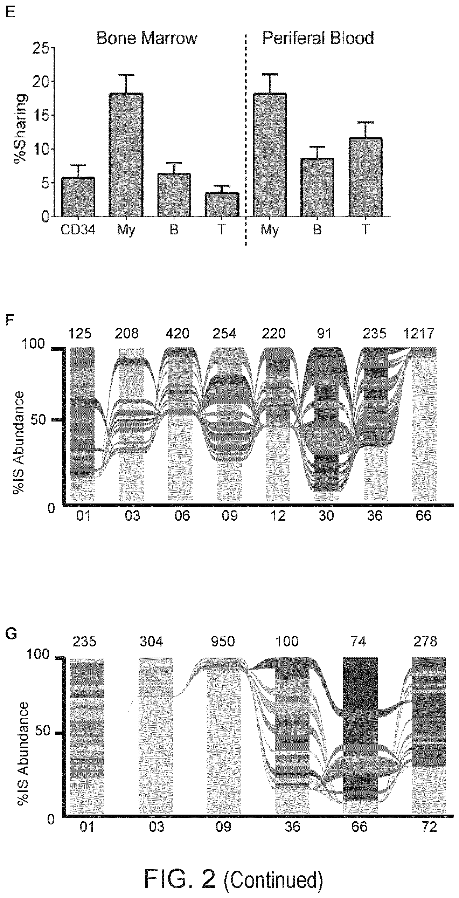

The IS retrieved from the circDNA of MLD displayed the typical features of LV IS collected from the genomic DNA of transduced blood cells that have been previously reported: preferential integration within the transcriptional unit of cellular genes; targeting with high frequency five genomic regions of the chromosome; and enrichment within the same Gene Ontology classes (Figure 2B, C). By comparing the circIS identified in each patient with IS retrieved from the genomic DNA isolated from peripheral blood mononuclear cells (PBMCs, ceIIIS), and cell lineages collected from the blood and bone marrow (BM) of the same patient, it was found that on average only 40% of the circIS were shared with cellIS (Figure 2D). Myeloid cells from blood and BM were those that showed the highest level of sharing with circIS (around 20%, Figure 2E), possibly because those cells are short-lived cells with a rapid turn-over.

Finally, from analysing the clonality and dynamics of circIS, a highly polyclonal pattern with absence of dominant clone expanding and/or persisting over time was observed (Figure 1D, E), resembling the picture observed using cellIS collected from PBMCs of the same patients over the same follow up period of time (Figure 2F, G).

Overall, these results recapitulate the safety data previously reported for this trial (Biffi, A. et al. (2013) Science 341: 1233158), and since only 40% of the circIS were shared with cellIS, it is possible that a fraction of such IS could derive from vector-marked clones embedded in solid organs.

### Retrieval of IS from the blood plasma circDNA of animal models treated with LV-mediated liver-directed gene therapy

To confirm that circIS could reveal IS of vector-marked cell clones embedded in solid organs, analysis was performed on LV-mediated liver-directed gene therapy in non-human primate models (NHP) and a dog model of haemophilia.

In the first animal models, NHPs were treated with two different LV batches, a control LV and a phagocytosis-shielded LV (3 for each LV type) characterised by a high CD47 surface content that enhances hepatocyte gene transfer compared to parental LV (CD47hi LV). Both LVs express the human factor IX antigen under the control of a hepatocyte-specific expression cassette (Cantore, A. et al. (2012) Blood 120: 4517-4520).

Blood plasma was collected at 15, 28, 60 and 90 days post injection in both groups of treated animals, and LV IS were retrieved from purified circDNA at each time point.

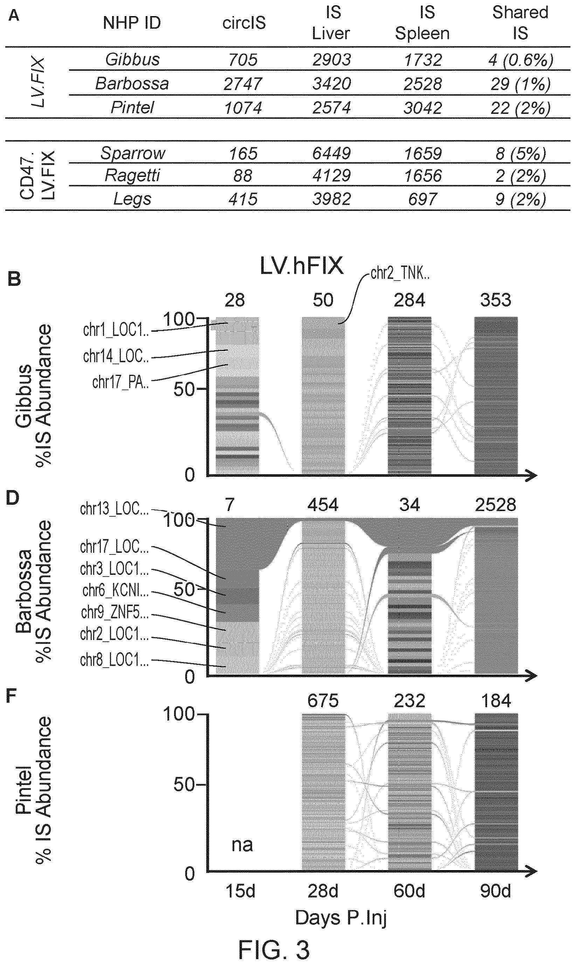

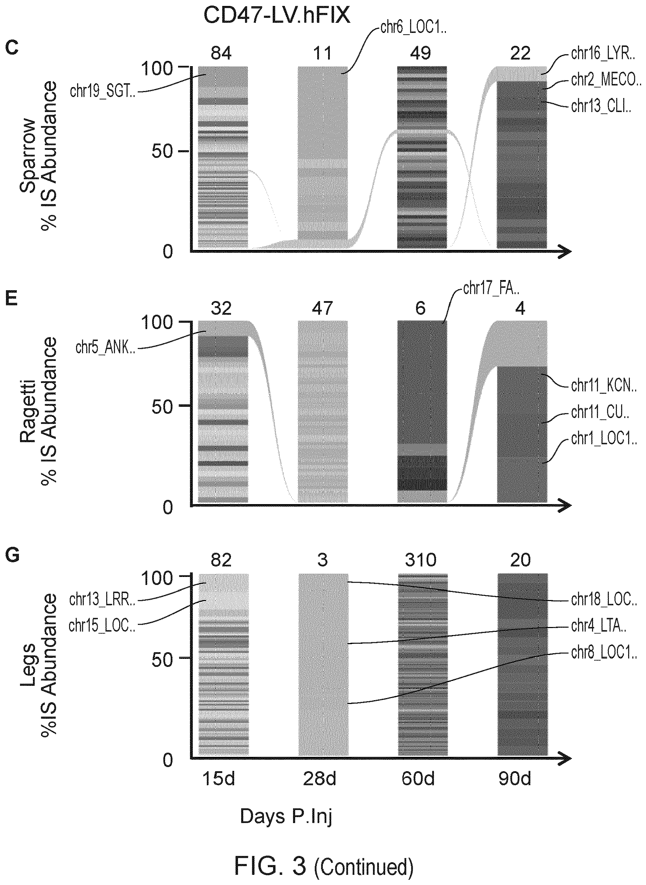

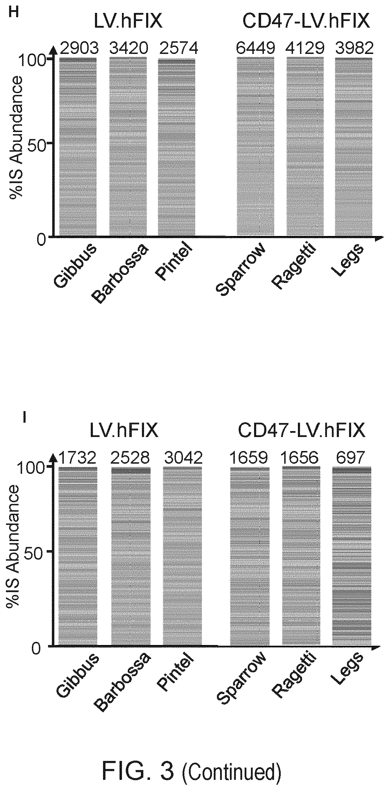

Overall, 165, 88 and 415 LV IS were retrieved from the NHPs treated with the CD47hi LV, and 1074, 705 and 2747 LV IS from the NHPs injected with the control LV (Figure 3A). IS analyses showed a highly polyclonal pattern in almost all the time points analysed, and absence of marked clones expanding and persisting over time (Figure 3B-G). These molecular profiles described by circIS closely resemble the IS results obtained from analysing liver and spleen genomic DNA at 3 months post-injection (Figure 3H, I, respectively). Indeed, the genomic integration profile revealed in these tissues and for all the NHP exhibited a remarkable polyclonal pattern (23457 LV IS in liver, 11314 LV IS in spleen tissue), in which nearly all unique IS were represented by 1-4 genomes. These results are consistent with the mostly quiescent state expected for the liver of treated NHPs, and which is also reflected by circIS. Moreover, on average ~2% of the circIS were shared with IS found in liver and spleen confirming the tissue origin of these LV IS.

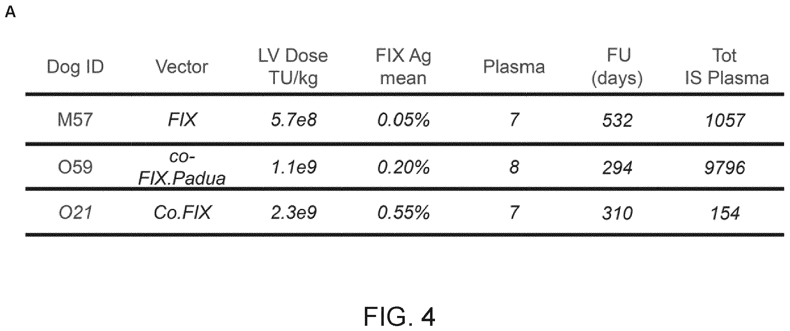

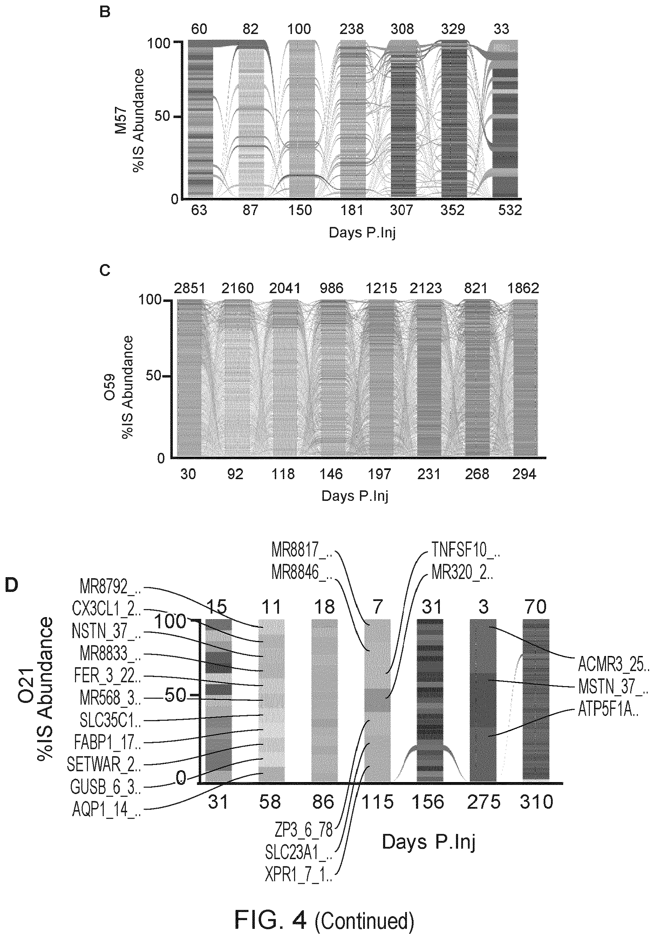

To further study the use of these methods as part of in vivo gene therapy procedures, circIS were retrieved from 3 haemophilic B male dogs that received a single LV injection by portal vein administration and had a longer follow up period of time (up to 522 days post injection). The LVs used for the treatment expressed the cDNA transgenes for canine factor IX (cFIX) under the control of a hepatocyte-specific expression cassette (Cantore, A. et al. (2012) Blood 120: 4517-4520). Each dog was injected using a different cFIX transgene: either wildtype (cFIX, M57 dog), codon-optimised (co-cFIX, O21 dog), or codon-optimised hyperfunctional cFIX carrying the Padua mutation associated with human thrombophilia (co-cFIXR338L, O59 dog) (Figure 4A). For each dog, circDNA was purified from 7-8 different plasma collections harvested starting from 30 to 532 days post-injection. 154 circIS were retrieved for O21, 1057 circIS for M57 and 9796 circIS for O59. As with the NHPs, all dogs analysed were characterised by a polyclonal pattern with many different ISs with low levels of abundance, and absence of dominant clones expanding and persisting over time (Figure4B-C). Despite the high variability in the overall amount of circIS recovered from the different animals, the number of circIS obtained among the different time points for each dog was consistent, suggesting a correlation with the level of clonality/transduction of the targeted organs.

Thus, the data shows that LiBIS-seq represents a valid procedure for IS studies in in vivo gene therapy applications. Compared to IS analyses using tissue biopsies, the retrieval of IS from circDNA is less risky, non-invasive and can be easily performed multiple times post treatment, thus providing a more comprehensive picture of the clonal repertoire of the transduced organs, as well as longitudinal monitoring of the transduced population.

### Retrieval of IS from the blood plasma of gene therapy patients who developed vector-induced T-cell acute lymphoblastic leukaemia (T-ALL) after haematopoietic stem cell gene therapy

The use of LiBIS-Seq for enabling the early identification of expanding marked clones residing in solid tissue was assessed.

This study utilised the clinical trials for γ-retroviral (RV)-based haematopoietic stem cell-based treatment of Severe Combined Immunodeficiency linked to X1 chromosome (SCID-X1). This trial was characterised by a high incidence of vector-driven side adverse events (SAE), since 5 out of 10 patients in the X-SCID trial developed T-ALL as a consequence of insertional mutagenesis. In all these T-ALL cases, γRV insertion led to the activation/deregulation of known proto-oncogenes, such as LMO2 (P4, P5, P9, P10 for SCID-X1) and CCND2 (P10 for SCID-X1), providing a proliferative advantage to the cell and favouring the neoplastic transformation process.

Conventional IS studies performed on DNA isolated from PBMCs or T cells purified from the blood of those patients failed to detect early signs of T-cell leukaemia. Because the LMO2, CCND2 and TAL1 clones associated with T-ALL suddenly expand in the blood cell of patients, at the latest time point preceding leukaemia they were either not detectable or detectable with a low contribution of ≤5%. These results were explained by the presence of a pre-leukemic clone that grew in the thymus niche and that was released into the circulation only after the acquisition of secondary mutation that led to full cell transformation.

In the present study, it was asked whether the retrieval of γRV IS from circDNA would allow early detection of leukemic clones that are expanding in peripheral organs, such as the thymus.

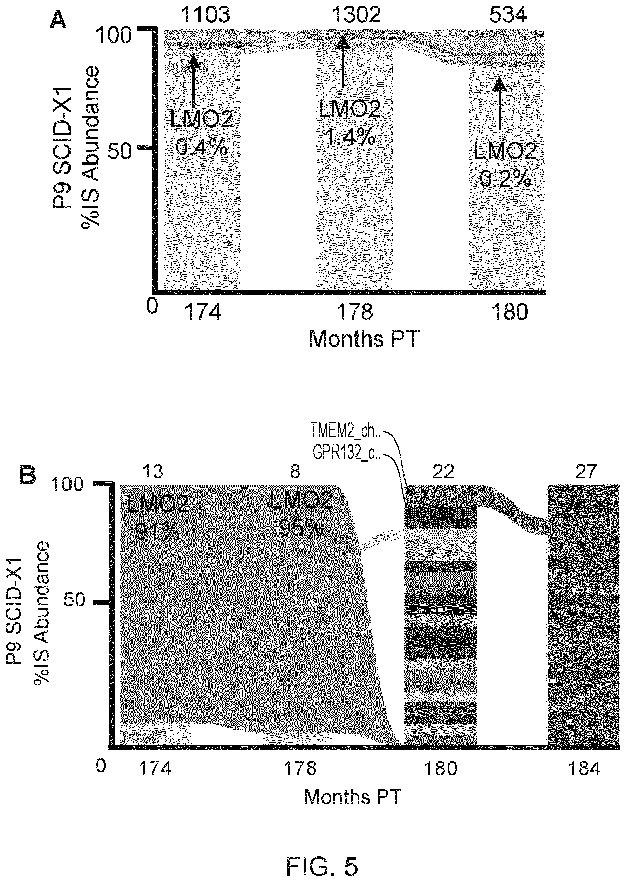

For the SCID-X1 gene therapy trial, γRV ISs were retrieved from plasma and PBMCs of patient P9, who developed an LMO2-driven T-ALL at 178 months post gene therapy. A total of 2635 cell IS were retrieved from PBMCs collected 4 months before the occurrence of the SAE, at the time of diagnosis and 2 months after chemotherapy (Figure 5A). The γRV insertion of the blast clone close to the LMO2 proto-oncogene was identified at all the time points analysed, however, the level of abundance of this γRV IS was very low with a highest peak of 1.4% at the time of diagnosis (178 months). On the other hand, 66 γRV circIS were retrieved from circDNA obtained from blood plasma collected 4 months before the occurrence of the SAE, at the time of diagnosis, and 2 and 4 months after chemotherapy (Figure 5B). Within this dataset, the γRV leukaemic IS was the most abundant at the time of diagnosis (95%) and even at 4 months before the T-ALL outcome (91%), while it became undetectable post-chemotherapy (Figure 5B).

Overall, these data show that LiBIS-Seq can identify and quantify vector-marked leukaemic clones growing in peripheral organs (such as the thymus) better than conventional IS studies performed on genomic DNA.

### CONCLUSIONS

The LiBIS-seq approach disclosed herein enables a more complete understanding of the clonal repertoire at the whole organism level in gene therapy patients, even revealing the presence of vector marked clones embedded in solid organs such as the liver. Moreover, it allows the early and sensitive detection of non-circulating premalignant clones triggered by insertional mutagenesis that may be residing in solid organs. These are in some cases barely detectable by conventional IS analysis. Hence, the method disclosed herein may be adopted as a strategy to study ISs in genomic DNA for safety monitoring of gene therapy patients, since it conveys information on transduced cell clones living in solid organs and could predict if clonal outgrowth of a transduced cell is occurring in peripheral organs (e.g. the LMO2-induced T-ALL developed in the SCIDX1 patients).

LiBIS-seq is highly informative in in vivo gene therapy procedures because for the first time this technology opens the door to longitudinal clonal tracking studies in in vivo gene therapy applications. Indeed, integration site studies are normally unfeasible in in vivo gene therapy procedures because the retrieval of ISs were previously strictly dependent on the collection of DNA with invasive tissue biopsies of the targeted organ. However, even when these surgical procedures are done, the amount of genomic information retrieved is limited to the single and small portion of tissue analysed. LiBIS-seq instead represents a non-invasive alternative to these surgical procedures because circDNA can be easily sampled, even at multiple times, from fluids such as blood plasma, thus overcoming the limitations of the analysis of single biopsies and providing an overall more comprehensive picture of the transduced organ.

### MATERIALS AND METHODS

### Purification of circDNA from blood plasma

Depending on the amount of the blood plasma available, different kits for circulating DNA purification were used: when 2 to 5 mL of plasma or serum was available, the QIAamp Circulating Nucleic Acid Kit was used (55114, QIAGEN); and when less than 2 mL of plasma was available, the Maxell RSC ccfDNA Plasma kit (AS1480, Promega) was used. In both cases, purification was performed following the manufacturer's instructions. After purification, cell-free DNA was immediately quantified and subjected to the LiBIS-seq procedure.

### LiBIS-seq: Retrieval of circulating IS from cell-free DNA

After purification, circulating cell-free DNA was split in 2 technical replicates, subjected to end repair and 3' adenylation using the NEBNext^{®} Ultra^{™} DNA Library Prep Kit for Illumina^{®} (New England Biolabs, Ipswich, MA.), and then ligated (DNA Technologies ligation kit, Skokie, IL.) to linker cassettes (LC) containing:
(i) an 8 nucleotide sequence barcode, used for sample identification;
(ii) a 12 nucleotide random sequence, used for quantification purposes; and
(iii) all the sequences required for the Read 2 Illumina paired end sequencing, tracked within our laboratory information management (Spinozzi, G. et al. (2017) BMC Bioinformatics 18: 520).

Ligation products were then subjected to 35 cycles of exponential PCR with primers complementary to lentiviral or retroviral vector LTR sequence, and the LC. Subsequently, ten additional PCR cycles were carried out, which added sequences required for sequencing.

Finally, the amplification products were sequenced using the Illumina Myseq/HiSeq platform (Illumina, San Diego, CA.). Sequencing reads were then processed as previously described (Spinozzi, G. et al. (2017) BMC Bioinformatics 18: 520). Briefly, paired sequence reads were filtered for quality standards, barcodes identified for sample demultiplexing of the sequence reads, the cellular genomic sequence mapped on the reference genome (Human or Crab-eating macaque (Human Genome_GRCh37/hg19 Feb. 2019, Macaca_fascicularis_5.0/macFas5, Jun. 2013) and the nearest RefSeq gene assigned to each unambiguously mapped integration site (IS).

The abundance of each IS was estimated by a method in which the number of genomes with the same IS is calculated by counting the different number of fragments identified within the cell-free DNA material. Then, the relative abundance of each IS is calculated as the percentage of cell-free DNA with a specific IS over the total number of fragment harbouring different IS identified (Firouzi, S. et al. (2014) Genome Medicine 6: 46; Gillet, N.A. et al. (2011) Blood 117: 3113-3122).

### Retrieval of circulating IS from cellular genomic DNA

For the retrieval of vector IS from genomic DNA, a sonication-based linker-mediated (LM) PCR method was used as previously described (Firouzi, S. et al. (2014) Genome Medicine 6: 46; Gillet, N.A. et al. (2011) Blood 117: 3113-3122). Briefly, genomic DNA was sheared using a Covaris E220 Ultrasonicator (Covaris Inc., Woburn (MA), generating fragments with an average size of 1000 bp. The fragmented DNA was then split into 3 technical replicates and subjected to end repair and 3' adenylation using the NEBNext^{®} Ultra^{™} DNA Library Prep Kit for Illumina^{®} (New England Biolabs, Ipswich, MA.), and then ligated (DNA Technologies ligation kit, Skokie, IL.) to linker cassettes (LC) as described above. Subsequently, PCR procedures were again applied for IS retrieval and mapping.

### Linker cassettes

The linker cassettes (LCs) have a partially double-stranded structure, which are generated by annealing two oligonucleotides:
A short oligonucleotide (5' to 3': GGATTGACGACACGGTGAC; SEQ ID NO: 1) is 5'-phosphorylated and at the 3' end contains a six carbon glycol spacer (hexanediol) capable of blocking extension by DNA polymerases.
A long oligonucleotide (described in Table 1) has a single 3' overhanging T, that allows ligation to the adenylated fragments. The T-overhang is linked to the first base in the oligonucleotide by a phosphorothioate bond, which renders the internucleotide linkage resistant to nuclease degradation.

The single-stranded portion contains: (i) a 12 nucleotide random sequence, that acts as Unique Molecular Identifier (UMI) for the fragments; (ii) an 8 nucleotide barcode for sample barcoding; and (iii) a sequence with the Illumina sequencing primer, P7.

**Table 1. Long oligonucleotide LC sequences (indicated from 5' to 3').**

| **Name** | **Sequence** | **SEQ ID NO** |
|---|---|---|
| FB-Block-LC.10 | GACGTGTGCTCTTCCGATCTNNNNNNNNNNNNAAGGTACAGTCACCGTGTCGTCAA TCC*T | 2 |
| FB-Block-LC.12 | GACGTGTGCTCTTCCGATCTNNNNNNNNNNNNNNAATGTTGCGTCACCGTGTCGTCAA TCC*T | 3 |
| FB-Block-LC.14 | GACGTGTGCTCTTCCGATCTNNNNNNNNNNNNACACAGAAGTCACCGTGTCGTCAA TCC*T | 4 |
| FB-Block-LC.16 | GACGTGTGCTCTTCCGATCTNNNNNNNNNNNNACAGATTCGTCACCGTGTCGTCAA TCC*T | 5 |
| FB-Block-LC.18 | GACGTGTGCTCTTCCGATCTNNNNNNNNNNNNACATTGGCGTCACCGTGTCGTCAA TCC*T | 6 |
| FB-Block-LC.2 | GACGTGTGCTCTTCCGATCTNNNNNNNNNNNNNAACAACCAGTCACCGTGTCGTCAA TCC*T | 7 |
| FB-Block-LC.20 | GACGTGTGCTCTTCCGATCTNNNNNNNNNNNNACCTCCAAGTCACCGTGTCGTCAA TCC*T | 8 |
| FB-Block-LC.22 | GACGTGTGCTCTTCCGATCTNNNNNNNNNNNNACGTATCAGTCACCGTGTCGTCAA TCC*T | 9 |
| FB-Block-LC.24 | GACGTGTGCTCTTCCGATCTNNNNNNNNNNNNNNAGAGTCAAGTCACCGTGTCGTCAA TCC*T | 10 |
| FB-Block-LC.26 | GACGTGTGCTCTTCCGATCTNNNNNNNNNNNNAGATGTACGTCACCGTGTCGTCAA TCC*T | 11 |
| FB-Block-LC.28 | GACGTGTGCTCTTCCGATCTNNNNNNNNNNNNNAGCAGGAAGTCACCGTGTCGTCAA TCC*T | 12 |
| FB-Block-LC.30 | GACGTGTGCTCTTCCGATCTNNNNNNNNNNNNAGGCTAACGTCACCGTGTCGTCAA TCC*T | 13 |
| FB-Block-LC.32 | GACGTGTGCTCTTCCGATCTNNNNNNNNNNNNAGTCACTAGTCACCGTGTCGTCAA TCC*T | 14 |
| FB-Block-LC.34 | GACGTGTGCTCTTCCGATCTNNNNNNNNNNNNATAGCGACGTCACCGTGTCGTCAA TCC*T | is |
| FB-Block-LC.36 | GACGTGTGCTCTTCCGATCTNNNNNNNNNNNNATCCTGTAGTCACCGTGTCGTCAA TCC*T | 16 |
| FB-Block-LC.38 | GACGTGTGCTCTTCCGATCTNNNNNNNNNNNNATTGAGGAGTCACCGTGTCGTCAA TCC*T | 17 |
| FB-Block-LC.4 | GACGTGTGCTCTTCCGATCTNNNNNNNNNNNNAACGCTTAGTCACCGTGTCGTCAA TCC*T | 18 |
| FB-Block-LC.40 | GACGTGTGCTCTTCCGATCTNNNNNNNNNNNNNNCAACCACAGTCACCGTGTCGTCAA TCC*T | 19 |
| FB-Block-LC.42 | GACGTGTGCTCTTCCGATCTNNNNNNNNNNNNCAAGGAGCGTCACCGTGTCGTCAA TCC*T | 20 |
| FB-Block-LC.44 | GACGTGTGCTCTTCCGATCTNNNNNNNNNNNNCACCTTACGTCACCGTGTCGTCAA TCC*T | 21 |
| FB-Block-LC.46 | GACGTGTGCTCTTCCGATCTNNNNNNNNNNNNCAGATCTGGTCACCGTGTCGTCAA TCC*T | 22 |
| FB-Block-LC.48 | GACGTGTGCTCTTCCGATCTNNNNNNNNNNNNCATACCAAGTCACCGTGTCGTCAA TCC*T | 23 |
| FB-Block-LC.50 | GACGTGTGCTCTTCCGATCTNNNNNNNNNNNNCCAGTTCAGTCACCGTGTCGTCAA TCC*T | 24 |
| FB-Block-LC.52 | GACGTGTGCTCTTCCGATCTNNNNNNNNNNNNNNCCGAAGTAGTCACCGTGTCGTCAA TCC*T | 25 |
| FB-Block-LC.54 | GACGTGTGCTCTTCCGATCTNNNNNNNNNNNNCCGTGAGAGTCACCGTGTCGTCAA TCC*T | 26 |
| FB-Block-LC.56 | GACGTGTGCTCTTCCGATCTNNNNNNNNNNNNNNNCCTCCTGAGTCACCGTGTCGTCAA TCC*T | 27 |
| FB-Block-LC.58 | GACGTGTGCTCTTCCGATCTNNNNNNNNNNNNCGAACTTAGTCACCGTGTCGTCAA TCC*T | 28 |
| FB-Block-LC.6 | GACGTGTGCTCTTCCGATCTNNNNNNNNNNNNNAACTCACCGTCACCGTGTCGTCAA TCC*T | 29 |
| FB-Block-LC.60 | GACGTGTGCTCTTCCGATCTNNNNNNNNNNNNNCGACTGGAGTCACCGTGTCGTCAA TCC*T | 30 |
| FB-Block-LC.62 | GACGTGTGCTCTTCCGATCTNNNNNNNNNNNNNNNNCGCTGATCGTCACCGTGTCGTCAA TCC*T | 31 |
| FB-Block-LC.64 | GACGTGTGCTCTTCCGATCTNNNNNNNNNNNNNCTAAGGTCGTCACCGTGTCGTCAA TCC*T | 32 |
| FB-Block-LC.66 | GACGTGTGCTCTTCCGATCTNNNNNNNNNNNNNNNNCTGAGCCAGTCACCGTGTCGTCAA TCC*T | 33 |
| FB-Block-LC.68 | GACGTGTGCTCTTCCGATCTNNNNNNNNNNNNNCTGTAGCCGTCACCGTGTCGTCAA TCC*T | 34 |
| FB-Block-LC.70 | GACGTGTGCTCTTCCGATCTNNNNNNNNNNNNGAATCTGAGTCACCGTGTCGTCAA TCC*T | 35 |
| FB-Block-LC.72 | GACGTGTGCTCTTCCGATCTNNNNNNNNNNNNGACTAGTAGTCACCGTGTCGTCAA TCC*T | 36 |
| FB-Block-LC.74 | GACGTGTGCTCTTCCGATCTNNNNNNNNNNNNGAGTTAGCGTCACCGTGTCGTCAA TCC*T | 37 |
| FB-Block-LC.76 | GACGTGTGCTCTTCCGATCTNNNNNNNNNNNNGATGAATCGTCACCGTGTCGTCAA TCC*T | 38 |
| FB-Block-LC.78 | GACGTGTGCTCTTCCGATCTNNNNNNNNNNNNGCCACATAGTCACCGTGTCGTCAA TCC*T | 39 |
| FB-Block-LC.8 | GACGTGTGCTCTTCCGATCTNNNNNNNNNNNNAAGAGATCGTCACCGTGTCGTCAA TCC*T | 40 |
| FB-Block-LC.80 | GACGTGTGCTCTTCCGATCTNNNNNNNNNNNNGCTAACGAGTCACCGTGTCGTCAA TCC*T | 41 |
| FB-Block-LC.82 | GACGTGTGCTCTTCCGATCTNNNNNNNNNNNNGGAGAACAGTCACCGTGTCGTCAA TCC*T | 42 |
| FB-Block-LC.84 | GACGTGTGCTCTTCCGATCTNNNNNNNNNNNNNNGTACGCAAGTCACCGTGTCGTCAA TCC*T | 43 |
| FB-Block-LC.86 | GACGTGTGCTCTTCCGATCTNNNNNNNNNNNNNGTCTGTCAGTCACCGTGTCGTCAA TCC*T | 44 |
| FB-Block-LC.88 | GACGTGTGCTCTTCCGATCTNNNNNNNNNNNNTAGGATGAGTCACCGTGTCGTCAA TCC*T | 45 |
| FB-Block-LC.90 | GACGTGTGCTCTTCCGATCTNNNNNNNNNNNNTCCGTCTAGTCACCGTGTCGTCAA TCC*T | 46 |
| FB-Block-LC.92 | GACGTGTGCTCTTCCGATCTNNNNNNNNNNNNNTGAAGAGAGTCACCGTGTCGTCAA TCC*T | 47 |
| FB-Block-LC.94 | GACGTGTGCTCTTCCGATCTNNNNNNNNNNNNNTGGCTTCAGTCACCGTGTCGTCAA TCC*T | 48 |
| FB-Block-LC.96 | GACGTGTGCTCTTCCGATCTNNNNNNNNNNNNTTCACGCAGTCACCGTGTCGTCAA TCC*T | 49 |

### PCR primers

The primers used in the first PCR amplification were:
Fw1: binds to vector LTR: AGCTTGCCTTGAGTGCTTCA (SEQ ID NO: 50)
Rw1: binds to vector P7 sequence: GACGTGTGCTCTTCCGATC (SEQ ID NO: 51)

The second PCR amplification was performed with Rw1 and an LTR-binding oligonucleotide (Table 2), which contained: (i) a common portion on the 3' end that annealed to vector LTR; (ii) an 8 nucleotide barcode for sample barcoding; (iii) a 12 nucleotide random sequence; and (iv) a sequence with Illumina sequencing primer, P5.

**Table 2. LTR-binding oligonucleotides (indicated from 5' to 3').**

| **Name** | **Sequence** | **SEQ ID NO** |
|---|---|---|
| LTR.1 | AATGATACGGCGACCACCGAGATCTACACTCTTTCCCTACACGACGCTCTTCCGAT CTNNNNNNNNNNNNAAACATCGACCCTTTTAGTCAGTGTGGA | 52 |
| LTR.9 | AATGATACGGCGACCACCGAGATCTACACTCTTTCCCTACACGACGCTCTTCCGAT CTNNNNNNNNNNNNNAAGGACACACCCTTTTAGTCAGTGTGGA | 53 |
| LTR.17 | AATGATACGGCGACCACCGAGATCTACACTCTTTCCCTACACGACGCTCTTCCGAT CTNNNNNNNNNNNNNACAGCAGAACCCTTTTAGTCAGTGTGGA | 54 |
| LTR.25 | AATGATACGGCGACCACCGAGATCTACACTCTTTCCCTACACGACGCTCTTCCGAT CTNNNNNNNNNNNNAGATCGCAACCCTTTTAGTCAGTGTGGA | 55 |
| LTR.33 | AATGATACGGCGACCACCGAGATCTACACTCTTTCCCTACACGACGCTCTTCCGAT CTNNNNNNNNNNNNAGTGGTCAACCCTTTTAGTCAGTGTGGA | 56 |
| LTR.41 | AATGATACGGCGACCACCGAGATCTACACTCTTTCCCTACACGACGCTCTTCCGAT CTNNNNNNNNNNNNCAAGACTAACCCTTTTAGTCAGTGTGGA | 57 |
| LTR.3 | AATGATACGGCGACCACCGAGATCTACACTCTTTCCCTACACGACGCTCTTCCGAT CTNNNNNNNNNNNNAACCGAGAACCCTTTTAGTCAGTGTGGA | 58 |
| LTR.11 | AATGATACGGCGACCACCGAGATCTACACTCTTTCCCTACACGACGCTCTTCCGAT CTNNNNNNNNNNNNAATCCGTCACCCTTTTAGTCAGTGTGGA | 59 |
| LTR.19 | AATGATACGGCGACCACCGAGATCTACACTCTTTCCCTACACGACGCTCTTCCGAT CTNNNNNNNNNNNNACCACTGTACCCTTTTAGTCAGTGTGGA | 60 |
| LTR.27 | AATGATACGGCGACCACCGAGATCTACACTCTTTCCCTACACGACGCTCTTCCGAT CTNNNNNNNNNNNNAGCACCTCACCCTTTTAGTCAGTGTGGA | 61 |
| LTR.35 | AATGATACGGCGACCACCGAGATCTACACTCTTTCCCTACACGACGCTCTTCCGAT CTNNNNNNNNNNNNATCATTCCACCCTTTTAGTCAGTGTGGA | 62 |
| LTR.43 | AATGATACGGCGACCACCGAGATCTACACTCTTTCCCTACACGACGCTCTTCCGAT CTNNNNNNNNNNNNCAATGGAAACCCTTTTAGTCAGTGTGGA | 63 |
| LTR.5 | AATGATACGGCGACCACCGAGATCTACACTCTTTCCCTACACGACGCTCTTCCGAT CTNNNNNNNNNNNNAACGTGATACCCTTTTAGTCAGTGTGGA | 64 |
| LTR.13 | AATGATACGGCGACCACCGAGATCTACACTCTTTCCCTACACGACGCTCTTCCGAT CTNNNNNNNNNNNNACAAGCTAACCCTTTTAGTCAGTGTGGA | 65 |
| LTR.21 | AATGATACGGCGACCACCGAGATCTACACTCTTTCCCTACACGACGCTCTTCCGAT CTNNNNNNNNNNNNNACGCTCGAACCCTTTTAGTCAGTGTGGA | 66 |
| LTR.29 | AATGATACGGCGACCACCGAGATCTACACTCTTTCCCTACACGACGCTCTTCCGAT CTNNNNNNNNNNNNNAGCCATGCACCCTTTTAGTCAGTGTGGA | 67 |
| LTR.37 | AATGATACGGCGACCACCGAGATCTACACTCTTTCCCTACACGACGCTCTTCCGAT CTNNNNNNNNNNNNATGCCTAAACCCTTTTAGTCAGTGTGGA | 68 |
| LTR.45 | AATGATACGGCGACCACCGAGATCTACACTCTTTCCCTACACGACGCTCTTCCGAT CTNNNNNNNNNNNNNNCACTTCGAACCCTTTTAGTCAGTGTGGA | 69 |
| LTR. 7 | AATGATACGGCGACCACCGAGATCTACACTCTTTCCCTACACGACGCTCTTCCGAT CTNNNNNNNNNNNNAAGACGGAACCCTTTTAGTCAGTGTGGA | 70 |
| LTR.15 | AATGATACGGCGACCACCGAGATCTACACTCTTTCCCTACACGACGCTCTTCCGAT CTNNNNNNNNNNNNNACACGACCACCCTTTTAGTCAGTGTGGA | 71 |
| LTR.23 | AATGATACGGCGACCACCGAGATCTACACTCTTTCCCTACACGACGCTCTTCCGAT CTNNNNNNNNNNNNNNACTATGCAACCCTTTTAGTCAGTGTGGA | 72 |
| LTR.31 | AATGATACGGCGACCACCGAGATCTACACTCTTTCCCTACACGACGCTCTTCCGAT CTNNNNNNNNNNNNNAGTACAAGACCCTTTTAGTCAGTGTGGA | 73 |
| LTR.39 | AATGATACGGCGACCACCGAGATCTACACTCTTTCCCTACACGACGCTCTTCCGAT CTNNNNNNNNNNNNATTGGCTCACCCTTTTAGTCAGTGTGGA | 74 |
| LTR.47 | AATGATACGGCGACCACCGAGATCTACACTCTTTCCCTACACGACGCTCTTCCGAT CTNNNNNNNNNNNNCAGCGTTAACCCTTTTAGTCAGTGTGGA | 75 |
| LTR.49 | AATGATACGGCGACCACCGAGATCTACACTCTTTCCCTACACGACGCTCTTCCGAT CTNNNNNNNNNNNNCATCAAGTACCCTTTTAGTCAGTGTGGA | 76 |
| LTR.57 | AATGATACGGCGACCACCGAGATCTACACTCTTTCCCTACACGACGCTCTTCCGAT CTNNNNNNNNNNNNCCTCTATCACCCTTTTAGTCAGTGTGGA | 77 |
| LTR.65 | AATGATACGGCGACCACCGAGATCTACACTCTTTCCCTACACGACGCTCTTCCGAT CTNNNNNNNNNNNNCTCAATGAACCCTTTTAGTCAGTGTGGA | 78 |
| LTR.73 | AATGATACGGCGACCACCGAGATCTACACTCTTTCCCTACACGACGCTCTTCCGAT CTNNNNNNNNNNNNNGAGCTGAAACCCTTTTAGTCAGTGTGGA | 79 |
| LTR.81 | AATGATACGGCGACCACCGAGATCTACACTCTTTCCCTACACGACGCTCTTCCGAT CTNNNNNNNNNNNNGCTCGGTAACCCTTTTAGTCAGTGTGGA | 80 |
| LTR.89 | AATGATACGGCGACCACCGAGATCTACACTCTTTCCCTACACGACGCTCTTCCGAT CTNNNNNNNNNNNNTATCAGCAACCCTTTTAGTCAGTGTGGA | 81 |
| LTR.51 | AATGATACGGCGACCACCGAGATCTACACTCTTTCCCTACACGACGCTCTTCCGAT CTNNNNNNNNNNNNCCATCCTCACCCTTTTAGTCAGTGTGGA | 82 |
| LTR.59 | AATGATACGGCGACCACCGAGATCTACACTCTTTCCCTACACGACGCTCTTCCGAT CTNNNNNNNNNNNNCGACACACACCCTTTTAGTCAGTGTGGA | 83 |
| LTR.67 | AATGATACGGCGACCACCGAGATCTACACTCTTTCCCTACACGACGCTCTTCCGAT CTNNNNNNNNNNNNNCTGGCATAACCCTTTTAGTCAGTGTGGA | 84 |
| LTR.75 | AATGATACGGCGACCACCGAGATCTACACTCTTTCCCTACACGACGCTCTTCCGAT CTNNNNNNNNNNNNGATAGACAACCCTTTTAGTCAGTGTGGA | 85 |
| LTR.83 | AATGATACGGCGACCACCGAGATCTACACTCTTTCCCTACACGACGCTCTTCCGAT CTNNNNNNNNNNNNGGTGCGAAACCCTTTTAGTCAGTGTGGA | 86 |
| LTR.91 | AATGATACGGCGACCACCGAGATCTACACTCTTTCCCTACACGACGCTCTTCCGAT CTNNNNNNNNNNNNTCTTCACAACCCTTTTAGTCAGTGTGGA | 87 |
| LTR.53 | AATGATACGGCGACCACCGAGATCTACACTCTTTCCCTACACGACGCTCTTCCGAT CTNNNNNNNNNNNNCCGACAACACCCTTTTAGTCAGTGTGGA | 88 |
| LTR.61 | AATGATACGGCGACCACCGAGATCTACACTCTTTCCCTACACGACGCTCTTCCGAT CTNNNNNNNNNNNNCGCATACAACCCTTTTAGTCAGTGTGGA | 89 |
| LTR.69 | AATGATACGGCGACCACCGAGATCTACACTCTTTCCCTACACGACGCTCTTCCGAT CTNNNNNNNNNNNNGAACAGGCACCCTTTTAGTCAGTGTGGA | 90 |
| LTR.77 | AATGATACGGCGACCACCGAGATCTACACTCTTTCCCTACACGACGCTCTTCCGAT CTNNNNNNNNNNNNGCCAAGACACCCTTTTAGTCAGTGTGGA | 91 |
| LTR.85 | AATGATACGGCGACCACCGAGATCTACACTCTTTCCCTACACGACGCTCTTCCGAT CTNNNNNNNNNNNNNGTCGTAGAACCCTTTTAGTCAGTGTGGA | 92 |
| LTR.93 | AATGATACGGCGACCACCGAGATCTACACTCTTTCCCTACACGACGCTCTTCCGAT CTNNNNNNNNNNNNTGGAACAAACCCTTTTAGTCAGTGTGGA | 93 |
| LTR.55 | AATGATACGGCGACCACCGAGATCTACACTCTTTCCCTACACGACGCTCTTCCGAT CTNNNNNNNNNNNNCCTAATCCACCCTTTTAGTCAGTGTGGA | 94 |
| LTR.63 | AATGATACGGCGACCACCGAGATCTACACTCTTTCCCTACACGACGCTCTTCCGAT CTNNNNNNNNNNNNCGGATTGCACCCTTTTAGTCAGTGTGGA | 95 |
| LTR.71 | AATGATACGGCGACCACCGAGATCTACACTCTTTCCCTACACGACGCTCTTCCGAT CTNNNNNNNNNNNNNGACAGTGCACCCTTTTAGTCAGTGTGGA | 96 |
| LTR.79 | AATGATACGGCGACCACCGAGATCTACACTCTTTCCCTACACGACGCTCTTCCGAT CTNNNNNNNNNNNNGCGAGTAAACCCTTTTAGTCAGTGTGGA | 97 |
| LTR.87 | AATGATACGGCGACCACCGAGATCTACACTCTTTCCCTACACGACGCTCTTCCGAT CTNNNNNNNNNNNNGTGTTCTAACCCTTTTAGTCAGTGTGGA | 98 |
| LTR.95 | AATGATACGGCGACCACCGAGATCTACACTCTTTCCCTACACGACGCTCTTCCGAT CTNNNNNNNNNNNNTGGTGGTAACCCTTTTAGTCAGTGTGGA | 99 |

### EXAMPLE 2

The inventors further investigated Liquid Biopsy Insertion Site Sequencing (LiBIS-seq) as disclosed below.

### LiBIS-Seq allows the retrieval of insertion sites (IS) from cell-free DNA (cfDNA) of metachromatic leukodystrophy (MLD) patients treated by lentiviral vector (LV)-based haematopoietic stem cell gene therapy (HSC-GT)

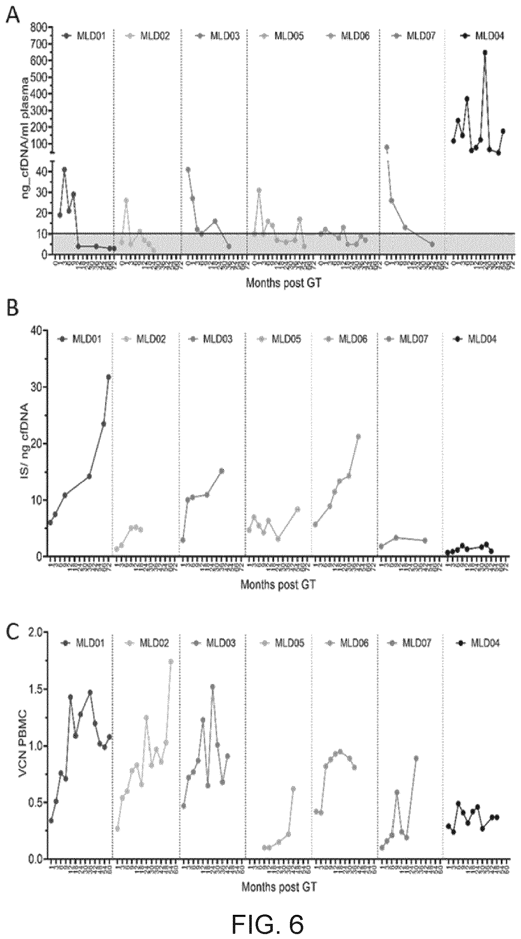

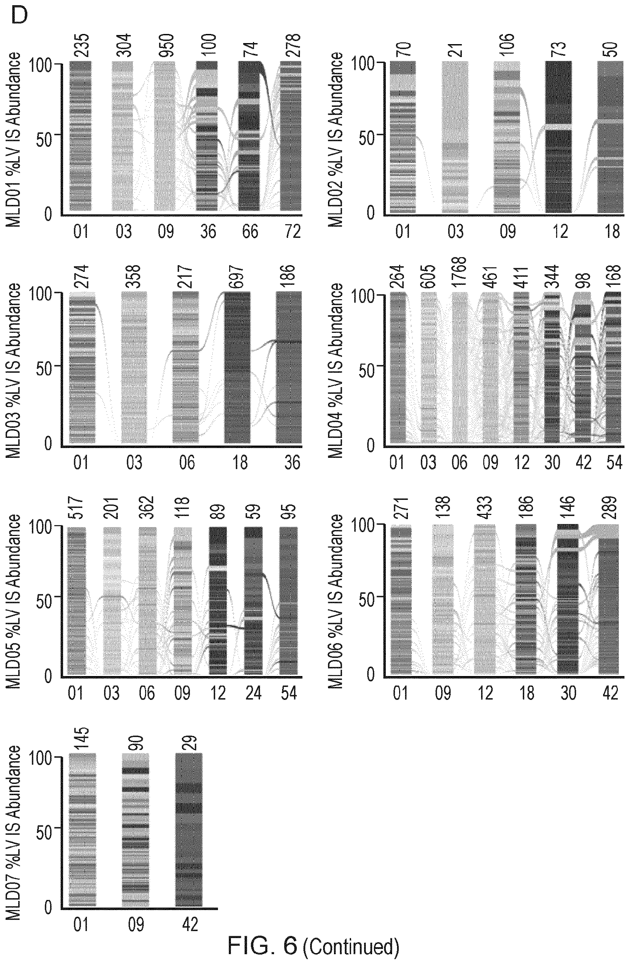

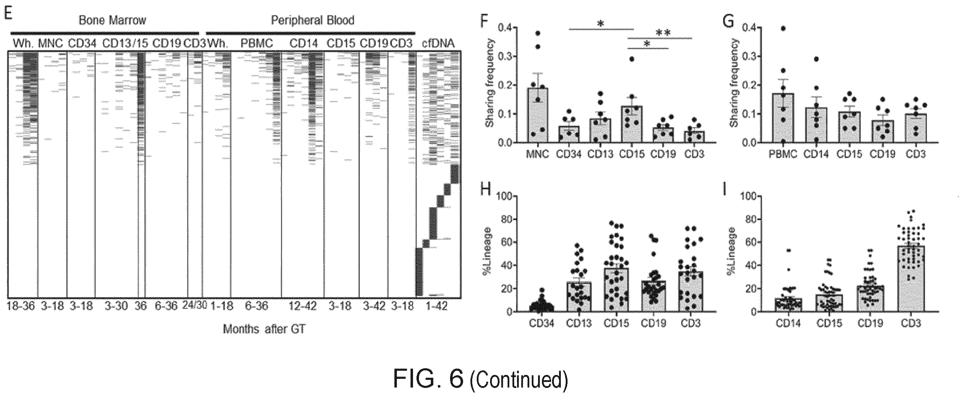

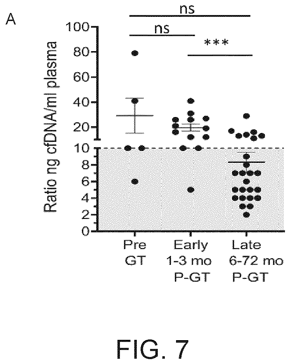

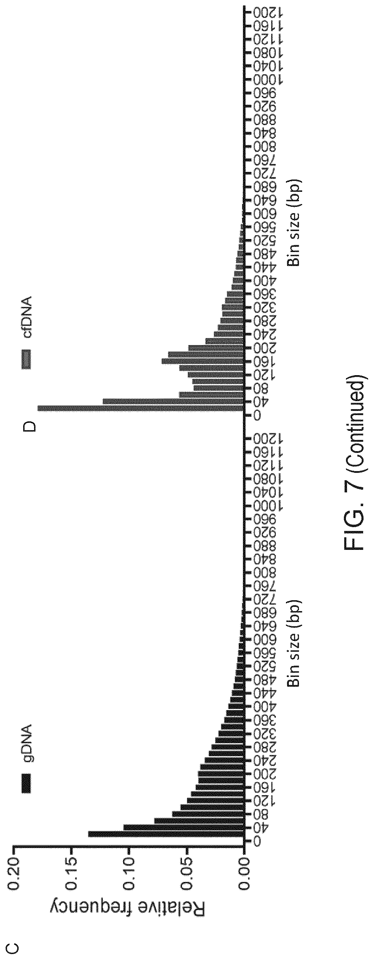

The inventors used a PCR protocol for IS retrieval to amplify the LV IS present in the cfDNA purified from 1-2 ml of blood-plasma collected at 4-8 different times post infusion of LV-transduced autologous CD34+ cells (range 1-72 months) from 7 HSC-GT Metachromatic Leukodystrophy (MLD) patients (Biffi, A. et al. (2013) Science 1233158; Sessa, M. et al. (2016) Lancet 388: 476-487). The amount of cfDNA recovered for each ml of plasma varied (ranging from 3 to ~600 ng/ml) with the time of blood collection and disease status of each patient (Figure 6A). By plotting the ng of cfDNA/ml over time we observed that before conditioning the concentration of cfDNA was above the pathological threshold of 10ng/ml in 5 out of 6 patients, while in all patients (7/7) it always reached pathological levels in the first 3 months post-HSC GT and then progressively decreased overtime to reach the normal range in most of the patients (Figure 7A). The only exception to this trend was represented by MLD04, which displayed very high plasma concentrations of cfDNA ranging from 46 ng/ml to 648ng/ml. MLD04 was the only patient of this cohort who was affected by the early juvenile form of the disease and for which the GT procedure was poorly effective since he was treated when already symptomatic and experienced disease progression. Overall, these data suggest that the amount of cfDNA retrieved from MLD patients may correlate with disease status (Figure 6A).

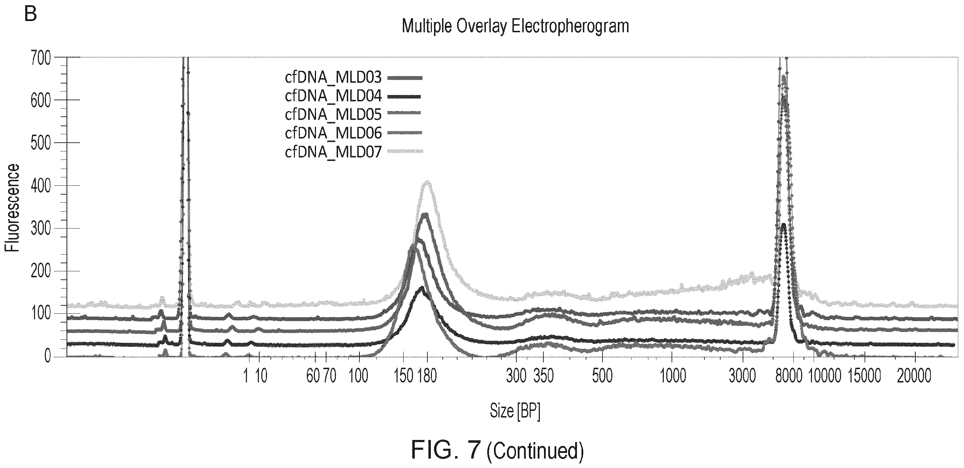

Purified cfDNA showed a fragment size distribution ranging from 100 bp to 220 bp with a peak at 167 bp, corresponding to the mononuclesomal protected fraction and followed by a faint peak between 300 and 370 bp corresponding to dinucleosomal fragments (Figure 7B).

After purification, cfDNA was subjected to end repair, phosphorylation, A-tailing, ligation to a synthetic barcoded DNA adaptor and to two rounds of PCR amplification with nested oligonucleotides complementary to the LV LTR and the DNA adaptor and sequenced using an Illumina platform. Sequencing data were then analysed with the bioinformatics pipeline VISPA228 to map the IS on the genome, identify the nearest gene and quantify the relative abundance of each IS (). The distribution of fragment sizes after PCR amplification and sequencing was similar (Figure 7C) to the distribution of PCR fragments obtained when LV IS were retrieved from sonicated cell-derived genomic DNA, with some surviving overrepresentation of the 100-220 bp size. From 38 cfDNA samples of the 7 MLD patients, we retrieved a total of >10,900 unique IS, with an average of 265 ± 163 IS for each time point analysed (Table 3). The number of IS retrieved at each time point for ng of cfDNA, increased over time in 5 out of 7 patients (Figure 6B). The progressive increase in IS retrieved by LiBIS-seq followed the increase in VCN measured in PBMC after transplantation (Figure 6C).

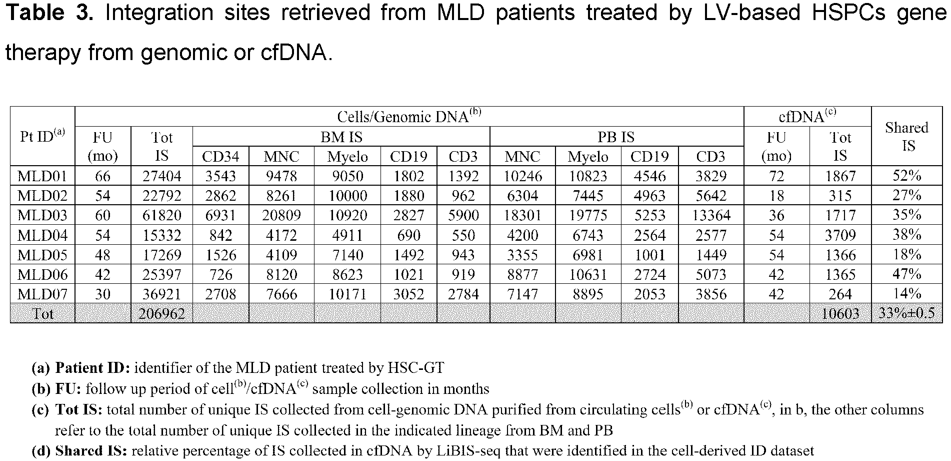

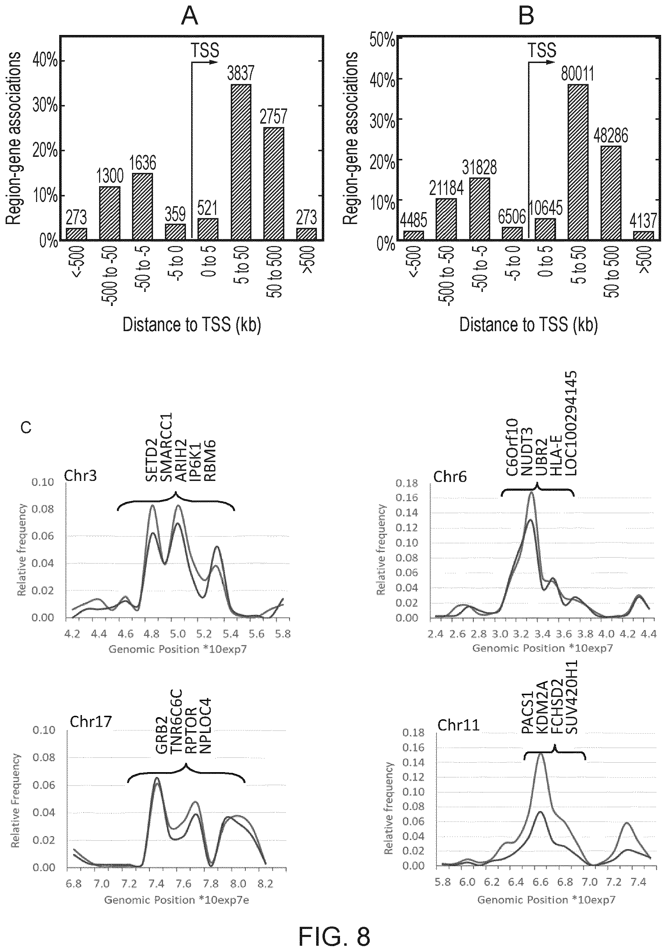

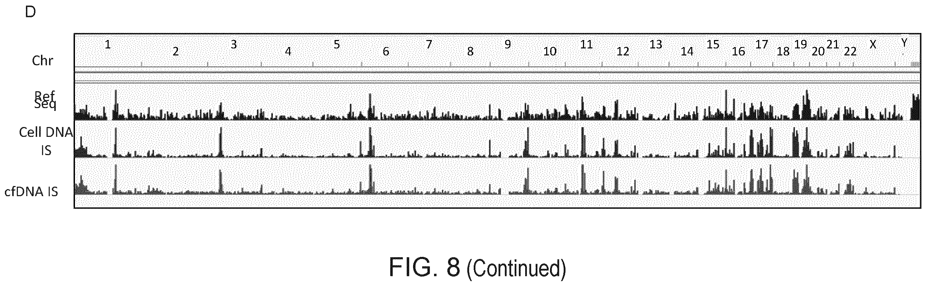

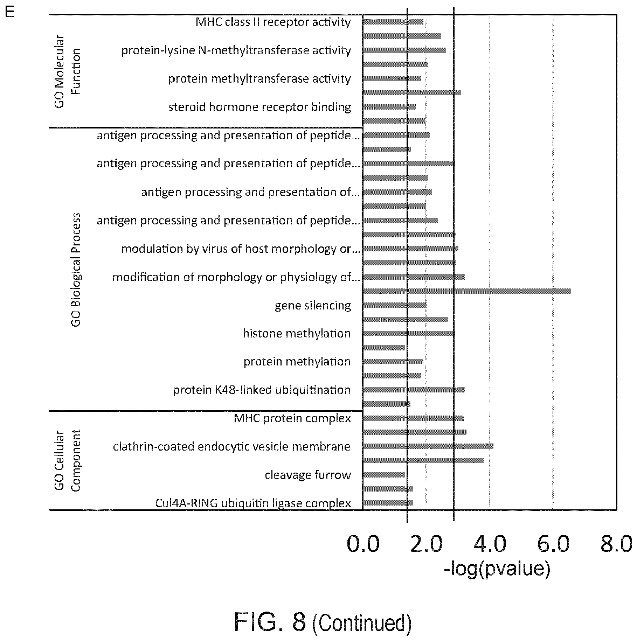

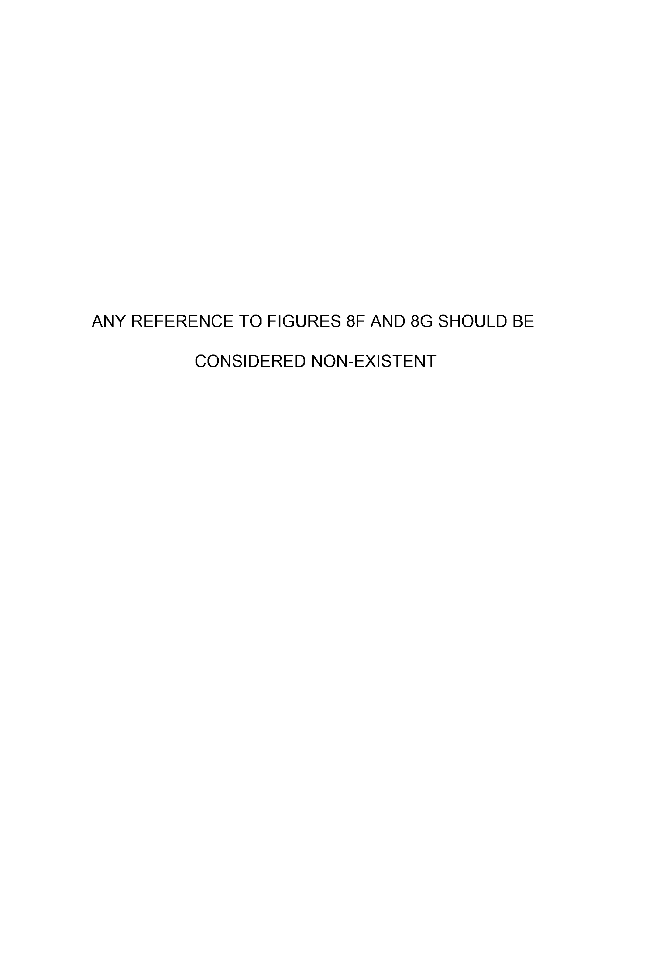

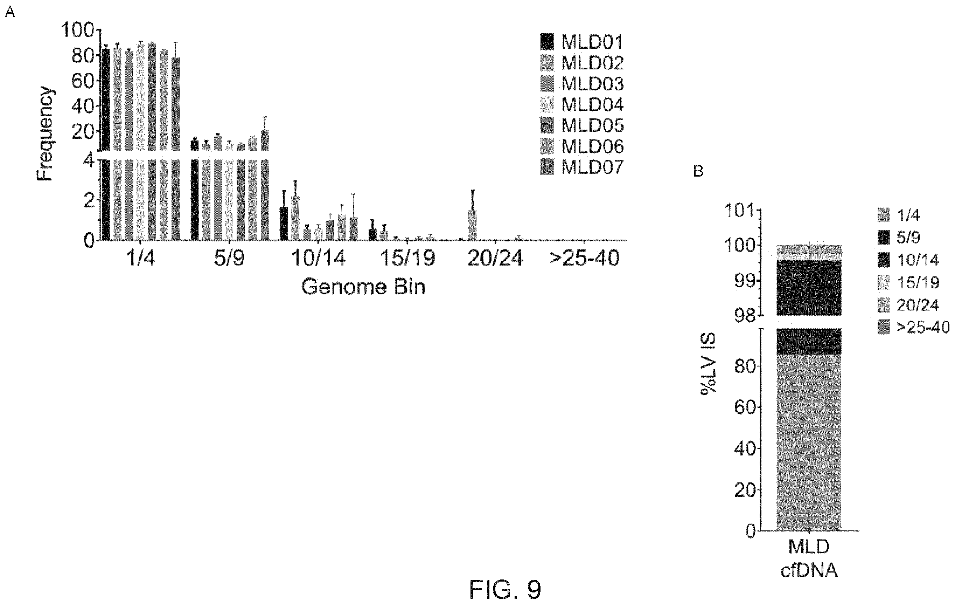

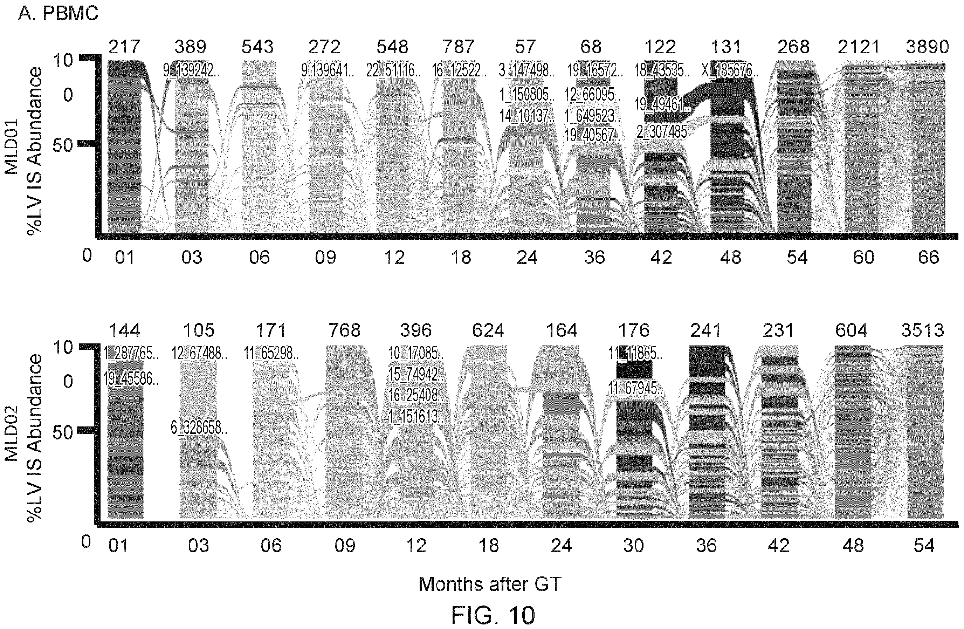

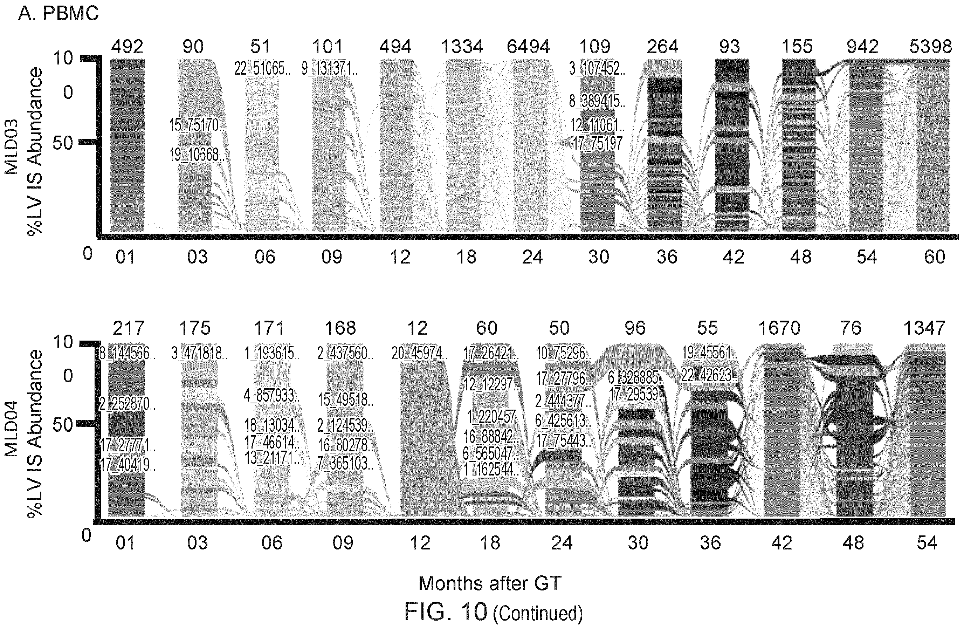

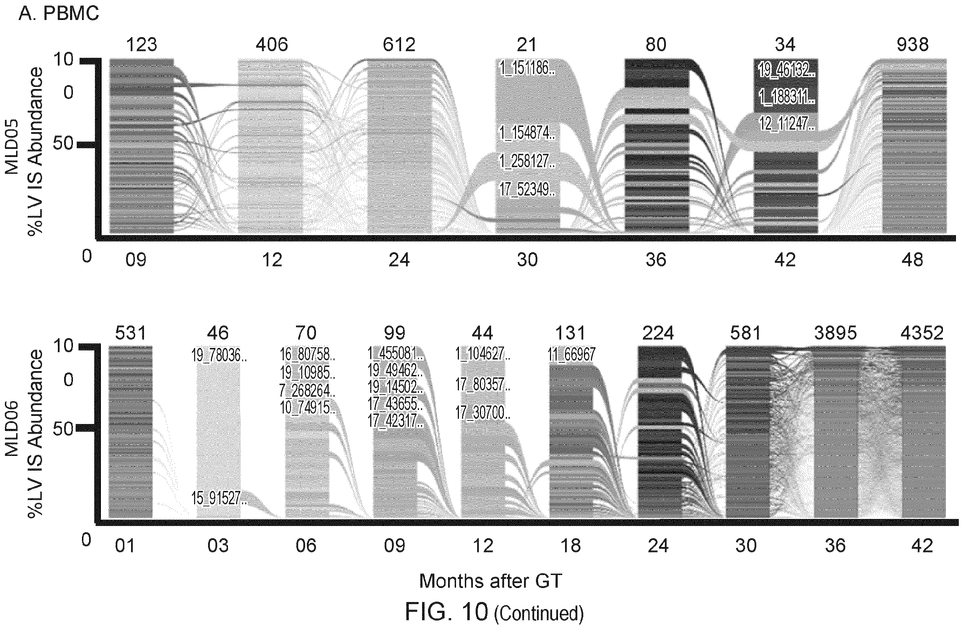

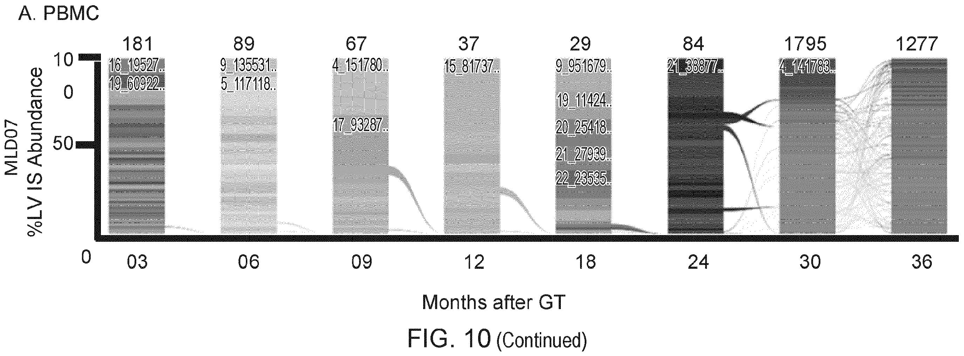

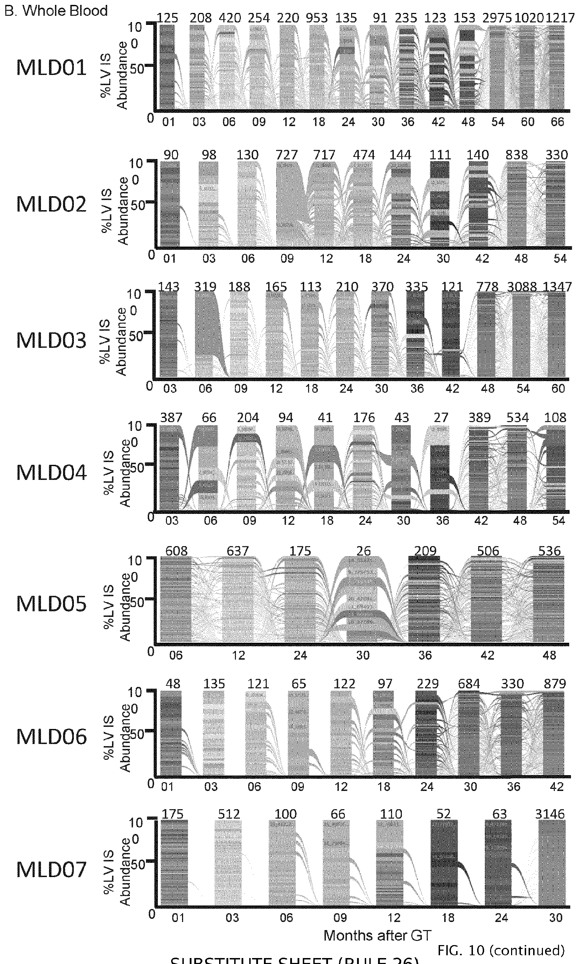

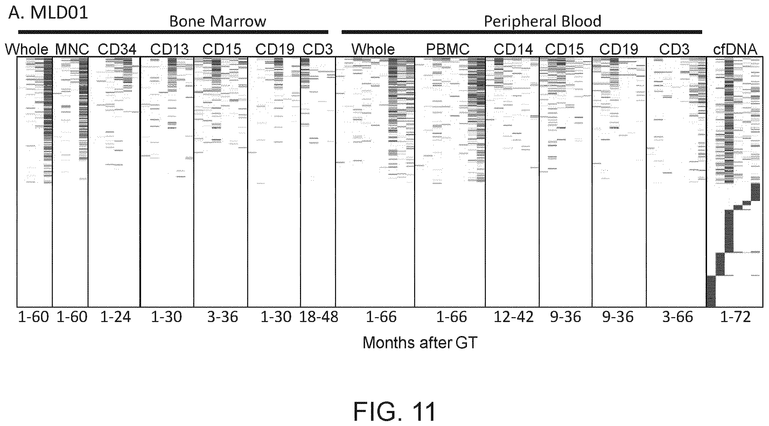

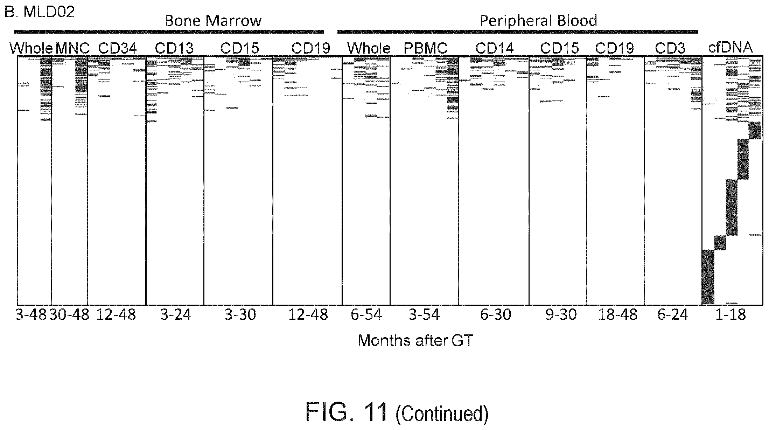

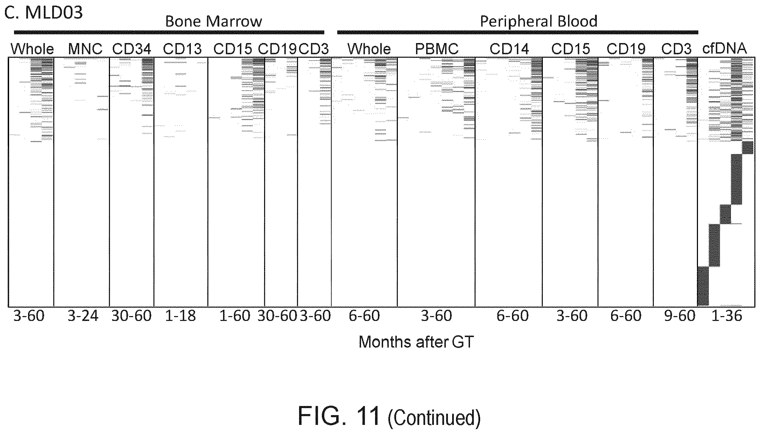

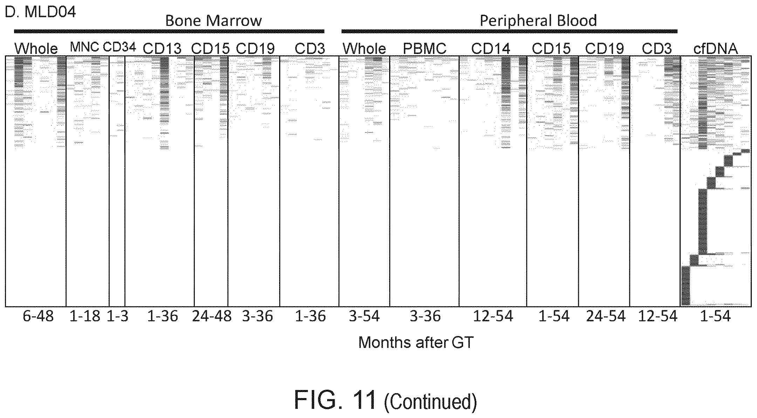

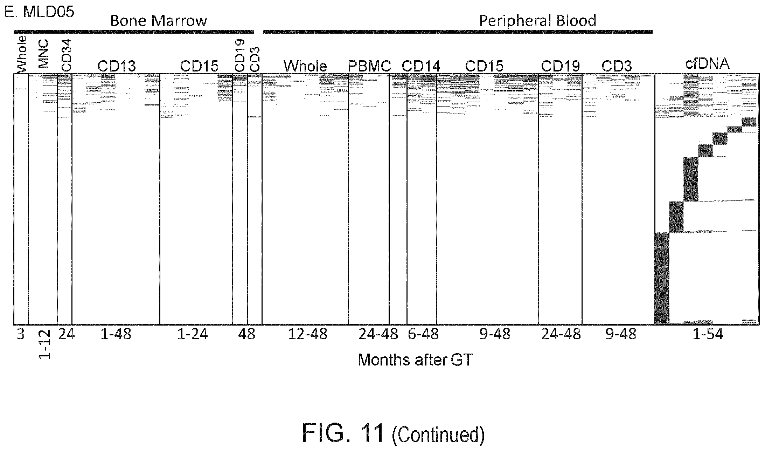

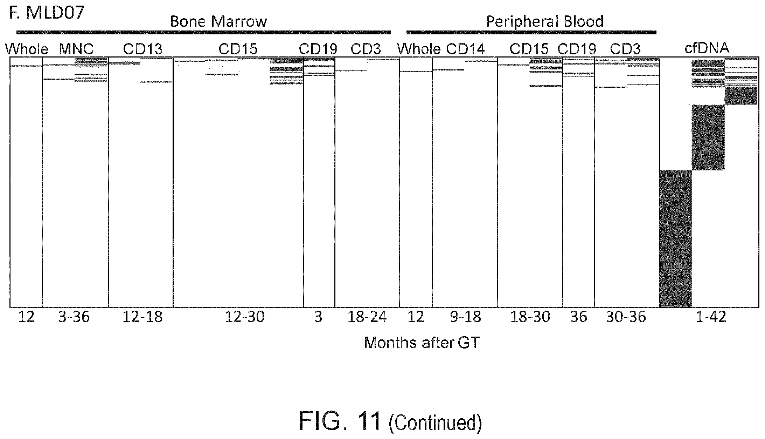

The genomic integration profile of cfDNA derived IS was highly similar to the classical LV integration profile described in previous IS studies on HSPCs and to the IS dataset specifically obtained from cell-derived genomic DNA collected from different BM and PBMC lineages of the same 7 MLD patients over a similar follow-up period by established PCR approaches (Table 3). The inventors showed: A) Marked tendency for IS to map within genes along the entire transcription unit (Figure 8A, B); B) Preference for megabase-wide genomic hot spots found in gene dense regions (Figure 8C, D) and without significant Common Insertion Sites; C) Enrichment of IS targeting gene classes involved in chromatin remodeling, HLA, virus host interactions and other similar ontological gene classes (Figure 8E). The abundance of each IS retrieved by LiBIS-seq was estimated using "SonicLength", a mathematical model that quantifys each IS considering the different number of DNA fragments based on the genomic shear site position and accounting for saturation events. Therefore, the number of different shear sites assigned to an IS will be proportional to the initial number of contributing cells, thus allowing estimation of the clonal abundance in the starting sample avoiding the biases introduced by PCR amplification. By applying this approach we observed that about 85.6% ± 1.15 of cfDNA derived IS were represented by 1 to 4 genomes, 12.8% ± 1.04 by 5 to 9 genomes and the remaining 1.6% ± 0.43 by 10 to 40 genomes (Figure 9). The average relative abundance of each IS with respect to the overall cfDNA IS dataset was low (0.82% ± 0.12) and no dominant IS expanding and/or persisting over time were observed in any patient (Figure 6B). ISs retrieved from cfDNA appeared and disappeared over time and their fluctuations in abundance levels was similar to clonal abundance evaluations carried on blood cells with previous analyses (Figure 10A, B) 20,26. Comparing the cfDNA IS data set to that encompassing IS retrieved from mononuclear cells (MNCs) and individual cell lineages purified from PB or BM, we found that on average 33± 0.5% of cfDNA-derived IS where shared between the two datasets. Considering the limitation imposed by sparse sampling of each IS source, these data indicate IS retrieved by LiBIS-seq were indeed originating from LV marked cells (Figure 6E-G, Figure 11 and Table 3). By measuring the level of sharing between cfDNA-derived IS and IS obtained from BM-purified HSPCs (CD34+), myeloid (CD13 and CD15), B (CD19) and T (CD3) cells, we observed that cfDNA-derived IS were significantly enriched for IS from CD15 cells compared to CD34+, T and B cells (Figure 6F). This higher sharing could be explained by the higher representation and turnover of myeloid cells compared to other BM lineages. Intriguingly, while in PB T cells were the most abundant population, cfDNA-derived IS did not show higher sharing with IS obtained from T cells as compared to myeloid CD14+ or CD15+ cells, likely reflecting a higher contribution to the cfDNA from the short-lived myeloid cells (Figure 6G,I).

### Retrieval of IS from cfDNA of dogs treated by LV-mediated liver directed GT

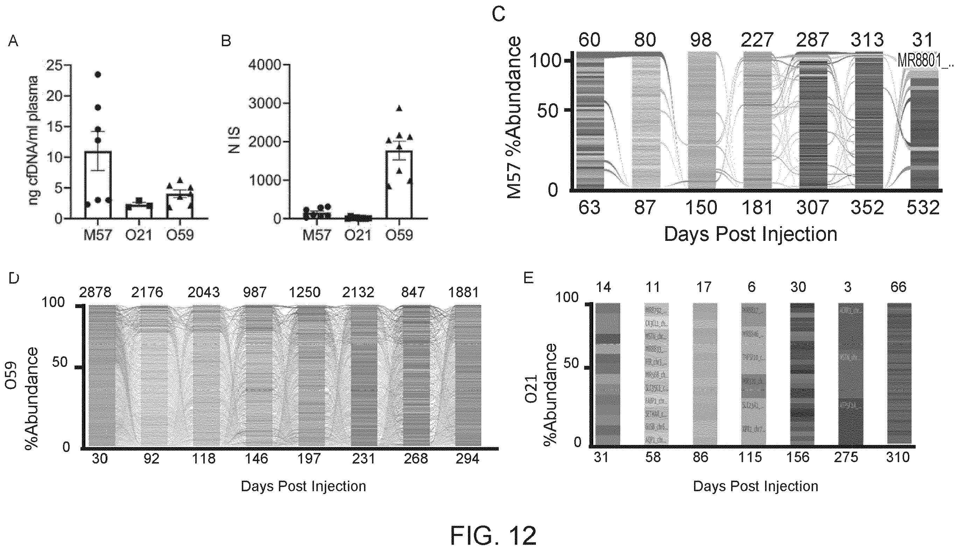

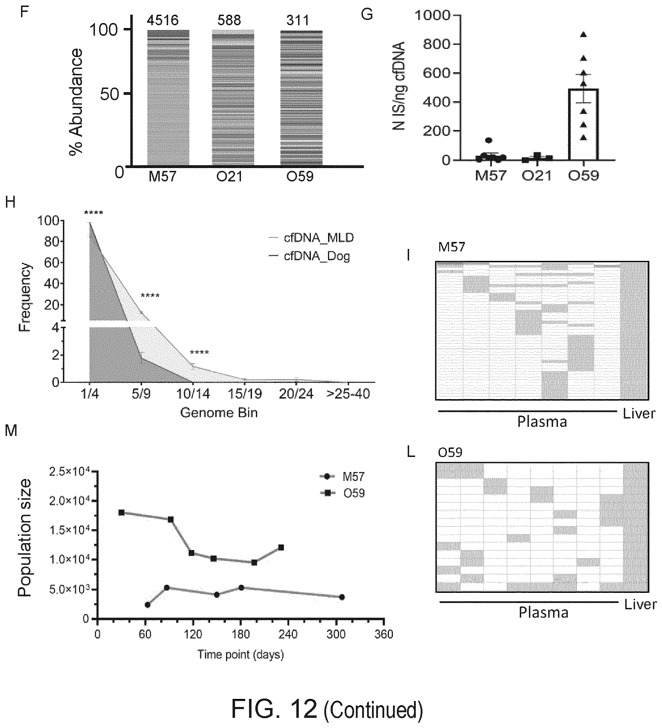

Having demonstrated that LiBIS-seq allows retrieving IS from cfDNA from HSC-GT patients, the inventors verified the capability of this technique to reveal IS of vector-marked cell clones embedded in solid organs. To this purpose the inventors analysed blood serum and liver tissue samples from a preclinical study on dogs subjected to LV-based liver-directed GT for the treatment of haemophilia B. In the study, three adult haemophilic dogs (O21, M57 and O59) received a single portal vein injection of low doses of LVs expressing different versions of canine coagulation factor IX (cFIX). For each dog, cfDNA was purified from blood serum harvested at 7-8 different time points starting from 30 to 532 days post injection, while liver DNA was purified from tissue harvested at necropsy at 3107 days (M57), 2185 days (O21) and 1848 days (O59) from LV administration. The amount of cfDNA obtained at each time point was highly variable and generally low (from 2 ng to a maximum of 23 ng) (Figure 12A). Thus, all available cfDNA was used for the LiBIS-seq amplification protocol. Liver genomic DNA was extracted from a mix of liver tissue sampled from multiple lobes and a total of ~1 µg of DNA was used for IS retrieval. After PCR amplification and sequencing, the ISs were mapped on the dog genome and the nearest gene identified by datamining using available human orthologue gene annotations on the dog genomic coordinates (UCSC table xenoRefGene). We retrieved: 154 IS from cfDNA and 588 IS from liver DNA of dog O21, 1057 IS from cfDNA and 4516 IS from liver DNA of dog M57, 9796 IS from cfDNA and 311 IS from liver DNA of dog O59 (Figure 12B-F). The number of insertions retrieved from liver and/or cfDNA indicate that all animals had a polyclonal repertoire of genetically modified cells. The number of cfDNA-derived IS retrieved at different time points from the same dog were similar and not correlated to the amount of cfDNA analysed (Figure 12G). Differently from what observed in ex-vivo HSC GT patients, the IS identified in the dog liver DNA or serum cfDNA were significantly less abundant, being mostly represented by 1 to 4 genomes (98.2%± 0.4) and reaching a maximum of 20 genomes (Figure 12H, Figure 10A, B), consistently with the expected low proliferation rate in the adult liver. No dominant clones expanding and persisting over time were observed in any dog (Figure 12C-E). The most targeted genes were NDGR4 and SMIM21 in cfDNA, while no significant enrichment for any targeted gene was found in the liver IS dataset. Overrepresentation analysis of the most targeted gene classes showed a significant tendency to target gene classes involved in ion channel, phosphatase and RHO exchange factor activity (Figure 10C). In the two dogs with the higher number of cfDNA-derived IS, 10.8% and 23.3% respectively, of IS were shared between at least two time points and 5% and 0.2% respectively, with IS from the sampled liver tissue (Figure 12I, L). By applying capture and recapture statistics on shared IS present in consecutive time points and across the whole follow-up time, we estimated the average population size of marked cell clones which contributed IS during time. From this analysis we estimated in dog M57 a persisting population of 4183±547 cells, which was stably maintained across the entire follow-up time (from 60 to 532 days) and in dog O59 a population of 12963±1466 cells at early time points (from 30 to 294 days) which decreased to 10726±558 cells in the subsequent time (from 120 to 240 days) (Figure 12M).

### LiBIS-seq allows early detection of aberrant clonal expansions in solid tissues

The inventors further investigated if LiBIS-seq allows the detection and quantification of premalignant or malignant clonal expansions triggered by insertional mutagenesis and if, compared to the conventional methods based on the interrogation of the cell-derived IS, it enables the earlier detection of aberrantly expanding clones residing in solid tissues.

For this purpose, the inventors analysed PBMCs and plasma samples collected from patients who developed leukaemia or lymphoma as a result of γRV insertional mutagenesis in two trials of Wiskott-Aldrich Syndrome (WAS) and X-linked Severe Combined Immunodeficiency (X-SCID) HSC GT. The inventors also analysed an experimental model of HSC transplantation in X-SCID mice, where 50% of transplanted mice spontaneously develop thymic lymphomas.

WAS patients P5 and P7 were diagnosed with T- Acute Lymphocytic Leukaemia (T-ALL) 36 and 37 months respectively, after HSC GT. Previous IS analyses carried by Linear Amplification Mediated (LAM)-PCR and other techniques have shown that for P7 the leukaemic clone was marked by two insertions targeting LMO2 and TAL1 (Figure 13A), while for P5 the leukemic clone was marked by nine insertions targeting LMO2, TAL1, C11orf74, TMEM217, UBB, ST8SIA6, CPSF6, CD46 and RIN3 (Figure 11A). For WAS P7, the inventors analysed genomic DNA of PBMCs and cfDNA collected at 7 and 3 months before and at the time of diagnosis of leukaemia. The inventors retrieved 24650 IS from PBMCs and 2885 IS from cfDNA. The γRV insertions of the leukaemic clone were identified in PBMCs and cfDNA with a low level of abundance (<2%) before the leukaemia onset, while their abundance reached 85% and 75% respectively at the time of diagnosis (Figure 13B, C). For WAS P5 the inventors analysed the PBMC and plasma collected at 10 months before and at the time of the leukaemia diagnosis and an additional plasma sample collected 6 months before leukaemia onset. From the PBMCs of this patient the inventors retrieved >13000 IS, while the cfDNA yielded >20000 IS (Figure 11B, C). The IS of the malignant clone were detected in PBMC and cfDNA samples. At 10 months before leukaemia onset the leukaemic clone showed low levels of abundance (0.45% in PBMCs and 0.38% in cfDNA), while at the time of leukaemia diagnosis the relative abundance of the malignant clone reached 85% in PBMCs (in agreement with previous reports) and -50% in cfDNA (Figure 11B, C). Moreover, the inventors observed that in both WAS patients the amounts of cfDNA per ml of plasma progressively increased reaching a maximum of 1200 ng/ml in P5 and 150 ng/ml in P7 at the time of leukaemia diagnosis (Figure 13D). Interestingly, the increased amounts of cfDNA was paralleled by a decrease in the number of IS retrieved for ng of cfDNA suggesting a reduction of the clonal complexity of the population likely due to the outgrowth of the malignant clone (Figure 13E).

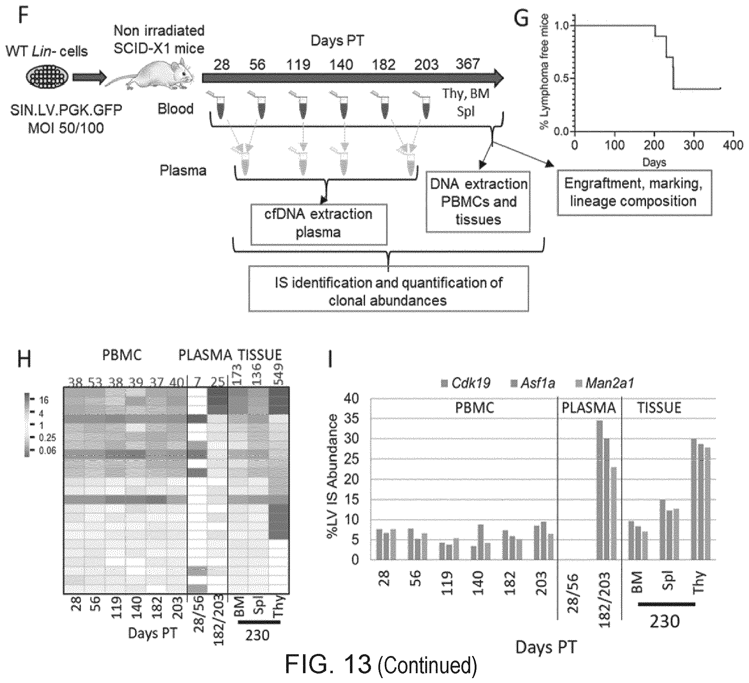

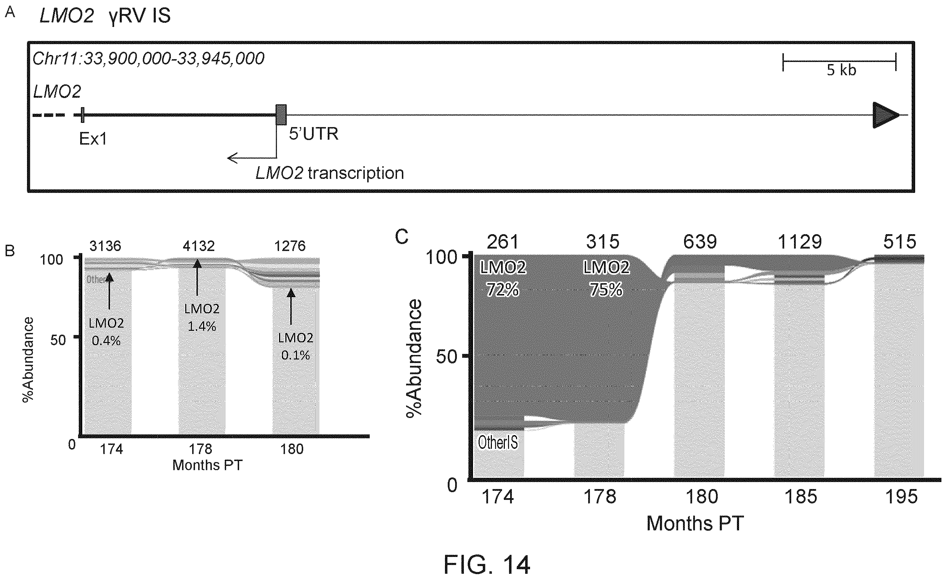

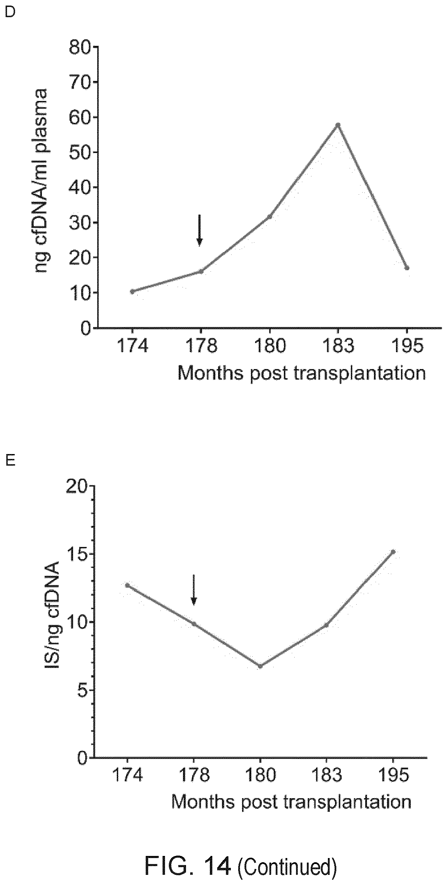

To further investigate the potential of LiBIS-seq to detect clones expanding in solid tissues, the inventors took advantage of an HSC transplantation model into X-SCID mice, where non conditioned mice infused with wild type (WT) BM-derived linage negative cells (lin-) develop T cell lymphoblastic lymphoma starting from 20 weeks after transplant and with an incidence of 50%. In this model, T cell progenitors spontaneously develop mutations likely due to the replication stress they are subjected in the empty thymic niche; tumours develop within the thymus and, at later time points, may infiltrate liver, bone marrow, spleen and peripheral blood. Therefore, the inventors investigated if the longitudinal monitoring of IS in plasma cfDNA of X-SCID mice transplanted with vector-marked Lin- cells would allow the earlier detection of lymphomatous expansions compared to conventional cell-derived IS studies based on PBMCs. A total of 10 X-SCID mice were transplanted with 400000 cluster of differentiation 45.1+ (CD45.1) BM-derived lin- cells transduced with a SIN LV expressing GFP under the control of the PGK promoter in internal position (Figure 13F). The insertions of this non-genotoxic LV are not involved in cell transformation and serve as surrogate marker of clonal identity and abundance. After transplantation, mice were monitored for their health status and euthanised if showing signs of disease or at 367 days, the end of the experiment. In agreement with previous results, six mice developed T-cell lymphoma starting from 200 to 250 days after transplant (Figure 13G). Phenotypic analyses showed engraftment levels of CD45.1 cells that ranged from 20 to 40%, associated with generally high gene marking levels (measured as 80-90% GFP+ cells) and virtually all represented by T-cells. IS analyses were performed on DNA isolated from PBMCs and cfDNA harvested at different time points after transplantation, and from thymus, BM and spleen harvested at euthanasia from 5 mice, 3 of which developed T-cell lymphoma. From the IS analyses, we observed that in the thymus of three mice that developed T cell lymphoma (mouse ID: A3, A4 and B1) from 2 to 6 IS had higher abundance levels compared to other IS, while in mice that did not develope T cell lymphoma the abundance of IS was much more homogeneously distributed. In the 3 mice that developed T cell lymphoma the ISs with the highest level of abundance in the thymus were also identified as the most abundant ISs in cfDNA collected at the last time points at 183/203 days, 27-45 days before symptoms became evident and led to euthanasia (Figure 13H, I). On the other hand, IS analyses on PBMC mostly failed to reveal such aberrant expansions, proving that LiBIS-seq can uniquely detect clones expanding in solid tissues. The inventors further investigated a X-SCID patient (P9) who was diagnosed with a late-onset T cell lymphoma 178 months after HSC-GT, when subcutaneous nodules and a mediastinal mass were detected. IS retrieved from leukaemic blast cells in the lymph nodes and PBMCs at the time of diagnosis had previously shown that the malignant cell clone was marked by a single γRV insertion 30 kb upstream of LMO2 (Figure 14A) reaching a relative abundance of 96% in the lymph nodes and 4% in PBMC. The inventors extracted the cellular- and cf-DNA from PBMCs and plasma samples collected 4 months before the diagnosis of T cell lymphoma, at the time of diagnosis and 2 months post chemotherapy (180 months after transplant). Two unmatched plasma samples harvested at 185 and 195 months after therapy were also analysed. IS from PBMCs were retrieved and mapped on the human genome leading to 3136, 4132 and 1276 IS for each time point (Figure 14B, C). The γRV IS targeting LMO2 of the malignant clone described previously was also identified by the inventors' IS retrieval method in all analysed time points. In agreement with previous independent measurements, the level of abundance of the malignant clone in PBMC was always low with the highest peak of 1.4% at the time of diagnosis (178 months). On the other hand, IS retrieved by LiBIS-seq on plasma cfDNA were a minimum of 261 to a maximum of 1129 for all analysed time points. The malignant clone γRV IS was retrieved from cfDNA with relative abundance of 72% and 75% 4 months before and at the time of diagnosis, respectively, and became undetectable after chemotherapy (Figure 14B, C). The increased amounts of cfDNA that was observed before the leukaemia diagnosis corresponded to a decrease in the amount of IS retrieved for ng of cfDNA, likely due to the outgrowth of the malignant clone, and recovered after chemotherapy (Figure 14D, E). Similarly, the amount of cfDNA decreased to almost normal levels at the last time point available and corresponding to 17 months post diagnosis.

### DISCUSSION

The inventors have shown that that blood plasma-derived cfDNA harvested from HSC GT patients and from preclinical models of ex-vivo and in vivo liver-directed GT with LVs or γRVs can be effectively exploited to retrieve vector genomic IS, a genetic marker specific for each transduced cell and its progeny which is commonly used for clonal monitoring and safety evaluations of GT. Because in the LiBIS-seq technology the naturally sheared cfDNA fragments containing vector-cellular genome junctions are molecularly tagged before PCR amplification, the method ensures sensitive and quantitative retrieval of IS from cfDNA avoiding biases consequent to exponential amplification.

The advantage foreseen using LiBIS-seq is that, compared to other approaches based on genomic DNA obtained from circulating cells or small tissue biopsies, the cfDNA produced by dying cells residing in different tissues provide insights into vector-marked clones residing in solid organs. By this approach one can expand the repertoire of transduced cells interrogated upon ex-vivo HSC GT and perform longitudinal clonal tracking studies also upon in vivo GT applications, avoiding the need for serial invasive biopsies and obtaining a more comprehensive picture of the transduced organ. Indeed, LiBIS-seq strategy applied on cfDNA from 7 HSC GT MLD patients allowed the retrieval of several thousand vector IS from a single ml of blood plasma, numbers comparable or even superior to those retrieved by LAM-PCR performed on DNA samples from whole or lineage sorted blood cell populations. The genomic integration preferences and clonal abundance analyses of the cfDNA-derived IS recapitulated the high polyclonality and positive safety profile of cells genetically modified by LV observed in this and previous studies using DNA from circulating blood cells. About 30% of the cfDNA-derived IS were shared with those identified in DNA from blood cells of MLD patients, indicating that the inventors' PCR approach captured at least in part the same IS obtained with other methods, including LAM PCR and sonication-based techniques for IS retrieval. Whereas the remaining IS might not have been previously captured in blood cells because of sparse sampling, the inventors also hypothesize that a fraction of them could derive from vector marked cell clones embedded in extravascular sites and solid organs. This hypothesis is supported by the successful tracking of LV IS in serum-derived cfDNA from three dogs treated by liver-directed GT. From each dog, despite the low vector marking level reported from the sampled liver tissue, we were able to retrieve a number of IS ranging from 147 to 9,730 IS in 7-8 time points spread across 500 days of follow-up. The different overall amount of IS retrieved from each dog might be related to different levels of original tissue transduction, since the average number of IS retrieved across time points and within the same animal were comparable and did not correlate with the amount of cfDNA produced by each dog, the amount of cfDNA used for PCR amplification nor with the level of IS collected from the liver tissue sampling. Compared to IS collected from cfDNA of MLD HSC-GT patients, each cfDNA-derived IS from dogs was represented by a much lower number of genomes, thus reflecting the original transduction of a high number of liver resident cells that turn over slowly in adult individuals as compared to haematopoietic cells. Intriguingly, a fraction of cfDNA IS (-10-20% total IS) were repeatedly captured across different time points, indicating the existence of a long lived and slowly turning over cell population ranging from 5,000 to 10,000 vector-marked cell clones. Based on previous LV distribution studies, it is expected that hepatocytes, liver sinusoidal endothelial and Kupffer cells are all potential contributors to cfDNA IS, since these cell types represent abundant cell populations in liver, are transduced by intravenously delivered vesicular stomatitis virus G-protein (VSV-G) pseudotyped LV, are long lived and endowed with some self-renewal capability under homeostatic and pathological conditions such as in case of liver damage where elevated proliferation rates are required to support the regeneration process. The inventors' molecular approach may help uncovering the dynamics of liver cells in GT treated individuals by providing a more comprehensive sampling of the transduced organ compared to IS analyses carried on solid biopsies which only represents a small and local portion of the organ.

LiBIS-seq can also detect the emergence and measure the relative abundance of malignant clones triggered by insertional mutagenesis. Indeed, the inventors showed that in cfDNA of two WAS patients who experienced such adverse events the clonal abundance of ISs specific for pre- and leukemic clones was in line with the estimates made on blood cells genomic DNA with multiple state of the art methods for quantitative or semi-quantitative IS retrieval including Linear Amplification Mediated (LAM)-PCR non-restrictive LAM-PCR and Targeted Enrichment Sequencing. Importantly, LiBIS-seq allowed more sensitive detection and quantification of marked malignant cells expanding in the thymus which were instead barely detectable by conventional IS analyses carried on circulating cells. For example, in patient P9 of the X-SCID GT clinical trial IS analysis on PBMCs showed that a pre-malignant clone carrying an LMO2 IS was below 2% abundance for 15 years, and even at the time of diagnosis the lymphomatous clone was barely circulating and representing only 4% of the gene corrected T cells. Instead, the quantification of the malignant clone in cfDNA at four months before diagnosis was already showing a very high level (72%) compared to that measured in the DNA from PBMCs (0.4%). This discrepancy can be explained by the biology of the patient's disease, a lymphoma localised in the thymus which did not circulate. By LiBIS-seq the inventors could also appreciate a strong reduction in the malignant clone IS after chemotherapy, indicating that the analysis of cfDNA faithfully reflected the therapeutic response to treatment. Using this approach, the inventors were also able to predict lymphoma occurrence in a mouse model of X-SCID GT. Hence, LiBIS-seq increases the predictive potential of IS studies performed on circulating blood cells that, in some cases, were not able to predict the occurrence of malignant proliferations which eventually appeared abruptly but likely took years to develop. However, it is important to note that the identification of dominant ISs, even when maintained over time, does not represent per se clinical evidence of cancer, nor justify therapeutic intervention. Nonetheless, the early detection of these potentially dangerous clones may positively impact on clinical practice by prompting for more in depth clinical investigation aimed at early detection of malignant cells or tumour masses and sensitive monitoring of the efficacy of treatment or relapse. Since in cancer diagnostics, sequencing of cfDNA has allowed to identify DNA risk variants originating from pre- or malignant cells residing in diverse solid tissues including liver, gastrointestinal tract, breast, lung, LiBIS-seq could potentially reveal aberrant clonal expansions occurring in organs other than the thymus.

Beside the retrieval of IS, the inventors found that in many MLD patients the amount of cfDNA per ml of blood plasma was above the pathological levels of 10 ng/ml before HSC-GT and within the early phases after transplant, while it significantly decreased after stable haematopoietic repopulation. The only exception was MLD04 for whom the GT procedure was not effective/curative and was constantly characterised by pathological levels of cfDNA concentrations. Elevated cfDNA levels have been described in cancer, aging and infection and several other disease conditions associated to inflammation. It is possible that the high levels of cfDNA observed before transplantation were due to the emerging neurodegenerative lysosomal storage disorder which can impact on cellular pathways associated with the control of cell death and proliferation. After HSC-GT, the residual effects of the myeloablative treatment and the fast turnover of progenitor cells that is occurring in the early phases of haematopoietic reconstitution could contribute to the increase cfDNA levels. The progressive reduction in cfDNA concentration at later time points may instead reflect the positive therapeutic outcome achieved in the different tissues by HSC-GT. Hence, these preliminary data suggest that the levels of cfDNA could be used as surrogate biomarker of the efficacy of the GT treatment in this lysosomal storage disorder, as it reflects the amount of dying cells at a given time as result of the accumulation of toxic lysosomal compounds. The amount of cfDNA in blood plasma were above the pathological levels also in the WAS and X-SCID patients that developed leukaemia and lymphoma respectively, and decreased after chemotherapy in the X-SCID patient mirroring what has been already described in cancer patients. It is important to note that in all these cases, the amount of cfDNA did not impact on the number of retrieved IS nor on the level of clonal abundance. Indeed, a reduction in the amount of IS retrieved for ng of cfDNA was observed with the outgrowth of the malignant clone and only the ISs of the expanding cell clones were represented by higher number of genomes (range: 250-2,000) compared to all other "neutral" insertions events.

In conclusion, the analysis of cfDNA by LiBIS-seq enables the study of the clonal repertoire, turnover and persistence of thousands of clones - each characterised by a specific IS - in animal models and patients subjected to in vivo or ex vivo GT overcoming the limitations of IS analysis performed on solid biopsies. Beyond the safety aspects, the chance to simultaneously track and quantify in cfDNA hundreds to thousands of clones residing in body organs from early to late phases after gene therapy enables exploration of basic biological properties of tissue turnover during regeneration, homeostasis and different therapy outcomes.

### MATERIALS AND METHODS

### Study design and patients

This study was performed using samples (PBMC and plasma) obtained from 7 patients diagnosed for MLD and that underwent HSC-GT by LV (Biffi, A. et al. (2013) Science 1233158; Sessa, M. et al. (2016) Lancet 388: 476-487). The LV-based HSC-GT trial for MLD is registered (NCT01560182) and approved by Istituto Superiore di Sanità, the Ethical Committee of the San Raffaele and by Agenzia Italiana del Farmaco. The medicinal product received Orphan Drug Designation (ODD) (EMEA/OD/102/06) by the European Medicines Agency (EMA) for the treatment of MLD.

PBMC and plasma samples were obtained from 2 patients diagnosed for WAS and that underwent γRV-based HSC-GT (Braun, C. J. et al. (2014) Sci Transl Med 6: 227ra233; Braun, C. J. et al. (2014) Rare Dis 2: e947749). The trial is registered in the German Clinical Trials Register (DRKS00000330), and the clinical GT protocol was reviewed and approved by the Ethics Committees of Hannover Medical School and Ludwig Maximilian University Munich.

PBMC and plasma samples were obtained from patient P9 enrolled in the LTR-driven γRV-based SCID-X1 gene therapy trial conducted between 1999-2002 at the French Hospital Necker-Enfants Malades, Paris. γc gene therapy trial at Hôpital Necker-Enfants Malades, Paris (Hacein-Bey-Abina, S. et al. (2010) N Engl J Med 363: 355-364). This P9 patient was previously reported as P8 in our publications (Hacein-Bey-Abina, S. et al. NEJM (2010) and Wang, G.P. et al. Blood (2010)). The protocol was registered under the local reference P971001, approved by the French Competent Authority (AFSSAPS) and the local Ethics Committee (Comité de protection des personnes of Hôpital Cochin,Paris, France).

For each patient, written informed consent was obtained from the parents or guardians of the patients.

### Dog experiments

Hemophilia B dogs were maintained at the Francis Owen Blood Research Laboratory, which provides for breeding, whelping, housing, treating and performing the experiments in the dogs on site. All animal procedures were performed according to specific protocols as described (Cantore, A. et al. (2015) Sci Transl Med 7: 277ra228).

### Purification of cfDNA from BP

Depending on the starting amount of plasma or serum available, different kit for circulating DNA purification have been adopted: when 2 to 5 ml of plasma or serum are available, QIAamp Circulating Nucleic Acid Kit is adopted (55114, QIAGEN), when less than 2 ml of plasma or serum were available Maxwell RSC ccfDNA Plasma kit (AS1480, Promega) is used. In both cases, purification is performed following manufacturer's instructions. After purification, cell-free DNA is immediately quantified and subjected to LiBIS-seq procedure.

### LiBIS-seq: Retrieval of circulating IS from cell-free DNA

After purification cell-free DNA was split in 2 technical replicates, subjected to end repair and 3' adenylation using the NEBNext^{®} Ultra^{™} DNA Library Prep Kit for Illumina^{®} (New England Biolabs, Ipswich, MA.), and then ligated (DNA Technologies ligation kit, Skokie, IL.) to linker cassettes (LC) containing: i) 8 nucleotide sequence barcode, used for sample identification, ii) 12 random nucleotide sequence, used for quantification purposes and iii) all the sequences required for the Read 2 Illumina paired end sequencing, tracked within our laboratory information management. Ligation products were then subjected to 35 cycles of exponential PCR with primers complementary to LV LTR or RV LTR and the LC, next ten additional PCR cycles were done to add sequences required for sequencing and a second 8 nucleotides DNA barcode. Finally, the amplification products were sequenced using the Illumina Myseq/HiSeq platform (Illumina, San Diego, CA.).

### Retrieval of IS from cell DNA

For the retrieval of vector IS from genomic DNA, we adopted a sonication-based linker-mediated (LM) PCR method previously described (Firouzi, S. et al. (2014) Genome Med 6: 46; Gillet, N. A. et al. (20110 Blood 117: 3113-3122). Briefly, genomic DNA was sheared using a Covaris E220 Ultrasonicator (Covaris Inc., Woburn (MA), generating fragments with an average size of 1000bp. The fragmented DNA was then split in 3 technical replicates and subjected to end repair and 3' adenylation using the NEBNext^{®} Ultra^{™} DNA Library Prep Kit for Illumina^{®} (New England Biolabs, Ipswich, MA.), and then ligated (DNA Technologies ligation kit, Skokie, IL.) to the same linker cassettes (LC) described before. The PCR procedure applied for IS retrieval and mapping is equal to what has been described above.

### Bioinformatics analysis

Sequencing reads were then processed by a dedicated bioinformatics pipeline (VISPA2) as previously described (Spinozzi, G. et al. (2017) BMC Bioinformatics 18: 520). Briefly, paired sequence reads are filtered for quality standards, barcodes identified for sample demultiplexing of the sequence reads, the cellular genomic sequence mapped on the reference Human genome (Human Genome_GRCh37/hg19 Feb. 2019), dog (Broad CanFam3.1/canFam3) or mouse (Mouse Genome_mm9) and the nearest RefSeq gene assigned to each unambiguously mapped integration site. For the quantification of the abundance of each clone we adopted an estimation method previously described (Firouzi, S. et al. (2014) Genome Med 6: 46; Gillet, N. A. et al. (2011) Blood 117: 3113-3122; Berry, C. C. et al. (2012) Bioinformatics 28: 755-762), where the abundance is determined by the number of different DNA fragments containing the same vector/cell genome junctions flanked by a genomic segment variable in size depending on the shear site position and that will be unique for each different cell genome present in the starting cell population. Therefore, the number of different shear sites assigned to an IS will be proportional to the initial number of contributing cells, allowing estimation of the clonal abundance in the starting sample avoiding the biases introduced by PCR amplification.

Further details are provided below.

### Molecular characterisation of PCR products obtained by LiBIS-seq

As described above, PCR products obtained by LiBIS-seq were sequenced using an Illumina platform. Sequencing reads were then processed by VISPA2 (Spinozzi, G., et al. (2017) BMC Bioinformatics 18: 520), a dedicated bioinformatics pipeline developed to correctly identify IS from a sequencing library of PCR fragments containing vector genome junctions. VISPA2 isolates the genomic sequences flanking the vector LTR sequences and maps them on the reference genome, retaining only alignments with a 98% match score. To quantify chimeric PCR artefacts within our sequencing libraries of IS fragments, we analysed the sequences discarded by VISPA2 accounting for the overall level of chimeric fragments whose ends mapped on distinct chromosomes, were not-properly paired (wrong orientation) or mapped too far away one from the other (>1200 bp). By this analysis, we found that the average number of chimeric artefacts is 1.28% in cell-derived datasets and 1.56% in cfDNA datasets (See Table 4).

**Table 4. Detailed table of mapped sequencing paired-end reads and chimeric fragment abundance. Each row of the table contains details of a different PCR sample related to a clinical trial (column name "Project"), collected in a source (column "Source", Plasma or Cells), with a custom identifier (column "ID"), and shows the amount of LTR identified and mapped PCR fragments (column "Mapped fragments") and the amount of "Chimeric Fragments" as number or reads and percentage.**

| **Project** | **Source** | **ID** | **Mapped fragments** | **Chimeric Fragments** | **Chimeric Fragments (%)** |
|---|---|---|---|---|---|
| **MLD** | Plasma | LTR11.LC76 | 6,364,048 | 90,260 | 1.42 |
| **MLD** | Plasma | LTR13.LC80 | 2,930,967 | 44,952 | 1.53 |
| **MLD** | Plasma | LTR17.LC46 | 4,876,642 | 14,383 | 0.29 |
| **MLD** | Plasma | LTR17.LC84 | 5,591,547 | 17,180 | 0.31 |
| **MLD** | Plasma | LTR1.LC56 | 6,976,501 | 27,226 | 0.39 |
| **MLD** | Plasma | LTR1.LC6 | 10,050,422 | 219,443 | 2.18 |
| **MLD** | Plasma | LTR21.LC92 | 6,300,218 | 33,012 | 0.52 |
| **MLD** | Plasma | LTR25.LC6 | 5,578,892 | 72,067 | 1.29 |
| **MLD** | Plasma | LTR27.LC10 | 1,699,243 | 26,902 | 1.58 |
| **MLD** | Plasma | LTR27.LC34 | 2,332,228 | 17,736 | 0.76 |
| **MLD** | Plasma | LTR27.LC62 | 7,917,059 | 22,773 | 0.29 |
| **MLD** | Plasma | LTR29.LC14 | 14,472,983 | 240,589 | 1.66 |
| **MLD** | Plasma | LTR29.LC66 | 11,504,647 | 97,109 | 0.84 |
| **MLD** | Plasma | LTR31.LC18 | 5,176,543 | 37,361 | 0.72 |
| **MLD** | Plasma | LTR33.LC22 | 8,635,245 | 91,149 | 1.06 |
| **MLD** | Plasma | LTR33.LC46 | 1,966,649 | 24,474 | 1.24 |
| **MLD** | Plasma | LTR39.LC78 | 8,469,616 | 96,168 | 1.14 |
| **MLD** | Plasma | LTR3.LC14 | 12,818,384 | 87,953 | 0.69 |
| **MLD** | Plasma | LTR3.LC60 | 7,684,412 | 195,246 | 2.54 |
| **MLD** | Plasma | LTR41.LC62 | 10,957,928 | 119,846 | 1.09 |
| **MLD** | Plasma | LTR43.LC86 | 2,521,241 | 13,983 | 0.55 |
| **MLD** | Plasma | LTR45.LC70 | 3,133,976 | 169,920 | 5.42 |
| **MLD** | Plasma | LTR47.LC94 | 2,857,555 | 46,680 | 1.63 |
| **MLD** | Plasma | LTR51.LC78 | 2,638,890 | 47,118 | 1.79 |
| **MLD** | Plasma | LTR53.LC82 | 3,020,297 | 6,514 | 0.22 |
| **MLD** | Plasma | LTR57.LC90 | 7,499,869 | 44,594 | 0.59 |
| **MLD** | Plasma | LTR59.LC94 | 8,185,773 | 33,395 | 0.41 |
| **MLD** | Plasma | LTR61.LC2 | 7,714,635 | 30,505 | 0.40 |
| **MLD** | Plasma | LTR65.LC4 | 9,847,900 | 37,632 | 0.38 |
| **MLD** | Plasma | LTR67.LC8 | 10,467,346 | 77,368 | 0.74 |
| **MLD** | Plasma | LTR69.LC12 | 6,525,325 | 114,866 | 1.76 |
| **MLD** | Plasma | LTR69.LC34 | 9,577,606 | 104,676 | 1.09 |
| **MLD** | Plasma | LTR71.LC16 | 8,464,083 | 136,156 | 1.61 |
| **MLD** | Plasma | LTR73.LC20 | 6,458,078 | 51,338 | 0.79 |
| **MLD** | Plasma | LTR75.LC24 | 2,639,646 | 26,990 | 1.02 |
| **MLD** | Plasma | LTR77.LC28 | 1,702,655 | 6,479 | 0.38 |
| **MLD** | Plasma | LTR77.LC6 | 9,317,948 | 106,847 | 1.15 |
| **MLD** | Plasma | LTR79.LC10 | 8,502,072 | 117,617 | 1.38 |
| **MLD** | Plasma | LTR7.LC30 | 13,846,910 | 288,007 | 2.08 |
| **MLD** | Plasma | LTR81.LC14 | 8,167,029 | 291,454 | 3.57 |
| **MLD** | Plasma | LTR85.LC22 | 10,894,649 | 226,572 | 2.08 |
| **MLD** | Plasma | LTR87.LC52 | 2,456,820 | 25,157 | 1.02 |
| **MLD** | Plasma | LTR89.LC30 | 7,798,474 | 109,770 | 1.41 |
| **MLD** | Plasma | LTR9.LC72 | 7,033,235 | 61,958 | 0.88 |
| **MLD** | Cells | LTR11.LC10 | 482,084 | 5,038 | 1.05 |
| **MLD** | Cells | LTR11.LC12 | 44,936 | 322 | 0.72 |
| **MLD** | Cells | LTR11.LC16 | 381,610 | 4,784 | 1.25 |
| **MLD** | Cells | LTR11.LC60 | 617,140 | 4,613 | 0.75 |
| **MLD** | Cells | LTR11.LC72 | 303,773 | 3,081 | 1.01 |
| **MLD** | Cells | LTR11.LC96 | 4,076 | 25 | 0.61 |
| **MLD** | Cells | LTR13.LC16 | 4,395 | 37 | 0.84 |
| **MLD** | Cells | LTR13.LC2 | 56,615 | 538 | 0.95 |
| **MLD** | Cells | LTR17.LC44 | 328,516 | 3,521 | 1.07 |
| **MLD** | Cells | LTR17.LC4 | 78,480 | 852 | 1.09 |
| **MLD** | Cells | LTR19.LC20 | 321,856 | 2,568 | 0.80 |
| **MLD** | Cells | LTR19.LC22 | 13,436 | 362 | 2.69 |
| **MLD** | Cells | LTR19.LC38 | 554,819 | 6,283 | 1.13 |
| **MLD** | Cells | LTR19.LC6 | 1,364,666 | 13,712 | 1.00 |
| **MLD** | Cells | LTR19.LC92 | 300,815 | 2,108 | 0.70 |
| **MLD** | Cells | LTR1.LC76 | 161,329 | 979 | 0.61 |
| **MLD** | Cells | LTR25.LC20 | 30,234 | 345 | 1.14 |
| **MLD** | Cells | LTR25.LC22 | 217,478 | 1,689 | 0.78 |
| **MLD** | Cells | LTR25.LC2 | 24,155 | 203 | 0.84 |
| **MLD** | Cells | LTR25.LC48 | 30,509 | 593 | 1.94 |
| **MLD** | Cells | LTR25.LC8 | 709,792 | 4,001 | 0.56 |
| **MLD** | Cells | LTR27.LC10 | 130,989 | 1,134 | 0.87 |
| **MLD** | Cells | LTR27.LC44 | 469,159 | 3,475 | 0.74 |
| **MLD** | Cells | LTR27.LC66 | 1,193,317 | 6,919 | 0.58 |
| **MLD** | Cells | LTR27.LC82 | 6,449 | 95 | 1.47 |
| **MLD** | Cells | LTR27.LC88 | 179,670 | 2,432 | 1.35 |
| **MLD** | Cells | LTR29.LC12 | 1,697,431 | 14,090 | 0.83 |
| **MLD** | Cells | LTR29.LC16 | 415,290 | 2,988 | 0.72 |
| **MLD** | Cells | LTR29.LC26 | 742,546 | 4,947 | 0.67 |
| **MLD** | Cells | LTR29.LC28 | 1,223,271 | 8,082 | 0.66 |
| **MLD** | Cells | LTR31.LC40 | 750,152 | 8,331 | 1.11 |
| **MLD** | Cells | LTR31.LC42 | 504,937 | 7,167 | 1.42 |
| **MLD** | Cells | LTR31.LC72 | 1,107,350 | 6,380 | 0.58 |
| **MLD** | Cells | LTR33.LC18 | 35,661 | 858 | 2.41 |
| **MLD** | Cells | LTR33.LC30 | 208,394 | 1,559 | 0.75 |
| **MLD** | Cells | LTR33.LC54 | 1,460,237 | 11,674 | 0.80 |
| **MLD** | Cells | LTR33.LC82 | 75,782 | 600 | 0.79 |
| **MLD** | Cells | LTR35.LC20 | 33,814 | 511 | 1.51 |
| **MLD** | Cells | LTR35.LC24 | 481,970 | 4,101 | 0.85 |
| **MLD** | Cells | LTR35.LC32 | 7,902 | 42 | 0.53 |
| **MLD** | Cells | LTR35.LC60 | 97,971,797 | 771,181 | 0.79 |
| **MLD** | Cells | LTR37.LC18 | 752,604 | 9,329 | 1.24 |
| **MLD** | Cells | LTR37.LC64 | 103,965 | 727 | 0.70 |
| **MLD** | Cells | LTR3.LC16 | 28,244 | 334 | 1.18 |
| **MLD** | Cells | LTR3.LC80 | 65,728 | 226 | 0.34 |
| **MLD** | Cells | LTR3.LC82 | 518,119 | 4,447 | 0.86 |
| **MLD** | Cells | LTR3.LC84 | 4,376 | 84 | 1.92 |
| **MLD** | Cells | LTR3.LC88 | 507,615 | 4,789 | 0.94 |
| **MLD** | Cells | LTR41.LC24 | 26,414 | 494 | 1.87 |
| **MLD** | Cells | LTR41.LC36 | 5,221 | 9 | 0.17 |
| **MLD** | Cells | LTR41.LC4 | 962,409 | 8,986 | 0.93 |
| **MLD** | Cells | LTR41.LC84 | 333,536 | 2,227 | 0.67 |
| **MLD** | Cells | LTR41.LC96 | 221,785 | 2,725 | 1.23 |
| **MLD** | Cells | LTR43.LC22 | 1,203,516 | 23,175 | 1.93 |
| **MLD** | Cells | LTR43.LC26 | 1,359,518 | 9,127 | 0.67 |
| **MLD** | Cells | LTR43.LC38 | 249,676 | 1,756 | 0.70 |
| **MLD** | Cells | LTR45.LC40 | 9,001 | 73 | 0.81 |
| **MLD** | Cells | LTR47.LC30 | 133,954 | 844 | 0.63 |
| **MLD** | Cells | LTR47.LC42 | 27,081 | 199 | 0.73 |
| **MLD** | Cells | LTR49.LC32 | 859,027 | 5,089 | 0.59 |
| **MLD** | Cells | LTR49.LC44 | 587,018 | 5,273 | 0.90 |
| **MLD** | Cells | LTR49.LC82 | 103,885 | 2,343 | 226 |
| **MLD** | Cells | LTR51.LC30 | 443,220 | 5,259 | 1.19 |
| **MLD** | Cells | LTR5 1.LC60 | 464,507 | 3,817 | 0.82 |
| **MLD** | Cells | LTR5 1.LC82 | 5,024 | 132 | 2.63 |
| **MLD** | Cells | LTR5 1.LC92 | 644,359 | 5,572 | 0.86 |
| **MLD** | Cells | LTR53.LC28 | 508,963 | 5,382 | 1.06 |
| **MLD** | Cells | LTR53.LC40 | 113,084 | 1,070 | 0.95 |
| **MLD** | Cells | LTR53.LC48 | 1,039,564 | 8,594 | 0.83 |
| **MLD** | Cells | LTR55.LC20 | 21,832 | 284 | 1.30 |
| **MLD** | Cells | LTR55.LC42 | 20,824 | 331 | 1.59 |
| **MLD** | Cells | LTR55.LC80 | 647,100 | 5,580 | 0.86 |
| **MLD** | Cells | LTR55.LC82 | 1,017,717 | 7,895 | 0.78 |
| **MLD** | Cells | LTR55.LC84 | 1,578,528 | 16,614 | 1.05 |
| **MLD** | Cells | LTR55.LC86 | 1,517,229 | 15,604 | 1.03 |
| **MLD** | Cells | LTR5.LC2 | 1,331,833 | 12,227 | 0.92 |
| **MLD** | Cells | LTR5.LC42 | 246,601 | 3,353 | 1.36 |
| **MLD** | Cells | LTR5.LC50 | 23,377 | 504 | 2.16 |
| **MLD** | Cells | LTR5.LC6 | 190,894 | 1,307 | 0.68 |
| **MLD** | Cells | LTR5.LC82 | 79,212 | 494 | 0.62 |
| **MLD** | Cells | LTR61.LC52 | 370,067 | 3,233 | 0.87 |
| **MLD** | Cells | LTR61.LC54 | 765,763 | 5,250 | 0.69 |
| **MLD** | Cells | LTR61.LC74 | 73,983 | 457 | 0.62 |
| **MLD** | Cells | LTR63.LC48 | 202,391 | 1,997 | 0.99 |
| **MLD** | Cells | LTR63.LC56 | 179,509 | 2,272 | 1.27 |
| **MLD** | Cells | LTR63.LC68 | 68,927 | 1,476 | 2.14 |
| **MLD** | Cells | LTR65.LC18 | 832,018 | 7,468 | 0.90 |
| **MLD** | Cells | LTR65.LC4 | 503,330 | 9,318 | 1.85 |
| **MLD** | Cells | LTR65.LC60 | 728,956 | 5,503 | 0.75 |
| **MLD** | Cells | LTR65.LC84 | 508,073 | 6,107 | 1.20 |
| **MLD** | Cells | LTR67.LC30 | 225,280 | 1,696 | 0.75 |
| **MLD** | Cells | LTR67.LC52 | 167,933 | 2,100 | 1.25 |
| **MLD** | Cells | LTR67.LC60 | 535,543 | 3,821 | 0.71 |
| **MLD** | Cells | LTR67.LC66 | 446,391 | 3,944 | 0.88 |
| **MLD** | Cells | LTR67.LC88 | 1,232,867 | 10,102 | 0.82 |
| **MLD** | Cells | LTR73.LC12 | 14,854 | 155 | 1.04 |
| **MLD** | Cells | LTR73.LC20 | 503,224 | 3,979 | 0.79 |
| **MLD** | Cells | LTR73.LC44 | 31,890 | 239 | 0.75 |
| **MLD** | Cells | LTR73.LC48 | 68,442 | 1,729 | 2.53 |
| **MLD** | Cells | LTR73.LC62 | 303,905 | 1,789 | 0.59 |
| **MLD** | Cells | LTR73.LC76 | 556,753 | 6,310 | 1.13 |
| **MLD** | Cells | LTR73.LC92 | 357,242 | 2,957 | 0.83 |
| **MLD** | Cells | LTR75.LC70 | 137 | 1 | 0.73 |
| **MLD** | Cells | LTR77.LC2 | 922,127 | 9,511 | 1.03 |
| **MLD** | Cells | LTR77.LC44 | 494,860 | 4,546 | 0.92 |
| **MLD** | Cells | LTR77.LC52 | 295,798 | 2,983 | 1.01 |
| **MLD** | Cells | LTR77.LC58 | 21,799 | 145 | 0.67 |
| **MLD** | Cells | LTR77.LC66 | 533,401 | 3,714 | 0.70 |
| **MLD** | Cells | LTR77.LC82 | 14,206 | 205 | 1.44 |
| **MLD** | Cells | LTR79.LC2 | 227,983 | 2,181 | 0.96 |
| **MLD** | Cells | LTR79.LC54 | 580,034 | 4,627 | 0.80 |
| **MLD** | Cells | LTR79.LC68 | 1,551,802 | 11,487 | 0.74 |
| **MLD** | Cells | LTR81.LC2 | 31,967 | 300 | 0.94 |
| **MLD** | Cells | LTR81.LC60 | 548,012 | 4,572 | 0.83 |
| **MLD** | Cells | LTR81.LC62 | 2,579 | 7 | 0.27 |
| **MLD** | Cells | LTR83.LC28 | 579,306 | 5,465 | 0.94 |
| **MLD** | Cells | LTR83.LC2 | 156,866 | 1,817 | 1.16 |
| **MLD** | Cells | LTR83.LC42 | 680,255 | 6,104 | 0.90 |
| **MLD** | Cells | LTR83.LC64 | 243,605 | 1,329 | 0.55 |
| **MLD** | Cells | LTR83.LC68 | 130,318 | 2,985 | 2.29 |
| **MLD** | Cells | LTR83.LC72 | 109,479 | 941 | 0.86 |
| **MLD** | Cells | LTR83.LC82 | 718,946 | 7,191 | 1.00 |
| **MLD** | Cells | LTR85.LC30 | 1,233,533 | 11,715 | 0.95 |
| **MLD** | Cells | LTR85.LC4 | 442,935 | 2,827 | 0.64 |
| **MLD** | Cells | LTR85.LC66 | 1,502,312 | 11,525 | 0.77 |
| **MLD** | Cells | LTR85.LC72 | 125,027 | 2,264 | 1.81 |
| **MLD** | Cells | LTR85.LC74 | 132,805 | 779 | 0.59 |
| **MLD** | Cells | LTR87.LC24 | 40,075 | 870 | 2.17 |
| **MLD** | Cells | LTR87.LC2 | 887,616 | 10,182 | 1.15 |
| **MLD** | Cells | LTR87.LC40 | 1,964 | 39 | 1.99 |
| **MLD** | Cells | LTR87.LC42 | 829,666 | 7,757 | 0.93 |
| **MLD** | Cells | LTR87.LC68 | 20,152 | 440 | 2.18 |
| **MLD** | Cells | LTR87.LC94 | 20,953 | 545 | 2.60 |
| **MLD** | Cells | LTR89.LC20 | 724,772 | 3,995 | 0.55 |
| **MLD** | Cells | LTR89.LC44 | 411,402 | 4,486 | 1.09 |
| **MLD** | Cells | LTR89.LC60 | 3,177,390 | 23,910 | 0.75 |
| **MLD** | Cells | LTR89.LC70 | 1,565,946 | 10,286 | 0.66 |
| **MLD** | Cells | LTR89.LC78 | 166,332 | 1,226 | 0.74 |
| **MLD** | Cells | LTR89.LC84 | 167,721 | 2,329 | 1.39 |
| **MLD** | Cells | LTR89.LC86 | 25,991 | 317 | 1.22 |
| **MLD** | Cells | LTR91.LC72 | 119,574 | 902 | 0.75 |
| **MLD** | Cells | LTR91.LC80 | 171,054 | 1,732 | 1.01 |
| **MLD** | Cells | LTR91.LC84 | 616,528 | 4,511 | 0.73 |
| **MLD** | Cells | LTR93.LC20 | 402,372 | 6,432 | 1.60 |
| **MLD** | Cells | LTR93.LC48 | 706,155 | 16,405 | 2.32 |
| **MLD** | Cells | LTR93.LC74 | 1,127,218 | 10,424 | 0.92 |
| **MLD** | Cells | LTR93.LC92 | 762,751 | 7,778 | 1.02 |
| **MLD** | Cells | LTR95.LC44 | 523,995 | 6,012 | 1.15 |
| **MLD** | Cells | LTR95.LC52 | 297,359 | 2,944 | 0.99 |
| **MLD** | Cells | LTR95.LC54 | 1,102,396 | 7,696 | 0.70 |
| **MLD** | Cells | LTR9.LC86 | 6,309 | 101 | 1.60 |
| **MLD** | Cells | LTR9.LC88 | 488,288 | 5,367 | 1.10 |
| **WAS** | Cells | LTR26.LC10 | 295,364 | 10,730 | 3.63 |
| **WAS** | Cells | LTR26.LC18 | 560,492 | 21,122 | 3.77 |
| **WAS** | Cells | LTR26.LC22 | 483,445 | 13,954 | 2.89 |
| **WAS** | Cells | LTR26.LC4 | 629,678 | 25,005 | 3.97 |
| **WAS** | Cells | LTR26.LC8 | 453,846 | 14,087 | 3.10 |
| **WAS** | Cells | LTR48.LC50 | 694,849 | 26,958 | 3.88 |
| **WAS** | Cells | LTR48.LC58 | 3,340,977 | 103,682 | 3.10 |
| **WAS** | Cells | LTR48.LC62 | 638,684 | 19,729 | 3.09 |
| **WAS** | Cells | LTR48.LC66 | 474,515 | 18,549 | 3.91 |
| **WAS** | Cells | LTR48.LC68 | 795,221 | 32,960 | 4.14 |
| **WAS** | Cells | LTR78.LC28 | 2,455,785 | 95,639 | 3.89 |
| **WAS** | Cells | LTR78.LC30 | 525,403 | 16,732 | 3.18 |
| **WAS** | Cells | LTR78.LC32 | 567,726 | 21,818 | 3.84 |
| **WAS** | Cells | LTR78.LC44 | 500,765 | 18,835 | 3.76 |
| **WAS** | Cells | LTR78.LC48 | 530,877 | 15,075 | 2.84 |
| **WAS** | Plasma | LTR48.LC62 | 10,663,796 | 391,030 | 3.67 |
| **WAS** | Plasma | LTR48.LC72 | 5,414,489 | 125,409 | 2.32 |
| **WAS** | Plasma | LTR48.LC94 | 6,306,825 | 109,662 | 1.74 |
| **WAS** | Plasma | LTR48.LC96 | 4,109,426 | 53,818 | 1.31 |
| **WAS** | Plasma | LTR78.LC28 | 7,624,704 | 300,013 | 3.93 |
| **WAS** | Plasma | LTR78.LC36 | 6,890,726 | 312,642 | 4.54 |
| **WAS** | Plasma | LTR78.LC58 | 7,363,206 | 250,473 | 3.40 |
| **WAS** | Plasma | LTR78.LC60 | 5,087,698 | 152,396 | 3.00 |
| **WAS** | Plasma | LTR78.LC90 | 4,118,764 | 114,899 | 2.79 |
| **WAS** | Plasma | LTR78.LC92 | 4,846,697 | 166,992 | 3.45 |

Although these chimeric PCR artefacts are completely removed by VISPA2 along the process of IS identification, it cannot be excluded that another set of PCR artefacts may escape from the stringent filters that were applied. However, the low percent of chimeric PCR artefacts observed in both types of IS dataset allow conclusion that PCR artefacts were not impacting in the analysis.

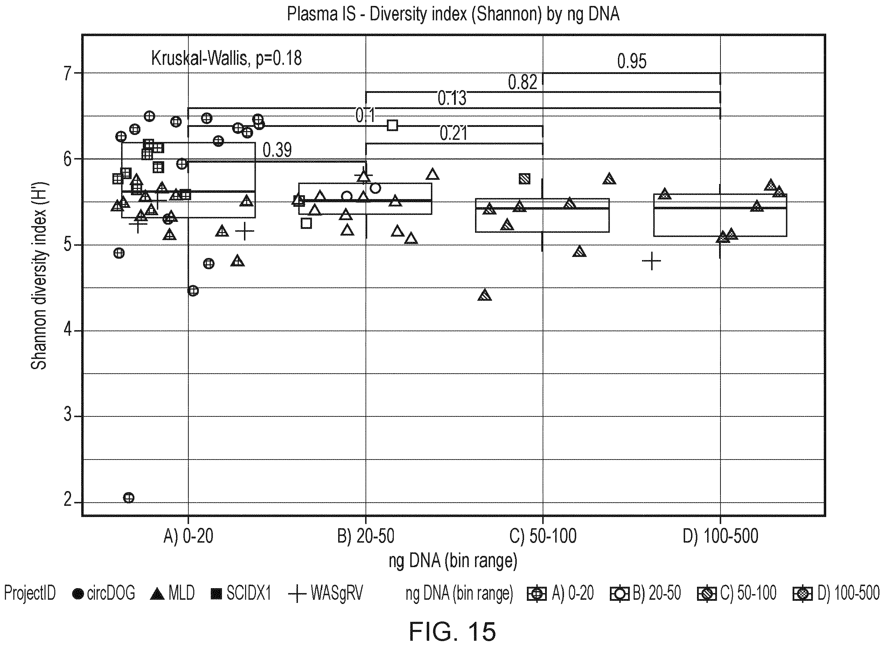

Furthermore, considering that the amount of cfDNA obtained from the different animal models and patients was highly variable, ranging from few ng/ml to >1ug/ml in patients that developed cancer, the inventors evaluated if the different amount of cfDNA used for IS retrieval could have impacted on the diversity of the distribution and abundance of the fragment size. Shannon Diversity Index (H-index) has been used as mathematical measure of the diversity of PCR-amplified fragments lengths, balancing the number of different fragments in each sample and their abundance (Haegeman, B. et al. (2013) ISME J 7 1092-101). Indeed, H-index has been calculated for each sample, and grouped by bins depending on amounts of cfDNA used for IS retrieval (0-20; 20-50; 50-100; 100-500 ng). No statistically significant difference has been observed among samples (among all samples we used non parametric test by Kruskal-Wallis; between each pair of samples the inventors used non parametric Mann-Withney), thus their results showed that the amount of cfDNA did not impact on the diversity of fragment sizes (Figure 15).

### Quantification abundance by SonicLength

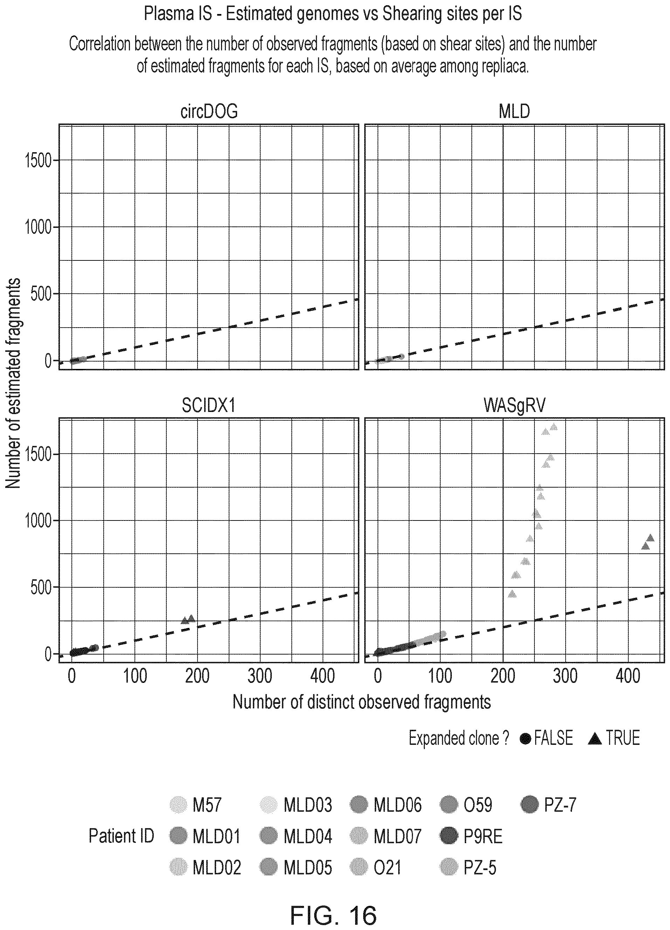

To estimate the abundance of each IS retrieved by genomic or cf-DNA, the inventors adopted the fragment estimate approach presented by Berry et al. (Berry, C.C. (2012) Bioinformatics 28: 755-62) and implemented in the R package "SonicLength". "SonicLength" implements a mathematical model that estimates the number of genomes/fragments per IS from the distribution of the observed fragment lengths. This method allows correction for shear site saturation, event that can highly impact on the quantification of highly abundant clones (such as the expanding clones) since in these cases multiple independent source DNA fragments may share the same shear site per IS. Based on this, "SonicLength" returns the estimate of the observed clones and this estimate could be much higher than the number of distinct observed shear sites. For this reason, the abundance of ISs from the malignant clones of WAS and X-SCID patients were represented by less than 300 observed fragments whose estimated abundance increased to >2000 fragments in some cases (Figure 16). In all other cases, the number of fragments observed for each IS was lower than 100 with a very similar number of estimated fragments, reflecting the different turnover rate of the parental cell clone.

The accuracy and detection limit of LiBIS-Seq compares well with the results obtained by our and others' state of the art methods for IS retrieval based on DNA extracted from blood cells. Indeed, for P5 and P7 of the WAS trial analysed in this study, the estimate abundances of IS-derived from pre- and leukaemic clones on cfDNA before and after diagnosis matched with the abundances calculated by our IS retrieval method based on genomic DNA purified from blood cells and with those obtained by others with multiple state of the art methods for quantitative or semi-quantitative IS retrieval including Linear Amplification Mediated (LAM) - PCR non-restrictive (nr) LAM-PCR and Targeted Enrichment Sequencing (Braun, C.J. et al. (2014) Science Translational Medicine 6: 227ra33). On the same line, for P9 of the X-SCID GT clinical trial, LiBIS-seq quantification of the IS harbouring the γRV insertion near LMO2 at the time before diagnosis was strikingly high compared to the other methods based on genomic DNA purified from blood cells. This discrepancy can be explained by the biology of the patient's disease, since in this case the lymphoma localised in the thymus and does not circulate, limiting its identification by conventional IS analyses on blood cells. However, we showed that its level of abundance in the blood at the time of diagnosis and quantified using our sonication-based PCR approaches on cell-derived DNA perfectly fit with the levels measured by others using real time PCR approach. Finally, LiBIS-seq allows detection of the strong reduction in abundance of the malignant clone after chemotherapy, indicating that our quantification methods reflect a therapeutic response to treatment.

### T cell lymphoblastic lymphoma in non-conditioned X-SCID mice transplanted with WT lin- cells

All animal procedures were performed according to protocols approved by the Animal Care and Use Committee of the San Raffaele Institute (IACUC 817) and communicated to the Ministry of Health and local authorities according to Italian law. All mice were bred and kept in a dedicated pathogen-free animal facility, and were euthanised when they showed signs of severe sickness. The in vivo model allowing the development of T cell lymphoblastic lymphoma in non-conditioned X-SCID mice transplanted with WT lin- cells is described in Schiroli, G. et al. (2017) Preclinical modeling highlights the therapeutic potential of hematopoietic stem cell gene editing for correction of SCID-X1 Sci Transl Med 9. Briefly, donor C57BL/6-Ly5.1 mice between 6 and 10 weeks of age were euthanised by CO₂, and BM cells were retrieved from femurs, tibias, and humeri. HSPCs were purified by Lin-selection using the mouse Lineage Cell Depletion Kit (Miltenyi Biotec) according to the manufacturer's instructions. Cells were then cultured in serum-free StemSpan medium (StemCell Technologies) containing penicillin, streptomycin, glutamine and a combination of mouse cytokines (20 ng/ml IL-3, 100 ng/ml SCF, 100 ng/ml Flt-3L, 50 ng/ml TPO all from Peprotech), at a concentration of 10⁶ cells/ml. Lin- cells were pre-stimulated for 2-3 hours and then infected with the SINLV.PGK.GFP at MOI 50/100 (Cesana, D. et al. (2014) Mol Ther 22: 774-785). 16 hours after infection cells were washed and 400000 CD45.1 BM-derived Lin- cells were injected into recipient non-conditioned X-SCID mice. After transplantation, mice were monitored for their health status and euthanised if showing signs of disease or at 367 days, the end of the experiments. PBMCs and BP were harvested at different time points after transplantation, while thymus BM and spleen tissues were harvested at euthanasia for phenotypic and clonal tracking analyses. FACS analysis was performed using lineage-specific antibodies against CD45.1 (clone A20 Biolegend), CD45.2 (clone 104 Biolegend), CD19 (clone 6D5 Biolegend), CD3e (clone 145-2C11 BD), CD4 (clone RM4-5 BD) and CD8a (clone 53-6.7 eBioscience) according to the manufacturer's instructions.

### Statistical Analysis

Statistical analyses were performed as indicated and using GraphPad Prism 8.0 software. Statistical significance for each CIS was established using the Grubbs test for outliers, as previously described (Aiuti, A. et al. (2013) Science 1233151; Biffi, A. et al. (2013) Science 1233158; Biffi, A. et al. (2011) Blood 117: 5332-5339). Gene Ontology enrichment analyses was performed using Great software (http://great.stanford.edu/public/html) f for MLD IS dataset and DAVID EASE software (https://david.ncifcrf.gov/) for dog IS dataset. Overall, we considered only significant Gene Ontology classes of the Cellular Component, Biological Process and Molecular Function System having a Fold enrichment score higher than 2. PRISM and R have been used as statistical software (package Vegan and R-capture for Mark-recapture models).

### Population size estimators

Population size estimators have been developed in ecology to solve the problem of estimating the number of species on a population in a specific environment given repeated sampling of animals without observing all different existing animals (Chao, A. et al. (2001) Stat Med 20: 3123-57; Chao, A. (1987) Biometrics 43: 783-91). In gene therapy, population size estimators, such as the Chao or Petersen-Schnabel models (Schnabel, Z.E. (1938) The American Mathematical Monthly 45: 348-352), have been widely used to estimate the number of active repopulating HSC as lower bound estimation of the population size. The cited models are based on capture-recapture methods, that are able to estimate the overall population size by exploiting repeated sampling (over time and/or in different places) of marked elements (animals or cells) and accounting for the number of shared elements among samplings.

Furthermore, to account for any potential bias in clonal size (heterogeneity) that could affect the probability of capturing elements, clonal dynamics and time variability, we used the Chao model for bias correction. All the estimates have been performed in R using the package Rcapture (http://cran.r-project.org/web/packages/Rcapture).

### EXAMPLE 3

AAV vectors are able to efficiently introduce therapeutic transgenes into millions of somatic cells directly within patients' organs and for this reason have been successfully exploited in clinical Gene Therapy (GT) to treat coagulation disorders, inherited blindness and neurodegenerative diseases. AAV genomes can remain in an episomal state within the nucleus of transduced cells for long term and undergo to intra- and intermolecular recombination events resulting in the generation of monomeric circles or concatenated molecules with different structures or even integrate within the host cell genome, albeit at low frequency. Several preclinical and clinical studies aimed to address the safety of AAV integrations have shown a good safety profile. Despite the positive safety results, the finding that AAV is able to integrate in the host cell genome poses safety concerns, since a single intravenous systemic AAV administrations may easily result in the transduction of hundreds of millions liver cells, thus even if the integration frequency is low, a relevant number of cells will harbour AAV insertions. These insertions may be genotoxic as shown in several preclinical safety studies, where animals treated with high AAV doses developed hepatocellular carcinoma as the result of insertional mutagenesis, which was also dependent backbone design and the sensitivity of disease model.

Therefore the concerns of insertional mutagenesis in retroviral based hematopoietic stem cell- based GT clinical trials could represent also a concern for AAV GT applications.

Previous severe adverse events observed in previous GT trials with γRVs, have prompted the adoption of molecular technologies enabling the detection and quantification of cell clones with vector insertions present in the transduced organs/tissues of subjects from preclinical and clinical GT applications. To this purpose, specialized PCR protocols have been devised to amplify cellular genomic portions flanking the integrated vector that, after sequencing, are mapped on the cellular genome. Since vector IS are specific hallmarks of clonal identity, integration site analyses allows to: A) Evaluate the clonality of the engraftment and the dynamics of hematopoietic reconstitution; B) Quantify the abundance of each clone in order to detect or exclude sustained clonal expansions; C) Identify the genes targeted by vector integrations.

Current PCR methods for AAV IS retrieval have been developed although the use of restriction enzymes as genomic DNA fragmentation method induces biases and hampers the detection and correct quantification of AAV IS. Moreover, since the AAV genome integration within the host cell genome, may also occur from internal vector fragments it is necessary to "scan" the entire vector genome for insertion site retrieval, increasing the complexity of the procedures and analyses and reducing the predictive power of IS analyses for this vector. There is a more relevant limit of IS analyses when applied for safety predictions for in vivo GT applications. Indeed, when solid tissues such as liver, brain muscle are transduced, the determination of the marking level of the targeted organ or the retrieval of vector IS must be performed on DNA purified from tissue biopsies, which are risky, invasive and provide information only for a small and spatially limited portion of the transduced organ. Furthermore the risks associated to the procedure of tissue extraction limit the possibility to perform repeated biopsies during time for longitudinal studies.

### Results

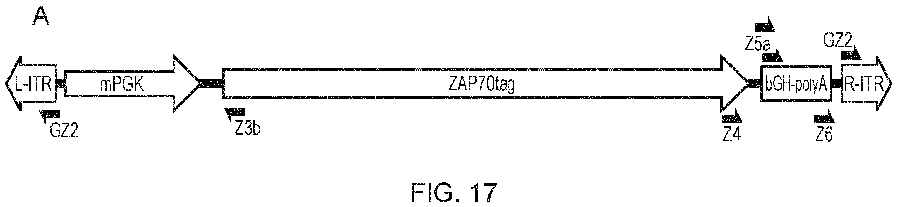

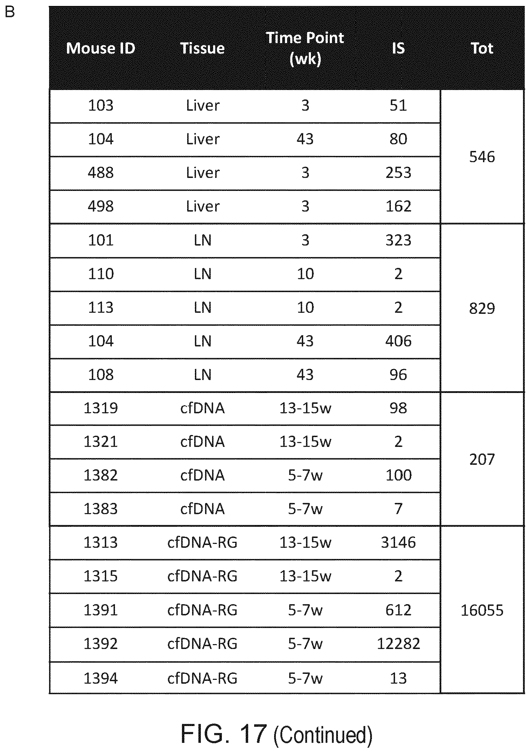

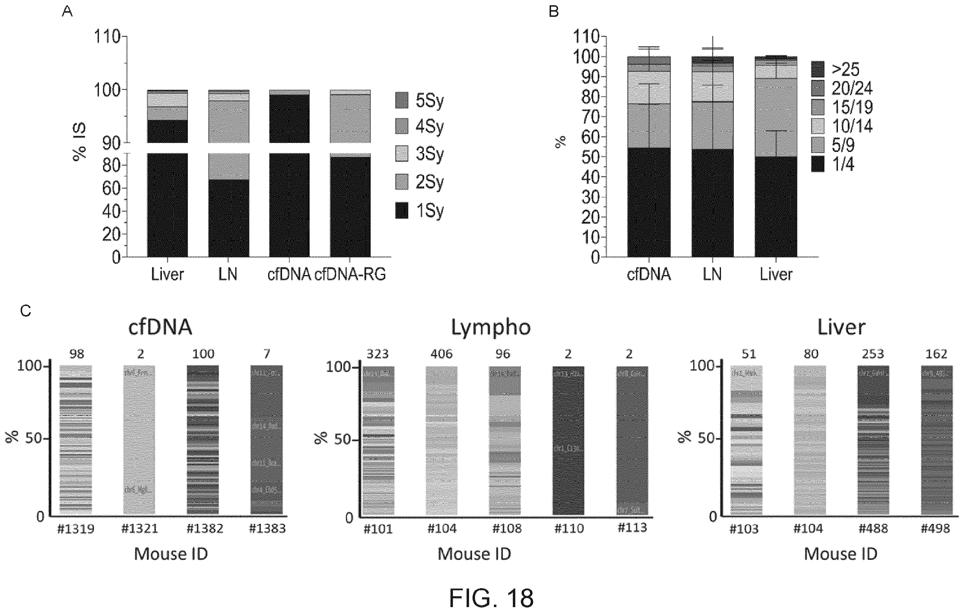

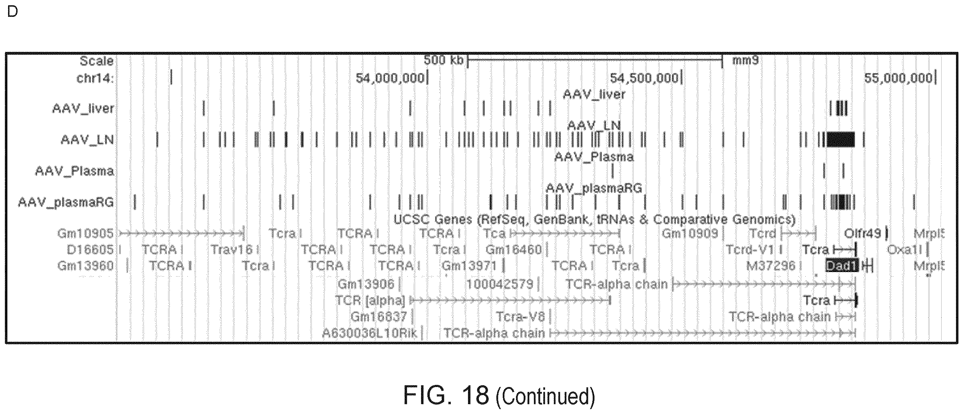

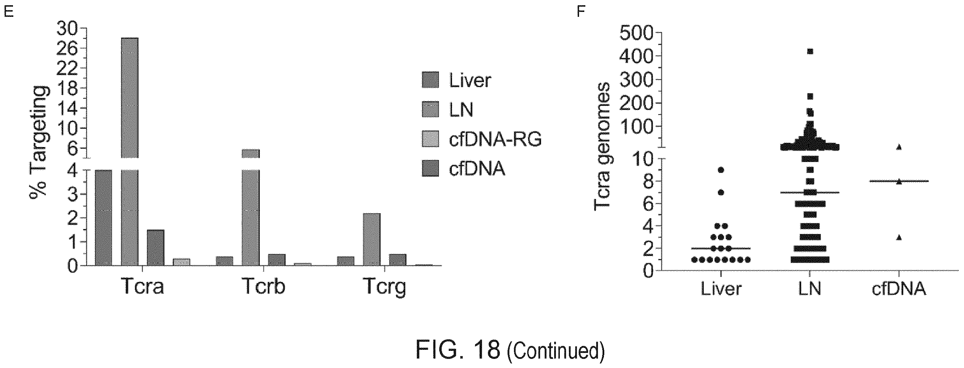

To test the ability of LiBIS-seq to retrieve AAV IS from cfDNA, the inventors took advantage of an in vivo mouse model of gene therapy, where immune-deficient ZAP70 knock-out mice were intra-thymically injected with an AAV8 vector expressing the therapeutic transgene under the control of a PGK promoter (Pouzolles, M., et al. (2019) Intrathymic AAV gene transfer rapidly restores thymic function and long-term persistence of gene-corrected T cells. The Journal of Allergy and Clinical Immunology). To scan the vector genome for recombination events, the inventors multiplexed the PCR protocol to amplify different flanking portions of the AAV genome (Figure 17A). The efficiency of the different PCR oligo systems in the retrieval of AAV IS was firstly evaluated on genomic DNA samples obtained from lymph node-derived mature T-cells and liver tissue by Sonication and Linker-mediated (SLiM) PCR protocol (Marktel, S. et al. (2019) Nat Med 25 234-241; Sessa, M. et al. (2016) Lancet 388: 476-487; Firouzi, S. et al. (2014) Genome medicine 6: 46; Gillet, N.A. et al. (2011) Blood 117: 3113-22). The "AAV-adapted" SLiM PCR protocol allowed the retrieval of 829 and 546 AAV IS from lymph-node and liver DNA respectively collected from 8 different injected-mice (Figure 17B). Given these results, the inventors purified cfDNA from plasma samples collected at two consecutive time points from 9 different mice and we performed LiBIS-seq using the same PCR oligo-system previously adopted. In 5 cases, cfDNA was amplified prior to LiBIS-seq procedure in order to increase the amount of material available (subsequently indicated as cfDNA-RG). By LiBIS-seq, we were able to collect 207 AAV IS from cfDNA and 16055 AAV IS from cfDNA-RG (Figure 17B). Interestingly, 16% (range: 2 to 38%) of the AAV IS retrieved from different sources were identified by multiple PCR systems, and 9 AAV IS were shared between the AAV IS retrieved in lymph node and liver of the same mouse 104, thus highlighting the reliability of the adopted PCR approach and the trafficking of corrected T cells among different tissue. Furthermore, 20% of AAV IS observed in LN and cfDNA dataset were represented by a high number of genomes (>10 genomes/fragments, Figure 18B), consistently with the high proliferative capacity of the targeted cell. No dominant clones were observed in any mouse among the different tissue (Figure 18C). Finally, we observed that AAV IS retrieved from lymph node-derived mature T-cells showed a strong bias to target T cell receptor genes, potentially in regions subjected to RAG recombinase-mediated rearrangements of TCR genes. Such enrichment can be explained by the trapping of AAV vector genome into the naturally occurring double strand breaks that are forms in the thymus by RAG enzyme during the T cell maturation process. Indeed, Tcra is the most targeted Tcr gene in all the different AAV dataset (Figure 18D, E), thus confirming the specificity of our PCR protocol and the ability of LiBIS-seq to efficiently retrieve AAV IS in an in vivo gene therapy context. Moreover, several AAV IS targeting the Tcra genes were represented by multiple genomes, indicating that AAV IS are stably inherited after cell replication and thus excluding that the insertion could have exclusively occurred into T-cell receptor excision circles (TRECs), episomal by-products of TCR recombination that do not have self-replication properties (Figure 18F).

Hence, the inventors' technology allows overcoming of the limitations imposed by the study of solid biopsies needed for IS analyses and safety predictions in in vivo GT applications. The safety and vector persistence in GT patients treated with AAV vectors can now can be monitored longitudinally by LiBIS-seq on liquid rather than solid biopsies avoiding the use of risky and invasive biopsies, providing a more comprehensive picture of the transduced organ and opening the door to longitudinal clonal tracking studies also for in vivo GT applications.

## Claims

1. A method for analysing insertion sites of an exogenous nucleotide sequence in a subject's genome, wherein the method comprises:
(a) using a provided sample from the subject comprising cell-free double-stranded DNA polynucleotides, wherein the sample is a body fluid sample from the subject;
(b) blunting the ends of the polynucleotides;
(c) ligating an oligonucleotide to both ends of the polynucleotides;
(d) amplifying polynucleotides comprising an insertion site; and
(e) sequencing the product of step (d),
wherein the exogenous nucleotide sequence is a viral vector, transposon or expression cassette.

2. The method of claim 1, wherein the analysing comprises identifying the location of one or more insertion sites in the subject's genome and/or quantifying the abundance of each of the one or more insertion sites.

3. The method of claim 1 or 2, wherein the analysing comprises determining the risk of insertional mutagenesis.

4. The method of any preceding claim, wherein the subject has been administered gene therapy or is about to be administered gene therapy.

5. The method of any preceding claim, wherein the viral vector is a viral gene therapy vector; and/or wherein the viral vector is a retroviral, lentiviral or AAV vector.

6. The method of any preceding claim, wherein the sample is a plasma, serum, urine or cerebrospinal fluid (CSF) sample, preferably a plasma sample.

7. The method of any preceding claim, wherein step (a) further comprises a step of purifying the polynucleotides; step (b) comprises end-repair or digestion of single-stranded overhangs, preferably end-repair; and/or step (b) further comprises 5' phosphorylation and/or 3' adenylation.

8. The method of any preceding claim, wherein the oligonucleotides are partially double-stranded.

9. The method of any preceding claim, wherein each oligonucleotide comprises:
(i) a first portion comprising a random nucleotide sequence;
(ii) a second portion comprising a barcode nucleotide sequence; and/or
(iii) a third portion comprising a binding site for a primer for use in the sequencing of step (e).

10. The method of any preceding claim, wherein the amplifying of step (d) is by PCR, preferably nested PCR, optionally wherein the PCR comprises amplifying the product of step (c) using a primer that binds to a portion of the exogenous nucleotide sequence and a primer that binds to a portion of the oligonucleotide.

11. The method of claim 10, wherein the PCR comprises:
(i) amplifying the product of step (c) using a first PCR step using a pair of outer primers to obtain a first PCR product; and
(ii) amplifying the first PCR product using a second PCR step using a pair of inner primers to obtain a second PCR product,
optionally wherein one primer of the pair of outer primers binds to a first portion of the exogenous nucleotide sequence and one primer of the pair of inner primers binds to a second portion of the exogenous nucleotide sequence; and/or wherein one primer of the pair of outer primers binds to a first portion of the oligonucleotide and one primer of the pair of inner primers binds to a second portion of the oligonucleotide.

12. The method of claim 11, wherein the nested PCR comprises:
(i) amplifying the product of step (c) using a first PCR step using a first primer that binds to a first portion of the exogenous nucleotide sequence and a second primer that binds to a first portion of an oligonucleotide to obtain a first PCR product; and
(ii) amplifying the first PCR product using a second PCR step using a third primer that binds to a second portion of the exogenous nucleotide sequence and a fourth primer that binds to a second portion of the oligonucleotide to obtain a second PCR product.

13. A method for determining the safety and/or efficacy of a gene therapy, wherein the method comprises:
(a) using a provided sample from a subject comprising cell-free double-stranded DNA polynucleotides, wherein the sample is a body fluid sample from the subject, preferably a plasma, serum, urine or cerebrospinal fluid (CSF) sample;
(b) blunting the ends of the polynucleotides;
(c) ligating an oligonucleotide to both ends of the polynucleotides;
(d) amplifying polynucleotides comprising a gene therapy vector insertion site; and
(e) sequencing the product of step (d) for identifying the location of one or more insertion sites in the subject's genome and/or quantifying the abundance of each of the one or more insertion sites.

14. A method for analysing vector insertion sites in a subject's genome, wherein the method comprises:
(a) using a provided sample from the subject comprising cell-free double-stranded DNA polynucleotides, wherein the sample is a body fluid sample from the subject, preferably a plasma, serum, urine or cerebrospinal fluid (CSF) sample;
(b) blunting the ends of the polynucleotides;
(c) ligating an oligonucleotide to both ends of the polynucleotides;
(d) amplifying polynucleotides comprising a vector insertion site; and
(e) sequencing the product of step (d) for identifying the location of one or more insertion sites in the subject's genome and/or quantifying the abundance of each of the one or more insertion sites.

15. A method for identifying gene therapy induced cancer cells in a subject, wherein the method comprises:
(a) using a provided sample from a subject comprising cell-free double-stranded DNA polynucleotides, wherein the sample is a body fluid sample from the subject, preferably a plasma, serum, urine or cerebrospinal fluid (CSF) sample;
(b) blunting the ends of the polynucleotides;
(c) ligating an oligonucleotide to both ends of the polynucleotides;
(d) amplifying polynucleotides comprising an insertion site of an exogenous nucleotide sequence; and
(e) sequencing the product of step (d) for identifying the location of one or more insertion sites in the subject's genome and/or quantifying the abundance of each of the one or more insertion sites,
wherein the exogenous nucleotide sequence is a viral vector, transposon or expression cassette.

## Patentansprüche

1. Verfahren zum Analysieren von Insertionsstellen einer exogenen Nukleotidsequenz im Genom eines Subjekts, wobei das Verfahren Folgendes umfasst:
(a) Verwendung einer bereitgestellten Probe aus dem Subjekt, die zellfreie doppelsträngige DNA-Polynukleotide umfasst, wobei es sich bei der Probe um eine Körperflüssigkeitsprobe aus dem Subjekt handelt;
(b) Stumpfmachen der Enden der Polynukleotide;
(c) Ligieren eines Oligonukleotids an beide Enden der Polynukleotide;
(d) Amplifizieren von Polynukleotiden, die eine Insertionsstelle umfassen; und
(e) Sequenzierung des Produkts aus Schritt (d), wobei die exogene Nukleotidsequenz ein viraler Vektor, ein Transposon oder eine Expressionskassette ist.

2. Verfahren nach Anspruch 1, wobei das Analysieren das Identifizieren der Position einer oder mehrerer Insertionsstellen im Genom des Subjekts und/oder das Quantifizieren der Häufigkeit jeder der einen oder der mehreren Insertionsstellen umfasst.

3. Verfahren nach Anspruch 1 oder 2, wobei das Analysieren die Bestimmung des Risikos einer Insertionsmutagenese umfasst.

4. Verfahren nach einem der vorhergehenden Ansprüche, wobei dem Subjekt eine Gentherapie verabreicht wurde oder es kurz davor steht, eine Gentherapie verabreicht zu bekommen.

5. Verfahren nach einem der vorhergehenden Ansprüche, wobei der virale Vektor ein viraler Gentherapievektor ist; und/oder wobei der virale Vektor ein retroviraler, lentiviraler oder AAV-Vektor ist.

6. Verfahren nach einem der vorhergehenden Ansprüche, wobei es sich bei der Probe um eine Plasma-, Serum-, Urin- oder Zerebrospinalflüssigkeit(CSF)-Probe, vorzugsweise eine Plasmaprobe handelt.

7. Verfahren nach einem der vorhergehenden Ansprüche, wobei Schritt (a) ferner einen Schritt der Reinigung der Polynukleotide umfasst; Schritt (b) die Enden-Reparatur oder den Verdau einzelsträngiger Überhänge, vorzugsweise die Enden-Reparatur umfasst; und/oder Schritt (b) ferner S'-Phosphorylierung und/oder 3'-Adenylierung umfasst.

8. Verfahren nach einem der vorhergehenden Ansprüche, wobei die Oligonukleotide teilweise doppelsträngig sind.

9. Verfahren nach einem der vorhergehenden Ansprüche, wobei jedes Oligonukleotid Folgendes umfasst:
(i) einen ersten Abschnitt, der eine zufällige Nukleotidsequenz umfasst;
(ii) einen zweiten Abschnitt, der eine Barcode-Nukleotidsequenz umfasst; und/oder
(iii) einen dritten Abschnitt, der eine Bindungsstelle für einen Primer zur Verwendung bei der Sequenzierung von Schritt (e) umfasst.

10. Verfahren nach einem der vorhergehenden Ansprüche, wobei das Amplifizieren von Schritt (d) durch PCR, vorzugsweise verschachtelte PCR (Nested-PCR), erfolgt, wobei die PCR optional das Amplifizieren des Produkts aus Schritt (c) unter Verwendung eines Primers, der an einen Abschnitt der exogenen Nukleotidsequenz bindet, und eines Primers, der an einen Abschnitt des Oligonukleotids bindet, umfasst.

11. Verfahren nach Anspruch 10, wobei die PCR umfasst:
(i) Amplifizieren des Produkts aus Schritt (c) mittels eines ersten PCR-Schritts unter Verwendung eines Paars äußerer Primer, um ein erstes PCR-Produkt zu erhalten; und
(ii) Amplifizieren des ersten PCR-Produkts mittels eines zweiten PCR-Schritts unter Verwendung eines Paars innerer Primer, um ein zweites PCR-Produkt zu erhalten, optional wobei ein Primer des Paares äußerer Primer an einen ersten Abschnitt der exogenen Nukleotidsequenz bindet und ein Primer des Paares innerer Primer an einen zweiten Abschnitt der exogenen Nukleotidsequenz bindet; und/oder wobei ein Primer des Paares äußerer Primer an einen ersten Abschnitt des Oligonukleotids bindet und ein Primer des Paares innerer Primer an einen zweiten Abschnitt des Oligonukleotids bindet.

12. Verfahren nach Anspruch 11, wobei die verschachtelte PCR Folgendes umfasst:
(i) Amplifizieren des Produkts aus Schritt (c) mittels eines ersten PCR-Schritts unter Verwendung eines ersten Primers, der an einen ersten Abschnitt der exogenen Nukleotidsequenz bindet, und eines zweiten Primers, der an einen ersten Abschnitt eines Oligonukleotids bindet, um ein erstes PCR-Produkt zu erhalten; und
(ii) Amplifizieren des ersten PCR-Produkts mittels eines zweiten PCR-Schritts unter Verwendung eines dritten Primers, der an einen zweiten Abschnitt der exogenen Nukleotidsequenz bindet, und eines vierten Primers, der an einen zweiten Abschnitt des Oligonukleotids bindet, um ein zweites PCR-Produkt zu erhalten.

13. Verfahren zur Bestimmung der Sicherheit und/oder Wirksamkeit einer Gentherapie, wobei das Verfahren Folgendes umfasst:
(a) Verwendung einer bereitgestellten Probe aus einem Subjekt, die zellfreie doppelsträngige DNA-Polynukleotide umfasst, wobei es sich bei der Probe um eine Körperflüssigkeitsprobe aus dem Subjekt, vorzugsweise eine Plasma-, Serum-, Urin- oder Zerebrospinalflüssigkeit(CSF)-Probe handelt;
(b) Stumpfmachen der Enden der Polynukleotide;
(c) Ligieren eines Oligonukleotids an beide Enden der Polynukleotide;
(d) Amplifizieren von Polynukleotiden, die eine Gentherapievektor-Insertionsstelle umfassen; und
(e) Sequenzierung des Produkts aus Schritt (d) zum Identifizieren der Position einer oder mehrerer Insertionsstellen im Genom des Subjekts und/oder Quantifizieren der Häufigkeit jeder der einen oder der mehreren Insertionsstellen.

14. Verfahren zum Analysieren von Vektorinsertionsstellen im Genom eines Subjekts, wobei das Verfahren Folgendes umfasst:
(a) Verwendung einer bereitgestellten Probe aus dem Subjekt, die zellfreie doppelsträngige DNA-Polynukleotide umfasst, wobei es sich bei der Probe um eine Körperflüssigkeitsprobe aus dem Subjekt, vorzugsweise eine Plasma-, Serum-, Urin- oder Zerebrospinalflüssigkeit(CSF)-Probe handelt;
(b) Stumpfmachen der Enden der Polynukleotide;
(c) Ligieren eines Oligonukleotids an beide Enden der Polynukleotide;
(d) Amplifizieren von Polynukleotiden, die eine Vektorinsertionsstelle umfassen; und
(e) Sequenzierung des Produkts aus Schritt (d) zum Identifizieren der Position einer oder mehrerer Insertionsstellen im Genom des Subjekts und/oder Quantifizieren der Häufigkeit jeder der einen oder der mehreren Insertionsstellen.

15. Verfahren zur Identifizierung gentherapieinduzierter Krebszellen in einem Subjekt, wobei das Verfahren Folgendes umfasst:
(a) Verwendung einer bereitgestellten Probe aus einem Subjekt, die zellfreie doppelsträngige DNA-Polynukleotide umfasst, wobei es sich bei der Probe um eine Körperflüssigkeitsprobe aus dem Subjekt, vorzugsweise eine Plasma-, Serum-, Urin- oder Zerebrospinalflüssigkeit(CSF)-Probe handelt;
(b) Stumpfmachen der Enden der Polynukleotide;
(c) Ligieren eines Oligonukleotids an beide Enden der Polynukleotide;
(d) Amplifizieren von Polynukleotiden, die eine Insertionsstelle einer exogenen Nukleotidsequenz umfassen; und
(e) Sequenzierung des Produkts aus Schritt (d) zum Identifizieren der Position einer oder mehrerer Insertionsstellen im Genom des Subjekts und/oder Quantifizieren der Häufigkeit jeder der einen oder der mehreren Insertionsstellen, wobei die exogene Nukleotidsequenz ein viraler Vektor, ein Transposon oder eine Expressionskassette ist.

## Revendications

1. Procédé pour l'analyse de sites d'insertion d'une séquence nucléotidique exogène dans le génome d'un sujet, le procédé comprenant :
(a) l'utilisation d'un échantillon fourni du sujet comprenant des polynucléotides d'ADN double brin sans cellule, l'échantillon étant un échantillon de fluide corporel provenant du sujet ;
(b) la formation d'extrémités franches des polynucléotides ;
(c) la ligature d'un oligonucléotide aux deux extrémités des polynucléotides ;
(d) l'amplification de polynucléotides comprenant un site d'insertion ; et
(e) le séquençage du produit de l'étape (d),
la séquence nucléotidique exogène étant un vecteur viral, un transposon ou une cassette d'expression.

2. Procédé selon la revendication 1, l'analyse comprenant l'identification de l'emplacement d'un ou plusieurs sites d'insertion dans le génome du sujet et/ou la quantification de l'abondance de chacun du ou des sites d'insertion.

3. Procédé selon la revendication 1 ou 2, l'analyse comprenant la détermination du risque de mutagenèse insertionnelle.

4. Procédé selon une quelconque revendication précédente, dans lequel on a administré une thérapie génique au sujet ou on est sur le point d'administrer une thérapie génique au sujet.

5. Procédé selon une quelconque revendication précédente, le vecteur viral étant un vecteur de thérapie génique viral ; et/ou le vecteur viral étant un vecteur rétroviral, lentiviral ou AAV.

6. Procédé selon une quelconque revendication précédente, l'échantillon étant un échantillon de plasma, de sérum, d'urine ou de fluide cérébrospinal (CSF), préférablement un échantillon de plasma.

7. Procédé selon une quelconque revendication précédente, l'étape (a) comprenant en outre une étape de purification des polynucléotides ; l'étape (b) comprenant une réparation d'extrémité ou une digestion de surplombs simples brins, préférablement une réparation d'extrémité ; et/ou l'étape (b) comprenant en outre une phosphorylation en 5' et/ou une adénylation en 3'.

8. Procédé selon une quelconque revendication précédente, les oligonucléotides étant partiellement à double brin.

9. Procédé selon une quelconque revendication précédente, chaque oligonucléotide comprenant :
(i) une première partie comprenant une séquence nucléotidique aléatoire ;
(ii) une deuxième partie comprenant une séquence nucléotidique à code-barres ; et/ou
(iii) une troisième portion comprenant un site de liaison pour une amorce pour une utilisation dans le séquençage de l'étape (e).

10. Procédé selon une quelconque revendication précédente, l'amplification de l'étape (d) étant par PCR, préférablement par PCR nichée, éventuellement la PCR comprenant l'amplification du produit de l'étape (c) en utilisant une amorce qui se lie à une partie de la séquence nucléotidique exogène et une amorce qui se lie à une partie de l'oligonucléotide.

11. Procédé selon la revendication 10, la PCR comprenant :
(i) l'amplification du produit de l'étape (c) en utilisant une première étape de PCR en utilisant une paire d'amorces externes pour obtenir un premier produit de PCR ; et
(ii) l'amplification du premier produit de PCR en utilisant une deuxième étape de PCR en utilisant une paire d'amorces internes pour obtenir un deuxième produit de PCR,
éventuellement, une amorce de la paire d'amorces externes se liant à une première partie de la séquence nucléotidique exogène et une amorce de la paire d'amorces internes se liant à une deuxième partie de la séquence nucléotidique exogène ; et/ou une amorce de la paire d'amorces externes se liant à une première partie de l'oligonucléotide et une amorce de la paire d'amorces internes se liant à une deuxième partie de l'oligonucléotide.

12. Procédé selon la revendication 11, la PCR nichée comprenant :
(i) l'amplification du produit de l'étape (c) en utilisant une première étape de PCR en utilisant une première amorce qui se lie à une première partie de la séquence nucléotidique exogène et une deuxième amorce qui se lie à une première partie d'un oligonucléotide pour obtenir un premier produit de PCR ; et
(ii) l'amplification du premier produit de PCR en utilisant une deuxième étape de PCR en utilisant une troisième amorce qui se lie à une deuxième partie de la séquence nucléotidique exogène et une quatrième amorce qui se lie à une deuxième partie de l'oligonucléotide pour obtenir un deuxième produit de PCR.

13. Procédé pour la détermination de l'innocuité et/ou de l'efficacité d'une thérapie génique, le procédé comprenant :
(a) l'utilisation d'un échantillon fourni d'un sujet comprenant des polynucléotides d'ADN double brin sans cellule, l'échantillon étant un échantillon de fluide corporel provenant du sujet, préférablement un échantillon de plasma, de sérum, d'urine ou de fluide cérébrospinal (CSF) ;
(b) la formation d'extrémités franches des polynucléotides ;
(c) la ligature d'un oligonucléotide aux deux extrémités des polynucléotides ;
(d) l'amplification de polynucléotides comprenant un site d'insertion de vecteur de thérapie génique ; et
(e) le séquençage du produit de l'étape (d) pour l'identification de l'emplacement d'un ou plusieurs sites d'insertion dans le génome du sujet et/ou la quantification de l'abondance de chacun du ou des sites d'insertion.

14. Procédé pour l'analyse de sites d'insertion de vecteur dans le génome d'un sujet, le procédé comprenant :
(a) l'utilisation d'un échantillon fourni du sujet comprenant des polynucléotides d'ADN double brin sans cellule, l'échantillon étant un échantillon de fluide corporel provenant du sujet, préférablement un échantillon de plasma, de sérum, d'urine ou de fluide cérébrospinal (CSF) ;
(b) la formation d'extrémités franches des polynucléotides ;
(c) la ligature d'un oligonucléotide aux deux extrémités des polynucléotides ;
(d) l'amplification de polynucléotides comprenant un site d'insertion de vecteur ; et
(e) le séquençage du produit de l'étape (d) pour l'identification de l'emplacement d'un ou de plusieurs sites d'insertion dans le génome du sujet et/ou la quantification de l'abondance de chacun du ou des sites d'insertion.

15. Procédé pour l'identification de cellules cancéreuses induites par une thérapie génique chez un sujet, le procédé comprenant :
(a) l'utilisation d'un échantillon fourni d'un sujet comprenant des polynucléotides d'ADN double brin sans cellule, l'échantillon étant un échantillon de fluide corporel provenant du sujet, préférablement un échantillon de plasma, de sérum, d'urine ou de fluide cérébrospinal (CSF) ;
(b) la formation d'extrémités franches des polynucléotides ;
(c) la ligature d'un oligonucléotide aux deux extrémités des polynucléotides ;
(d) l'amplification de polynucléotides comprenant un site d'insertion d'une séquence nucléotidique exogène ; et
(e) le séquençage du produit de l'étape (d) pour l'identification de l'emplacement d'un ou de plusieurs sites d'insertion dans le génome du sujet et/ou la quantification de l'abondance de chacun du ou des sites d'insertion,
la séquence nucléotidique exogène étant un vecteur viral, un transposon ou une cassette d'expression.
